# EUROPÄISCHE PATENTSCHRIFT

(11) **EP 4 451 921 B1**
(45) Veröffentlichungstag und Bekanntmachung des Hinweises auf die Patenterteilung: **02.12.2026**
(21) Anmeldenummer: 22843206.8
(22) Anmeldetag: 20.12.2022
(51) Int. Cl.: A23P 10/40, C07H 3/04, C13B 50/00, C13K 3/00

(54) **RIESELFÄHIGE KOHLENHYDRATE UND VERFAHREN ZU IHRER HERSTELLUNG**
FREE-FLOWING CARBOHYDRATES AND METHOD FOR PREPARING SAME
GLUCIDES COULANTS ET LEUR PROCÉDÉ DE PRODUCTION

(30) Priorität: 21.12.2021 EP 21216373
(43) Veröffentlichungstag der Anmeldung: 30.10.2024
(73) Patentinhaber: Südzucker AG, 68165 Mannheim (DE)
(72) Erfinder: KLISS, Rainer, 67283 Obrigheim (DE); STRACK, Jan, 67283 Obrigheim (DE); BERNARD, Jörg, 67283 Obrigheim (DE)
(74) Vertreter: Schrell, Andreas
(86) Internationale Anmeldenummer: PCT/EP2022/087056
(87) Internationale Veröffentlichungsnummer: WO 2023/118187

(56) Entgegenhaltungen:
- WO-A1-2021/158932
- WO-A1-90/14821
- DE-A1- 102006 022 506
- DE-A1- 102018 201 916

## Beschreibung

### BESCHREIBUNG

Die vorliegende Erfindung betrifft ein Verfahren zur Herstellung einer getrockneten Kohlenhydratpräparation aus einer wasserhaltigen kristallinen Kohlenhydrat-Ausgangspräparation und Kohlenhydratpartikel.

Kristalline Kohlenhydratpräparationen, die an der Oberfläche mit einen Sirupfilm überzogen sind, oder teilamorphe feste Kohlenhydratpräparationen neigen dazu, während der Lagerung Wasser aufzunehmen und in der Folge zu verkleben. Ein derartiger Sirupfilm kann aus vorhergehenden Prozeßschritten der Kohlenhydrataufarbeitung stammen. Dies führt zu verbackenen Kohlenhydratpräparationen, die nicht oder nur schlecht riesel-, transport-, dosier- und lagerfähig sind. Für vermarktungsfähige Kohlenhydratpräparationen ist es jedoch notwendig, lagerstabile und dosierbare Kohlenhydratpräparationen bereitzustellen. Sämtliche Prozessschritte der Förderung, Wägung, Dosierung und ähnliche mechanischen Behandlungen stellen bei der Verarbeitung von leicht verklumpenden, nicht rieselfähigen festen Kohlenhydratpräparationen ein Problem dar. Feste kristalline Kohlenhydratpräparationen, die weniger gereinigt sind oder von einem Sirupfilm mit gewünschten geschmacklichen Eigenschaften, zum Beispiel für ein verbessertes Aroma, überzogen sind, werden grundsätzlich allerdings zumindest von bestimmten Verbrauchergruppen besonders geschätzt. Allerdings werden nur in besonderen Einzelfällen und in lokal begrenzten Märkten sogenannte "Soft-Sugar Kohlenhydratpräparationen" (wie "Farin-Zucker" oder "Basterd Sugar"), also klebrige Kohlenhydratpräparationen vom Käufer tatsächlich als Spezialität akzeptiert. In der Regel besteht der Käufer, sowohl der Endverbraucher als auch der Weiterverarbeiter, dagegen auf gut rieselfähigen und lagerstabilen Kohlenhydratpräparationen. Insbesondere stellen "Soft-Sugar Kohlenhydratpräparationen" den Verarbeiter in der Handhabung und der Abpackung vor Probleme mit wirtschaftlich ungünstigen Abpackzeiten und häufigen Produktionsstörungen. Darüber hinaus sollen die Kohlenhydratpräparationen möglichst mit wenigen Zusatzstoffen oder Hilfsstoffen versehen werden. Darunter fällt auch der Einsatz von sogenannten Rieselhilfsmitteln.

Kohlenhydratpräparationen aus dem Stand der Technik werden häufig so stark getrocknet, dass ein auf den Kristallen anhaftender Sirupfilm nicht mehr direkt zum Verkleben neigt. In solchen Fällen wird eine hermetisch verschlossene Verpackung notwendig, um eine nachträgliche Wasseraufnahme durch einen Sorptionsvorgang zu vermeiden. Es lassen sich jedoch nicht alle Kohlenhydratpräparationen in dieser Art behandeln. Das Hauptproblem bei dieser Methode ist die aufwändige Verpackung und Lagerung, da diese eine nachträgliche Wasseraufnahme verhindern muss. Schlecht zu trocknende Kohlenhydratpräparationen können durch einen Sprühtrocknungsprozess in eine trockene Form überführt werden. Diese Technik ist jedoch kostenaufwändig und eignet sich nicht für Kohlenhydratpräparationen, in denen Kristalle dominieren und nur eine geringe Sirupmenge vorliegt. Auch bei dieser Methode sind eine aufwändige Verpackung und Lagerung notwendig, um eine nachträgliche Wasseraufnahme und ein Verkleben zu vermeiden. Bei kristallinen Kohlenhydratpräparationen, deren Oberflächen zum Verkleben neigen, können sogenannte Rieselhilfsmittel eingesetzt werden. Diese bestehen typischerweise nicht aus denselben Kohlenhydraten, aus denen das gewünschte Endprodukt besteht, sondern gehören der Klasse der anorganischen Substanzen (wie SiO₂, Blutlaugensalz) oder Stärken an. Dies hat den Nachteil, dass weitere Hilfsstoffe in der Kohlenhydratpräparation deklariert werden müssen, was eine regulatorische Hürde darstellen kann oder von Konsumenten nicht akzeptiert wird. Auch eine geringfügige sensorische Beeinflussung kann nicht ausgeschlossen werden.

Nachteilhaft für bestimmte Kohlenhydratpräparationen, insbesondere für kristalline Saccharose-haltige Kohlenhydratpräparationen, ist, dass sich bei erhöhtem Wassergehalt (>0,05 Gew.-%, bezogen auf Gesamtgewicht der kristallinen, insbesondere Saccharose-haltigen, Kohlenhydratpräparation), insbesondere bei einem auf den kristallinen Partikeln vorhandenem Sirupfilm, Verklumpungen und ein Verlust der Rieselfähigkeit zeigen. Dazu können auf der Kristalloberfläche verbleibende Stoffe die Hygroskopizität erhöhen.

WO2021/158932A1 offenbart ein Verfahren zur Herstellung fester Glukoseprodukte aus hochkonzentrierten wässrigen Glukoselösungen durch Zugabe von Kohlenhydratpartikeln und anschließende Verfestigung.

Das der Erfindung zugrunde liegende technische Problem liegt daher darin, vorgenannte Nachteile zu überwinden. Insbesondere liegt das der Erfindung zugrunde liegende technische Problem darin, kostengünstige und leicht durchzuführende Verfahren bereitzustellen, die es ermöglichen, eine riesel-, transport-, dosier- und lagerfähige Kohlenhydratpräparation bereitzustellen, insbesondere aus einer Kohlenhydratausgangspräparation, die Sirupfilm-beschichtete kristalline Partikel aufweist, wobei erhaltene Kohlenhydratpräparation insbesondere mit möglichst wenigen bis gar keinen Zusatzstoffen oder Hilfsstoffen, insbesondere Rieselhilfsmitteln, bereitgestellt wird und insbesondere einfach abzupacken ist.

Insbesondere liegt der vorliegenden Erfindung das technische Problem zugrunde, ein Verfahren zur Trocknung von einer in Wasser gelösten kristallinen Kohlenhydrat-Ausgangspräparation bereitzustellen, welche zumindest zwei Kohlenhydrate mit unterschiedlicher Wasserlöslichkeit, insbesondere mindestens ein schlecht-lösliches Kohlenhydrat und mindestens ein gut-lösliches Kohlenhydrat aufweist, welches effizient und möglichst störungsarm zur Bereitstellung getrockneter Kohlenhydratpräparationen führt und insbesondere das Trocknen von mit üblichen Verfahren schwer oder gar nicht zu trocknenden Kohlenhydratpräparationen ermöglicht.

Das der Erfindung zugrunde liegende technische Problem wird durch die Lehre der vorliegenden Erfindung gelöst, insbesondere durch ein Verfahren zur Herstellung einer getrockneten Kohlenhydratpräparation aus einer wasserhaltigen kristallinen Kohlenhydrat-Ausgangspräparation, umfassend die Verfahrensschritte:
a) Bereitstellung einer wasserhaltigen 0,4 bis 22,5 Gew.-%, insbesondere 2,5 bis 12 Gew.-% Wasser (jeweils bezogen auf Gesamtgewicht der Kohlenhydrat-Ausgangspräparation) aufweisenden kristallinen Kohlenhydrat-Ausgangspräparation, wobei die Kohlenhydrat-Ausgangspräparation ausgewählt ist aus der Gruppe bestehend aus einer Saccharose-haltigen Zusammensetzung, einer Isomaltulose- und Trehalulose-haltigen Zusammensetzung und einer Isomalt-haltigen Zusammensetzung in einem Trocknungsreaktor, sowie von Kohlenhydratpartikeln, wobei die Kohlenhydratpartikel einen Anteil von Kohlenhydratkristallen mit einem Durchmesser von kleiner oder gleich 100 µm von mindestens 80 Gew.-% aufweisen, insbesondere wobei die Kohlenhydrat-Ausgangspräparation einen über einen gegebenenfalls vorhandenen Kristallwassergehalt hinausgehenden Gehalt an Wasser, insbesondere in Form eines Flüssigkeitsfilms auf kristallinen Partikeln der Kohlenhydrat-Ausgangspräparation, aufweist,
b) Eindosieren der Kohlenhydratpartikel über eine dem Trocknungsreaktor zugeordnete Dosiervorrichtung in die in dem Trocknungsreaktor vorgelegte wasserhaltige kristalline Kohlenhydrat-Ausgangspräparation in einer Menge von 2 bis 30 Gew.-% (bezogen auf Gesamtgewicht der Kohlenhydrat-Ausgangspräparation) bei einem Druck von 10 bis 1100 mbar, wobei die in dem Trocknungsreaktor vorgelegte wasserhaltige Kohlenhydrat-Ausgangspräparation eine Temperatur von 20 bis 80 °C, insbesondere 40 bis 60 °C aufweist, und
c) Homogenisieren der erhaltenen Mischung mittels einer Mischvorrichtung unter den in Verfahrensschritt b) genannten Bedingungen zum Erhalten einer getrockneten Kohlenhydratpräparation.

Erfindungsgemäß bevorzugt weist die in Verfahrensschritt a) bereitgestellte wasserhaltige 0,4 bis 22,5 Gew.-%, insbesondere 2,5 bis 12 Gew.-% Wasser (jeweils bezogen auf Gesamtgewicht der Kohlenhydrat-Ausgangspräparation) aufweisende kristalline Kohlenhydrat-Ausgangspräparation Flüssigkeitsfilm-überzogene kristalline Kohlenhydratpartikel auf, insbesondere besteht sie aus derartigen Flüssigkeitsfilm-überzogenen kristallinen Partikeln. Bevorzugt wird in Verfahrensschritt c) eine getrocknete Kohlenhydratpräparation umfassend Flüssigkeitsfilmbefreite kristalline Kohlenhydratpartikeln, insbesondere bestehend aus diesen, erhalten.

Die erfindungsgemäß eingesetzte wasserhaltige kristalline Kohlenhydrat-Ausgangspräparation ist insbesondere eine Präparation, in der die kristalline Masse der Kohlenhydrate, insbesondere die kristallinen Partikel mit einem Flüssigkeitsfilm beschichtet ist, vorliegend auch als Flüssigkeitsfilm-überzogen bezeichnet. Der Flüssigkeitsfilm, vorliegend auch als Sirupfilm bezeichnet, stellt eine oberflächlich auf den kristallinen Partikeln vorhandene wäßrige Lösung oder Suspension des Kohlenhydrats oder der Kohlenhydrate der Ausgangspräparation dar, und kann aus vorhergehenden Prozeßschritten der Kohlenhydrataufarbeitung stammen. Der Sirupfilm stellt damit einen nicht integral im kristallinen Partikel vorhandenen Wasseranteil dar, sondern liegt oberflächlich auf den kristallinen Partikeln vor, insbesondere ist der Wasseranteil des Sirupfilms kein Kristallwasser. Sofern in einem Kohlenhydrat Kristallwasser vorliegt, stellt der Sirupfilm einen weiteren, also zusätzlichen, Wasseranteil im Produkt dar. Erfindungsgemäß bevorzugt weist die wasserhaltige kristalline Kohlenhydrat-Ausgangspräparation daher kristalline Partikel auf, die einen über den gegebenenfalls vorhandenen Kristallwassergehalt hinausgehenden Wassergehalt aufweisen. Die erfindungsgemäß eingesetzte wasserhaltige kristalline Kohlenhydrat-Ausgangspräparation ist daher keine getrocknete Kristallwasser-haltige oder getrocknete Kristallwasser-freie Kohlenhydratpräparation.

Überraschenderweise führt das erfindungsgemäß vorgesehene Eindosieren von Kohlenhydratpartikeln, wobei die Kohlenhydratpartikel einen Anteil von Kohlenhydratkristallen mit einem Durchmesser von kleiner oder gleich 100 µm von mindestens 80 Gew.-% aufweisen, zu den Sirupfilm-aufweisenden Kohlenhydratpartikeln der wasserhaltigen Ausgangspräparation nicht zu der erwarteten Verklumpung von kristallinen Partikeln, sondern vielmehr genau entgegengesetzt zu einer vorteilhaften erhöhten Rieselfähigkeit.

Das der Erfindung zugrunde liegende technische Problem wird durch die Lehre der vorliegenden Erfindung gelöst, insbesondere durch ein Verfahren zur Herstellung einer getrockneten Kohlenhydratpräparation aus einer wasserhaltigen kristallinen Kohlenhydrat-Ausgangspräparation, umfassend die Verfahrensschritte:
a) Bereitstellung einer wasserhaltigen 0,4 bis 22,5 Gew.-%, insbesondere 2,5 bis 12 Gew.-% Wasser (jeweils bezogen auf Gesamtgewicht der Kohlenhydrat-Ausgangspräparation) aufweisenden kristallinen Kohlenhydrat-Ausgangspräparation, wobei die Kohlenhydrat-Ausgangspräparation ausgewählt ist aus der Gruppe bestehend aus einer Saccharose-haltigen Zusammensetzung, einer Isomaltulose- und Trehalulose-haltigen Zusammensetzung und einer Isomalt-haltigen Zusammensetzung in einem Trocknungsreaktor, sowie von Kohlenhydratpartikeln, wobei die Kohlenhydratpartikel einen Anteil von Kohlenhydratkristallen mit einem Durchmesser von kleiner oder gleich 100 µm von mindestens 80 Gew.-% aufweisen,
b) Eindosieren der Kohlenhydratpartikel über eine dem Trocknungsreaktor zugeordnete Dosiervorrichtung in die in dem Trocknungsreaktor vorgelegte wasserhaltige kristalline Kohlenhydrat-Ausgangspräparation in einer Menge von 2 bis 30 Gew.-% (bezogen auf Gesamtgewicht der Kohlenhydrat-Ausgangspräparation) bei einem Druck von 10 bis 1100 mbar, wobei die in dem Trocknungsreaktor vorgelegte wasserhaltige Kohlenhydrat-Ausgangspräparation eine Temperatur von 20 bis 80 °C, insbesondere 40 bis 60 °C aufweist, und
c) Homogenisieren der erhaltenen Mischung mittels einer Mischvorrichtung unter den in Verfahrensschritt b) genannten Bedingungen zum Erhalten einer getrockneten Kohlenhydratpräparation, insbesondere mit einem Wassergehalt von höchstens 6,0, insbesondere höchstens 5,0, insbesondere höchstens 1,9 Gew.-%, insbesondere 0,01 bis 0,70 Gew.-% (jeweils bezogen auf Gesamtgewicht der getrockneten Kohlenhydratpräparation).

Die Erfindung geht von einer wasserhaltigen 0,4 bis 22,5 Gew.-% Wasser (bezogen auf Gesamtgewicht der Kohlenhydrat-Ausgangspräparation) aufweisenden kristallinen Kohlenhydrat-Ausgangspräparation aus, die in Verfahrensschritt a) in einem Trocknungsreaktor bereitgestellt wird und einem Verfahren unterzogen wird, das insbesondere unter Einsatz der Verfahrensschritte b) und c) zum Erhalten einer getrockneten Kohlenhydratpräparation führt, wobei die getrocknete Kohlenhydratpräparation einen geringeren Wassergehalt als die wasserhaltige kristalline Kohlenhydrat-Ausgangspräparation aufweist.

Die erfindungsgemäß eingesetzte wasserhaltige kristalline Kohlenhydrat-Ausgangspräparation ist ausgewählt aus der Gruppe bestehend aus einer Saccharose-haltigen Zusammensetzung, einer Isomaltulose- und Trehalulose-haltigen Zusammensetzung und einer Isomalt-haltigen Zusammensetzung. In Verfahrensschritt a) werden außerdem Kohlenhydratpartikel, wobei das Kohlenhydrat der Kohlenhydratpartikel bevorzugt identisch mit dem Kohlenhydrat der Kohlenhydrat-Ausgangspräparation ist, bereitgestellt, die einen Anteil von Kohlenhydratkristallen mit einem Durchmesser von kleiner oder gleich 100 µm von mindestens 80 Gew.-% aufweisen. Die in Verfahrensschritt a) bereitgestellten Kohlenhydratpartikel werden in einem Verfahrensschritt b) über eine dem Trocknungsreaktor zugeordnete Dosiervorrichtung in die in dem Trocknungsreaktor vorgelegte wasserhaltige kristalline Kohlenhydrat-Ausgangspräparation in einer Menge von 2 bis 30 Gew.-% (bezogen auf Gesamtgewicht der Kohlenhydrat-Ausgangspräparation) bei einem Druck von 10 bis 1100 mbar eindosiert, wobei die in dem Trocknungsreaktor vorgelegte wasserhaltige Kohlenhydrat-Ausgangspräparation eine Temperatur von 20 bis 80 °C aufweist, und so eine Mischung aus Kohlenhydratpartikeln und wasserhaltige Ausgangspräparation erhalten wird. Die so erhaltene Mischung wird in einem Verfahrensschritt c) mittels einer Mischvorrichtung unter den in Verfahrensschritt b) genannten Bedingungen homogenisiert und dabei eine getrocknete Kohlenhydratpräparation, insbesondere mit einem Wassergehalt von höchstens 6,0, insbesondere höchstens 5,0, insbesondere höchstens 1,9 Gew.-% (jeweils bezogen auf Gesamtgewicht der getrockneten Kohlenhydratpräparation) erhalten.

Die erfindungsgemäße Durchführung der Verfahrensschritte b) und c) führt zu einer Trocknung der eingesetzten einen bestimmten Wassergehalt aufweisenden kristallinen Kohlenhydrat-Ausgangspräparation, das heißt zum Erhalten einer getrockneten Kohlenhydratpräparation, die einen geringeren Wassergehalt als die in Verfahrensschritt a) eingesetzte wasserhaltige 0,4 bis 22,5 Gew.-% Wasser (bezogen auf Gesamtgewicht der Kohlenhydrat-Ausgangspräparation) aufweisende kristalline Kohlenhydrat-Ausgangspräparation aufweist. Die Verfahrensschritte b) und c) führen daher in jedem Fall, unabhängig vom konkreten Wassergehalt der eingesetzten kristallinen Kohlenhydrat-Ausgangspräparation zu einer Reduktion dieses Wassergehalts und gleichzeitig zu einer überraschend deutlich verbesserten Rieselfähigkeit. Insbesondere zeichnet sich die gemäß der Verfahrensschritte b) und c) erhaltene getrocknete Kohlenhydratpräparation dadurch aus, daß sie in einem optional folgenden Konditionierschritt ohne weiteres zu einem besonders gut dosierbaren und rieselfähigen getrocknetem und konditioniertem Produkt weiterbehandelt werden kann, was ohne die erfindungsgemäß vorgesehene vorgeschaltete Trocknung gemäß der Schritte b) und c) nicht möglich wäre.

Insbesondere ermöglicht es die vorliegende Erfindung, dass eine in Verfahrensschritt a) in einem Trocknungsreaktor bereitgestellte wasserhaltige kristalline Kohlenhydrat-Ausgangspräparation, zum Beispiel eine mit einem Sirupfilm überzogene Saccharose-haltige Ausgangspräparation, gar nicht beziehungsweise nur bis zu einem wirtschaftlich günstigen Trocknungsgrad vor der Bereitstellung gemäß Verfahrensschritt a) getrocknet werden muss. Erfindungsgemäß können so unmittelbar aus einem Kohlenhydrat-Kristallisationsprozeß stammende Präparationen, zum Beispiel noch Restwassergehalte, insbesondere Restmutterlaugengehalte, aufweisende Kristallsuspensionen ohne kostenaufwändiges Trocknen in einem erfindungsgemäßen Verfahren durch die in Verfahrensschritt b) vorgesehene Zugabe von Kohlenhydratpartikeln in einer Menge von 2 bis 30 Gew.-% (bezogen auf Gesamtgewicht der Kohlenhydrat-Ausgangspräparation) getrocknet werden.

Die Erfindung sieht demgemäß ein Verfahren zur Herstellung einer getrockneten Kohlenhydratpräparation aus einer wasserhaltigen kristallinen Kohlenhydrat-Ausgangspräparation und Kohlenhydratpartikeln vor, wobei eine wasserhaltige 0,4 bis 22,5 Gew.- % Wasser (bezogen auf Gesamtgewicht der Kohlenhydrat-Ausgangspräparation) aufweisende kristalline Kohlenhydrat-Ausgangspräparation, vorgelegt in einem Trocknungsreaktor, und Kohlenhydratpartikel in einem Verfahrensschritt a) bereitgestellt werden und die Kohlenhydratpartikel in einem Verfahrensschritt b) in die in dem Trocknungsreaktor vorgelegte kristalline wasserhaltige Kohlenhydrat-Ausgangspräparation eindosiert werden, wobei die erhaltene Mischung in einem Verfahrensschritt c) homogenisiert wird und so mithilfe der in die wasserhaltige kristalline Kohlenhydrat-Ausgangspräparation eindosierten Kohlenhydratpartikel eine getrocknete Kohlenhydratpräparation erhalten wird.

In vorteilhafter und besonders bevorzugter Weise resultiert das erfindungsgemäße Verfahren in einer einfachen und effizienten, insbesondere kosteneffizienten, Prozessführung. Insbesondere wird durch die erfindungsgemäße Verfahrensweise eine getrocknete Kohlenhydratpräparation erhalten, die riesel-, transport-, dosier-, und lagerfähig ist, insbesondere riesel-, transport-, dosier-, und lagerfähiger als im Stand der Technik bekannte getrocknete Kohlenhydratpräparationen. Bevorzugt ist die durch das erfindungsgemäße Verfahren erhaltene getrocknete Kohlenhydratpräparation einfacher abzupacken als im Stand der Technik bekannte getrocknete Kohlenhydratpräparationen, die beispielsweise hermetisch verschlossene Verpackungen benötigen. Bevorzugt kommt das erfindungsgemäße Verfahren vorteilhafterweise mit möglichst wenigen bis gar keinen Zusatzstoffen oder Hilfsstoffen, insbesondere Rieselhilfsmitteln, aus, um eine getrocknete Kohlenhydratpräparation zu erhalten.

Weiterhin ist die vorliegende Erfindung vorteilhaft, da die erhaltene Kohlenhydratpräparation auch bei einer nachträglichen Wasseraufnahme nicht verklebt und die erhaltene Kohlenhydratpräparation somit lagerstabil ist.

Die vorliegende Erfindung ist auch insofern vorteilhaft, als dass der Einsatz von Zusatzstoffen oder Hilfsstoffen vermieden werden kann, wobei dennoch eine lagerstabile und dosierfähige getrocknete Kohlenhydratpräparation erhalten wird. Des Weiteren ist die vorliegende Erfindung insofern vorteilhaft, als dass die Lagerstabilität der aus dem erfindungsgemäßen Verfahren erhaltenen Kohlenhydratpräparation nicht an ein aufwändiges Verpackungskonzept gebunden ist, um eine nachträgliche Wasseraufnahme und damit eine Verklumpung zu vermeiden.

Die vorliegende Erfindung ermöglicht es mit im Vergleich zum Stand der Technik vergleichsweise einfachem technischem Aufwand mittels eines energiearmen Verfahrens aus einer schwer zu trocknenden Kohlenhydrat-Ausgangspräparation eine lagerstabile getrocknete Kohlenhydratpräparation zu erhalten. Bevorzugt werden Misch- und Dosiervorrichtungen sowie ein Trocknungsreaktor, insbesondere ein verschließbarer Trocknungsreaktor, verwendet. Bevorzugt ist ein geringer Unterdruck im Trocknungsreaktor für eine erfindungsgemäße Trocknung besonders geeignet. Bevorzugt ermöglicht es die vorliegende Erfindung, dass die zu trocknende Kohlenhydrat-Ausgangspräparation Mischungen von gut-löslichen Kohlenhydraten und schlecht-löslichen Kohlenhydraten umfasst, was insbesondere aus sensorischen Gründen vorteilhaft ist. Bevorzugt ermöglicht es die vorliegende Erfindung außerdem, dass im Vergleich zum Stand der Technik aus einer zur Kristallisation neigenden wasserhaltigen Kohlenhydrat-Ausgangspräparation mit wenigen und einfachen Verfahrensschritten eine besser vermarktungsfähige erfindungsgemäße getrocknete Kohlenhydratpräparation erhalten wird. Besonders bevorzugt umfasst ein erfindungsgemäßes Verfahren lediglich vergleichsweise einfache Verfahrensschritte, nämlich ein Bereitstellen, Dosieren, Homogenisieren und optional Abpacken. Die erfindungsgemäßen Kohlenhydratpräparationen zeigen im Vergleich zum Stand der Technik eine gute Lagerstabilität und bleiben auch bei nachträglicher Wasseraufnahme durch Sorption rieselfähig. Insbesondere ist es durch das erfindungsgemäße Verfahren möglich, eine einer traditionellen Soft-Sugar Kohlenhydratpräparation sensorisch vergleichbare getrocknete Kohlenhydratpräparation herzustellen, ohne dass die getrocknete Kohlenhydratpräparation dieselben schwer zu verarbeitenden Eigenschaften aufweist.

In einer bevorzugten Ausführung der vorliegenden Erfindung wird das erfindungsgemäße Verfahren kontinuierlich, semi-kontinuierlich oder batch-weise durchgeführt.

In einer bevorzugten Ausführung der vorliegenden Erfindung ist der in den Verfahrensschritten a) und b) und bevorzugt auch Verfahrensschritt c) eingesetzte Trocknungsreaktor ein Behälter, insbesondere ein Behälter mit mindestens einer Mischvorrichtung oder ein Behälter, der zur Ausführung von, bevorzugt regelmäßigen, Mischbewegungen ausgebildet ist, insbesondere in der Lage ist, rotierende oder schwenkende Bewegungen durchzuführen, insbesondere wobei in dem Behälter, eine oder mehrere Einbauten vorhanden sind.

In einer bevorzugten Ausführung der vorliegenden Erfindung ist der in den Verfahrensschritten a) und b) eingesetzte Trocknungsreaktor insbesondere
a) ein senkrecht oder im Wesentlichen aufrecht stehender Trockner mit zylindrischer Bauform, vorzugweise mit einer oder mehreren senkrecht eingebauten Rührwellen, oder
b) ein Trockner mit zylindrischem und konischem Anteil des Trocknungsgehäuses oder ein vollständig konischer Trockner, vorzugsweise mit senkrecht angeordneten Rührwelle, oder
c) ein waagerecht oder im Wesentlichen horizontal oder geneigt angeordneter Trockner, vorzugsweise mit einer oder mehreren horizontal oder waagerecht angeordneten Rührwellen, oder
d) ein sich bewegender, das heisst rotierender oder asymmetrisch sich bewegender Trockner mit oder ohne Einbauten.

In einer bevorzugten Ausführung der vorliegenden Erfindung können die erfindungsgemäß einsetzbaren Trocknungsreaktoren mit feststehenden oder sich bewegenden zum Beispiel rotierenden Einbauten, insbesondere Zerhackern (Chopper), zur besseren Durchmischung des zu trocknenden Materials versehen sein.

In einer bevorzugten Ausführung der vorliegenden Erfindung können die Trockner senkrecht, waagerecht oder in einem beliebigen Winkel zur Erdoberfläche angeordnet sein.

In einer bevorzugten Ausführung der vorliegenden Erfindung können die Trockner, insbesondere die Trocknergehäuse, symmetrische oder asymmetrische Bewegungen vollziehen.

In einer bevorzugten Ausführung der vorliegenden Erfindung können die Trockner eine symmetrische, oder asymmetrische Bauform aufweisen oder beliebige Kombination daraus, insbesondere doppel- oder dreifach-zylindrische Bauform aufweisen. Bevorzugt können die Trockner insbesondere als runder oder ellipsoider oder konischer Zylinder ausgebildet sein.

In einer bevorzugten Ausführung der vorliegenden Erfindung können die Trockner eine oder mehrere Rührwellen haben.

In einer bevorzugten Ausführung der vorliegenden Erfindung können die Rührwellen eine feststehende Rotationsachse oder sich bewegende Position besitzen.

In einer bevorzugten Ausführung der vorliegenden Erfindung können die Trockner im Vakuum oder bei leichtem Überdruck betrieben werden.

In einer bevorzugten Ausführung der vorliegenden Erfindung sind die Trockner mit Heiz- und/oder Kühlsystemen versehen, um eine bestimmte Temperatur einzustellen oder zu kontrollieren.

In einer bevorzugten Ausführung der vorliegenden Erfindung weist die in Verfahrensschritt a) bereitgestellte wasserhaltige kristalline Kohlenhydrat-Ausgangspräparation 0,4 bis 22,5 Gew.-%, insbesondere 0,5 bis 22,5 Gew.-%, insbesondere 0,7 bis 20,0 Gew.-%, insbesondere 1,0 bis 18,0 Gew.-%, insbesondere 1,5 bis 15,0 Gew.-%, insbesondere 2,0 bis 13,0 Gew.-%, insbesondere 2,5 bis 12,0 Gew.-%, insbesondere 4,0 bis 10,0 Gew.-%, insbesondere 6,0 bis 8,0 Gew.-%, insbesondere 0,4 bis 4,0 Gew.-%, insbesondere 0,4 bis 3,5 Gew.- %, insbesondere 0,4 bis 3,0 Gew.-%, insbesondere 0,4 bis 2,5 Gew.-%, insbesondere 0,6 bis 2,0 Gew.-%, insbesondere 0,8 bis 1,5 Gew.-%, insbesondere 1,0 Gew.-%, Wasser (jeweils bezogen auf Gesamtgewicht der Kohlenhydrat-Ausgangspräparation) auf. Dieser Wassergehalt ist in Form von einem Flüssigkeitsfilm auf den festen kristallinen Kohlenhydratpartikeln der Kohlenhydrat-Ausgangspräparation vorhanden. Sofern das Kohlenhydrat oder die Kohlenhydrate der Kohlenhydrat-Ausgangspräparation Kristallwasser-haltig ist oder sind, wird der Wassergehalt durch den Wassergehalt des Flüssigkeitsfilm und den Kristallwassergehalt gebildet.

Besonders bevorzugt ist die in Verfahrensschritt a) bereitgestellte wasserhaltige kristalline Kohlenhydrat-Ausgangspräparation eine mit einem Sirupfilm überzogene kristalline Masse kristalliner Kohlenhydratpartikel, insbesondere in fester oder halbfester Form.

Besonders bevorzugt ist die in Verfahrensschritt a) bereitgestellte wasserhaltige kristalline Kohlenhydrat-Ausgangspräparation eine Suspension kristalliner Kohlenhydratpartikel in einem wässrigen Medium, insbesondere ein Kristallmagma.

In einer bevorzugten Ausführung der vorliegenden Erfindung umfasst die in Verfahrensschritt a) bereitgestellte wasserhaltige Kohlenhydrat-Ausgangspräparation mindestens ein schlecht-lösliches Kohlenhydrat mit einer Löslichkeit bei 20 °C in Wasser von 5,0 bis 53,0 g/100 g, insbesondere 14,5 bis 53,0 g/100 g, insbesondere 15,2 bis 53,0 g/100 g, insbesondere 29,0 bis 53,0 g/100 g, insbesondere 47,1 bis 53,0 g/100 g, insbesondere 5,0 g/100 g, insbesondere 14,5 g/100 g, insbesondere 15,2 g/100 g, insbesondere 29,0 g/100 g, insbesondere 47,1 g/100 g, und mindestens ein gut-lösliches Kohlenhydrat mit einer Löslichkeit bei 20 °C in Wasser von mehr als 53,0 g/100 g, insbesondere von mindestens 58,6 g/100 g, insbesondere von mindestens 66,7 g/100 g, insbesondere von mindestens 68,7 g/100 g, insbesondere von mindestens 70,0 g/100 g, insbesondere von mindestens 78,9 g/100 g.

In einer bevorzugten Ausführung der vorliegenden Erfindung umfasst die wasserhaltige Kohlenhydrat-Ausgangspräparation mindestens ein schlecht-lösliches Kohlenhydrat ausgewählt aus der Gruppe bestehend aus 1,1-GPM (1-O-α-D-Glucopyranosyl-D-mannit), Isomaltulose, Glucose und Mannit.

In einer bevorzugten Ausführung der vorliegenden Erfindung umfasst die wasserhaltige Kohlenhydrat-Ausgangspräparation mindestens ein gut-lösliches Kohlenhydrat ausgewählt aus der Gruppe bestehend aus Saccharose, 1,6-GPS (6-O-α-D-Glucopyranosyl-D-sorbit), Fructose, Trehalulose und Sorbit.

In einer bevorzugten Ausführung der vorliegenden Erfindung umfasst die wasserhaltige Kohlenhydrat-Ausgangspräparation mindestens ein schlecht-lösliches Kohlenhydrat ausgewählt aus der Gruppe bestehend aus 1,1-GPM, Isomaltulose, Glucose und Mannit und ein gut-lösliches Kohlenhydrat ausgewählt aus der Gruppe bestehend aus Saccharose, 1,6-GPS, Fructose, Trehalulose und Sorbit.

In einer bevorzugten Ausführung der vorliegenden Erfindung ist das schlecht-lösliche Kohlenhydrat Glucose. In einer bevorzugten Ausführung der vorliegenden Erfindung liegt die Glucose als Bestandteil einer Kohlenhydrat-haltigen Komponente ausgewählt aus Invertzucker, Karamellzuckersirup und Rohrzuckersirup vor.

In einer bevorzugten Ausführung der vorliegenden Erfindung umfasst die wasserhaltige Kohlenhydrat-Ausgangspräparation mindestens ein schlecht-lösliches Kohlenhydrat ausgewählt aus der Gruppe bestehend aus 1,1-GPM, Isomaltulose, Glucose und Mannit mit einer Löslichkeit bei 20 °C in Wasser von 5,0 bis 53,0 g/100 g, insbesondere 14, 5 bis 53,0 g/100 g, insbesondere 15,2 bis 53,0 g/100 g, insbesondere 29,0 bis 53,0 g/100 g, insbesondere 47,1 bis 53,0 g/100 g, insbesondere 5,0 g/ 100 g, insbesondere 14,5 g/100 g, insbesondere 15,2 g/100 g, insbesondere 29,0 g/100 g, insbesondere 47,1 g/100 g, und mindestens ein gut-lösliches Kohlenhydrat ausgewählt aus der Gruppe bestehend aus Saccharose, 1,6-GPS, Fructose, Trehalulose und Sorbit mit einer Löslichkeit bei 20 °C in Wasser von mehr als 53,0 g/100 g, insbesondere von mindestens 58,6 g/100 g, insbesondere von mindestens 66,7 g/100 g, insbesondere von mindestens 68,7 g/ 100 g, insbesondere von mindestens 70,0 g/100 g, insbesondere von mindestens 78,9 g/100 g.

In einer bevorzugten Ausführung der vorliegenden Erfindung ist die in Verfahrensschritt a) bereitgestellte wasserhaltige kristalline Kohlenhydrat-Ausgangspräparation eine Saccharose-haltige Ausgangspräparation.

In einer bevorzugten Ausführung der vorliegenden Erfindung ist die in Verfahrensschritt a) bereitgestellte wasserhaltige Kohlenhydrat-Ausgangspräparation eine Saccharose-haltige Ausgangspräparation, die Saccharose, vorzugsweise Saccharose in kristalliner Form, aufweist, insbesondere aus dieser besteht oder mindestens einen weiteren Stoff enthält.

In einer bevorzugten Ausführung der vorliegenden Erfindung ist die in Verfahrensschritt a) bereitgestellte wasserhaltige Kohlenhydrat-Ausgangspräparation eine Saccharose-haltige Ausgangspräparation, die Saccharose, vorzugsweise Saccharose in kristalliner Form, Glucose und Fructose aufweist, insbesondere besteht aus diesen.

In einer bevorzugten Ausführung der vorliegenden Erfindung umfasst die in Verfahrensschritt a) bereitgestellte wasserhaltige kristalline Kohlenhydratpräparation Saccharose, insbesondere 91,0 bis 99,5 Gew.-%, insbesondere 92,0 bis 98,5 Gew.-%, insbesondere 92,5 bis 98,2 Gew.-%, insbesondere 92,7 bis 98,1 Gew.-%, insbesondere 93,0 bis 98,1 Gew.-% (jeweils bezogen auf das Gesamtgewicht der Trockensubstanz der in Verfahrensschritt a) bereitgestellten Kohlenhydratpräparation), Glucose, insbesondere 0,30 bis 1,00 Gew.-%, 0,40 bis 1,00 Gew.-%, insbesondere 0,44 bis 0,77 Gew.-%, insbesondere 0,46 bis 0,77 Gew.-%, insbesondere 0,48 bis 0,77 Gew.-% (jeweils bezogen auf das Gesamtgewicht der Trockensubstanz der in Verfahrensschritt a) bereitgestellten Kohlenhydratpräparation), Fructose, insbesondere 0,10 bis 0,70 Gew.-%, 0,30 bis 0,60 Gew.-%, insbesondere 0,41 bis 0,55 Gew.-%, insbesondere 0,43 bis 0,54 Gew.-%, insbesondere 0,44 bis 0,50 Gew.-% (jeweils bezogen auf das Gesamtgewicht der Trockensubstanz der in Verfahrensschritt a) bereitgestellten Kohlenhydratpräparation) und Wasser, sowie optional, Nebenkomponenten, insbesondere 0,40 bis 4,50 Gew.-%, insbesondere 0,40 bis 4,36 Gew.-%, insbesondere 0,42 bis 4,24 Gew.-%, insbesondere 0,40 bis 4,04 Gew.-% (jeweils bezogen auf das Gesamtgewicht der Trockensubstanz der in Verfahrensschritt a) bereitgestellten Kohlenhydratpräparation).

In einer bevorzugten Ausführung der vorliegenden Erfindung ist die in Verfahrensschritt a) bereitgestellte wasserhaltige Kohlenhydrat-Ausgangspräparation eine wasserhaltige Saccharose-haltige Zusammensetzung umfassend kristalline Saccharose und mindestens eine weitere Kohlenhydrat-haltige Komponente ausgewählt aus der Gruppe bestehend aus Invertzucker, Karamellzuckersirup und Rohrzuckersirup.

In einer bevorzugten Ausführung der vorliegenden Erfindung ist die in Verfahrensschritt a) bereitgestellte wasserhaltige Kohlenhydrat-Ausgangspräparation eine Saccharose-haltige Ausgangspräparation, die Saccharose, vorzugsweise Saccharose in kristalliner Form, Glucose und Fructose in Form von Invertzuckersirup aufweist, insbesondere besteht aus diesen.

In einer bevorzugten Ausführung der vorliegenden Erfindung ist die in Verfahrensschritt a) bereitgestellte wasserhaltige Kohlenhydrat-Ausgangspräparation eine Saccharose-haltige Ausgangspräparation, die Saccharose aus Zuckerrübe in kristalliner Form und Saccharose in Form von Karamellzuckersirup aufweist, insbesondere besteht aus diesen.

In einer bevorzugten Ausführung der vorliegenden Erfindung ist die in Verfahrensschritt a) bereitgestellte wasserhaltige Kohlenhydrat-Ausgangspräparation eine Saccharose-haltige Ausgangspräparation, die Saccharose aus Zuckerrübe in kristalliner Form und Saccharose aus Zuckerrohr in Form von Rohrzuckersirup aufweist, insbesondere besteht aus diesen.

In einer bevorzugten Ausführung der vorliegenden Erfindung umfasst die in Verfahrensschritt a) bereitgestellte wasserhaltige Saccharose-haltige Ausgangspräparation Saccharose, vorzugsweise Saccharose in kristalliner Form, und Invertzuckersirup oder Saccharose, vorzugsweise Saccharose in kristalliner Form, und Karamellzuckersirup oder Saccharose, vorzugsweise Saccharose in kristalliner Form, und Rohrzuckersirup oder Saccharose, vorzugsweise Saccharose in kristalliner Form, und Rohrzuckersirup und Karamellzuckersirup.

In einer bevorzugten Ausführung der vorliegenden Erfindung umfasst die in Verfahrensschritt a) bereitgestellte wasserhaltige Saccharose-haltige Ausgangspräparation Saccharose aus Zuckerrübe, insbesondere besteht aus dieser.

In einer bevorzugten Ausführung der vorliegenden Erfindung umfasst die in Verfahrensschritt a) bereitgestellte wasserhaltige Saccharose-haltige Ausgangspräparation Saccharose aus Zuckerrohr, insbesondere besteht aus dieser.

In einer bevorzugten Ausführung der vorliegenden Erfindung umfasst die in Verfahrensschritt a) bereitgestellte wasserhaltige Saccharose-haltige Ausgangspräparation Saccharose, insbesondere Saccharose aus Zuckerrübe und Glucose oder Saccharose aus Zuckerrohr und Glucose, insbesondere besteht aus diesen.

In einer bevorzugten Ausführung der vorliegenden Erfindung umfasst die in Verfahrensschritt a) bereitgestellte wasserhaltige Saccharose-haltige Ausgangspräparation Saccharose, insbesondere Saccharose aus Zuckerrübe und Fructose oder Saccharose aus Zuckerrohr und Fructose, insbesondere besteht aus diesen.

In einer bevorzugten Ausführung der vorliegenden Erfindung umfasst die in Verfahrensschritt a) bereitgestellte wasserhaltige Saccharose-haltige Ausgangspräparation Saccharose, insbesondere Saccharose aus Zuckerrübe und Glucose und Fructose oder Saccharose aus Zuckerrohr und Glucose und Fructose, insbesondere besteht aus diesen.

In einer bevorzugten Ausführung der vorliegenden Erfindung umfasst die in Verfahrensschritt a) bereitgestellte wasserhaltige Saccharose-haltige Ausgangspräparation, insbesondere Saccharose aus Zuckerrübe oder Zuckerrohr, Glucose und/oder Fructose, insbesondere in Form von Invertzuckersirup, Saccharose in Form von Karamellzuckersirup und Saccharose aus Zuckerrohr in Form von Rohrzuckersirup, insbesondere besteht aus diesen.

In einer besonders bevorzugten Ausführung der vorliegenden Erfindung umfasst die in Verfahrensschritt a) bereitgestellte wasserhaltige kristalline Saccharose-haltige Ausgangspräparation insbesondere 70,0 bis 98,0 Gew.-%, insbesondere 80 bis 98,0 Gew.-%, insbesondere 90,0 bis 98,0 Gew.-%, insbesondere 95,0 bis 98,0 Gew.-% Saccharose, insbesondere kristalline Saccharose (jeweils bezogen auf Gesamtgewicht der Trockensubstanz der wasserhaltigen Saccharose-Ausgangspräparation).

In einer besonders bevorzugten Ausführung der vorliegenden Erfindung umfasst die in Verfahrensschritt a) bereitgestellte wasserhaltige Saccharose-haltige Ausgangspräparation Saccharose, insbesondere 80,0 bis 98,0 Gew.-%, insbesondere 90,0 bis 98,0 Gew.-%, insbesondere 95,0 bis 98,0 Gew.-% (jeweils bezogen auf Gesamtgewicht der wasserhaltigen Saccharose-Ausgangspräparation), Invertzuckersirup, insbesondere 1,40 bis 12,0 Gew.-%, insbesondere 1,40 bis 6,0 Gew.-%, insbesondere 1,40 bis 3,0 Gew.-% (jeweils bezogen auf das Gesamtgewicht der wasserhaltigen Saccharose-Ausgangspräparation) und Karamellzuckersirup, insbesondere 0,60 bis 8,0 Gew.-%, insbesondere 0,6 bis 4,0 Gew.-%, insbesondere 0,6 bis 2,0 Gew.-% (jeweils bezogen auf das Gesamtgewicht der wasserhaltigen Saccharose-Ausgangspräparation), insbesondere besteht aus diesen.

In einer besonders bevorzugten Ausführung der vorliegenden Erfindung umfasst die in Verfahrensschritt a) bereitgestellte wasserhaltige Saccharose-haltige Ausgangspräparation Saccharose, insbesondere 80,0 bis 92,2 Gew.-%, insbesondere 85,0 bis 92,2 Gew.-%, insbesondere 90,0 bis 92,2 Gew.-% (jeweils bezogen auf Gesamtgewicht der wasserhaltigen Saccharose-Ausgangspräparation), Rohrzuckersirup, insbesondere 1,8 bis 4,6 Gew.-%, insbesondere 1,80 bis 3,4 Gew.-%, insbesondere 1,8 bis 2,40 Gew.-% (jeweils bezogen auf das Gesamtgewicht der wasserhaltigen Saccharose-Ausgangspräparation) und Karamellzuckersirup, insbesondere 6,00 bis 15,4 Gew.-%, insbesondere 6,0 bis 11,6 Gew.-%, insbesondere 6,0 bis 7,6 Gew.-% (jeweils bezogen auf das Gesamtgewicht der wasserhaltigen Saccharose-Ausgangspräparation), insbesondere besteht aus diesen.

In einer besonders bevorzugten Ausführungsform betrifft die vorliegende Erfindung ein vorgenanntes Verfahren, wobei die in Verfahrensschritt a) bereitgestellte wasserhaltige kristalline Kohlenhydrat-Ausgangspräparation eine in Wasser gelöste Isomalt-Ausgangspräparation oder eine in Wasser gelöste Isomaltulose- und Trehalulose-haltige Ausgangspräparation ist.

In einer besonders bevorzugten Ausführungsform betrifft die vorliegende Erfindung ein vorgenanntes Verfahren, wobei die in Verfahrensschritt a) bereitgestellte wasserhaltige kristalline Kohlenhydrat-Ausgangspräparation eine in Wasser gelöste Isomalt-Ausgangspräparation ist.

In einer besonders bevorzugten Ausführungsform der vorliegenden Erfindung ist das Isomalt Isomalt ST oder Isomalt GS.

In einer besonders bevorzugten Ausführungsform der vorliegenden Erfindung weist die in Verfahrensschritt a) bereitgestellte wasserhaltige Isomalt-Ausgangspräparation einen 1,1-GPM (1-O-alpha-D-glucopyranosyl-D-mannit)- und 1,6-GPS (6-O-alpha-D-glucopyranosyl-D-sorbit)-Gehalt von 90,0 bis 100,0 Gew.-%, insbesondere von 92,0 bis 99,0 Gew.-%, insbesondere von 93,0 bis 98,0 Gew.-%, insbesondere von 95,0 bis 100,0 Gew.-% (bezogen auf Gesamtgewicht der Trockensubstanz der wasserhaltigen Isomalt-Ausgangspräparation) auf.

In einer besonders bevorzugten Ausführungsform der vorliegenden Erfindung weist die in Verfahrensschritt a) bereitgestellte Isomalt-Ausgangspräparation einen 1,1-GPM (1-O-alpha-D-glucopyranosyl-D-mannit)-Gehalt von 45,0 bis 50,0 Gew.-% und einen 1,6-GPS (6-O-alpha-D-glucopyranosyl-D-sorbit)-Gehalt von 50 bis 55 Gew.-% (jeweils bezogen auf Gesamtgewicht der Trockensubstanz der wasserhaltigen Isomalt-Ausgangspräparation) auf.

In einer besonders bevorzugten Ausführungsform der vorliegenden Erfindung weist die in Verfahrensschritt a) bereitgestellte Isomalt-Ausgangspräparation einen 1,1-GPM (1-O-alpha-D-glucopyranosyl-D-mannit)-Gehalt von 20,0 bis 30,0 Gew.-% und einen 1,6-GPS (6-O-alpha-D-glucopyranosyl-D-sorbit)-Gehalt von 70,0 bis 80,0 Gew.-% (jeweils bezogen auf Gesamtgewicht der Trockensubstanz der wasserhaltigen Isomalt-Ausgangspräparation) auf.

In einer besonders bevorzugten Ausführungsform der vorliegenden Erfindung weist die in Verfahrensschritt a) bereitgestellte Isomalt-Ausgangspräparation 1,1-GPS (1-O-alpha-D-glucopyranosyl-D-sorbit), Sorbit, Mannit, oder GPI (Glucopyranosyl-idit) oder eine Mischung von zwei oder mehr davon auf.

In einer besonders bevorzugten Ausführungsform der vorliegenden Erfindung weist die in Verfahrensschritt a) bereitgestellte Isomalt-Ausgangspräparation Sorbit, Mannit, oder GPI jeweils in einer Menge von 0,00 bis 0,20 Gew.-%, insbesondere von 0,04 bis 0,17 Gew.-% auf (jeweils bezogen auf Gesamtgewicht der Trockensubstanz der wasserhaltigen Isomalt-Ausgangspräparation, bestimmt mittels GC).

In einer besonders bevorzugten Ausführungsform der vorliegenden Erfindung weist die in Verfahrensschritt a) bereitgestellte Isomalt-Ausgangspräparation 1,1-GPS in einer Menge von 0,20 bis 0,70, insbesondere von 0,30 bis 0,60 Gew.-% auf (jeweils bezogen auf Gesamtgewicht der Trockensubstanz der wasserhaltigen Isomalt-Ausgangspräparation, bestimmt mittels GC).

In einer besonders bevorzugten Ausführungsform der vorliegenden Erfindung weist die in Verfahrensschritt a) bereitgestellte Isomalt-Ausgangspräparation einen pH-Wert von 4,0 bis 4,7, insbesondere 4,1 bis 4,5 auf.

In einer besonders bevorzugten Ausführungsform betrifft die vorliegende Erfindung ein vorgenanntes Verfahren, wobei die in Verfahrensschritt a) bereitgestellte Kohlenhydrat-Ausgangspräparation eine in Wasser gelöste Isomaltulose- und Trehalulose-haltige Ausgangspräparation ist.

In einer besonders bevorzugten Ausführungsform betrifft die vorliegende Erfindung ein vorgenanntes Verfahren, wobei die in Verfahrensschritt a) bereitgestellte Kohlenhydrat-Ausgangspräparation eine in Wasser gelöste Isomaltulose- und Trehalulose-haltige Ausgangspräparation ist, wobei deren Isomaltulosegehalt von 65,0 bis 90,0 Gew.-%, insbesondere von 70,0 bis 90,0 Gew.-%, insbesondere von 75,0 bis 88,0 Gew.-%, insbesondere von 75,0 bis 85,0 Gew.-% und der Trehalulosegehalt von 5,0 bis 15,0 Gew.-%, insbesondere von 6,5 bis 13,0 Gew.-%, insbesondere von 6,0 bis 12,0 Gew.-%, insbesondere von 7,0 bis 10,0 Gew.-% beträgt (jeweils bezogen auf Gesamtgewicht der Trockensubstanz der wasserhaltigen Isomaltulose- und Trehalulose-haltigen Ausgangspräparation).

In einer besonders bevorzugten Ausführungsform betrifft die vorliegende Erfindung ein vorgenanntes Verfahren, wobei die in Verfahrensschritt a) bereitgestellte Kohlenhydrat-Ausgangspräparation eine in Wasser gelöste Isomaltulose- und Trehalulose-haltige Ausgangspräparation ist, wobei deren Isomaltulosegehalt von 65,0 bis 90,0 Gew.-%, insbesondere von 70,0 bis 90,0 Gew.-%, insbesondere von 75,0 bis 88,0 Gew.-%, insbesondere von 75,0 bis 85,0 Gew.-% und der Trehalulosegehalt von 5,0 bis 15,0 Gew.-%, insbesondere von 6,5 bis 13,0 Gew.-%, insbesondere von 6,0 bis 12,0 Gew.-%, insbesondere von 7,0 bis 10,0 Gew.-% beträgt (jeweils bezogen auf Gesamtgewicht der Trockensubstanz der wasserhaltigen Isomaltulose- und Trehalulose-haltigen Ausgangspräparation), und wobei diese Fructose, Glucose und Saccharose, sowie gegebenenfalls Isomelezitose und Isomaltose aufweist.

In einer besonders bevorzugten Ausführungsform betrifft die vorliegende Erfindung ein vorgenanntes Verfahren, wobei die in Verfahrensschritt a) bereitgestellte Kohlenhydrat-Ausgangspräparation eine in Wasser gelöste Isomaltulose- und Trehalulose-haltige Ausgangspräparation ist, wobei deren Isomaltulosegehalt von 65,0 bis 90,0 Gew.-%, insbesondere von 70,0 bis 90,0 Gew.-%, insbesondere von 75,0 bis 88,0 Gew.-%, insbesondere von 75,0 bis 85,0 Gew.-% und der Trehalulosegehalt von 5,0 bis 15,0 Gew.-%, insbesondere von 6,5 bis 13,0 Gew.-%, insbesondere von 6,0 bis 12,0 Gew.-%, insbesondere von 7,0 bis 10,0 Gew.-% beträgt, und wobei diese einen Fructosegehalt von 2,0 bis 4,0 Gew.-%, einen Glucosegehalt von 1,0 bis 3,0 Gew.-% und einen Saccharosegehalt von 0,1 bis 12,0 Gew.-%, insbesondere 0,2 bis 3,0 Gew.- % (jeweils bezogen auf Gesamtgewicht der Trockensubstanz der wasserhaltigen Isomaltulose- und Trehalulose-haltigen Ausgangspräparation), sowie gegebenenfalls Isomelezitose und Isomaltose aufweist.

In einer bevorzugten Ausführung der vorliegenden Erfindung ist die wasserhaltige kristalline Kohlenhydrat-Ausgangspräparation teil-kristallin oder voll-kristallin.

In einer bevorzugten Ausführung der vorliegenden Erfindung wird in einem Verfahrensschritt a1) vor Verfahrensschritt a) die wasserhaltige Kohlenhydrat-Ausgangspräparation aus mindestens zwei verschiedenen Kohlenhydraten hergestellt.

In einer bevorzugten Ausführung der vorliegenden Erfindung wird die wasserhaltige kristalline Kohlenhydrat-Ausgangspräparation in einem Verfahrensschritt a1) durch Vermengen von mindestens zwei Kohlenhydraten, insbesondere zwei Kohlenhydraten mit unterschiedlicher Löslichkeit, insbesondere mindestens einem Kohlenhydrat mit guter und mindestens einem Kohlenhydrat mit schlechter Löslichkeit hergestellt.

In einer bevorzugten Ausführung der vorliegenden Erfindung wird die wasserhaltige kristalline Kohlenhydrat-Ausgangspräparationen in einem Verfahrensschritt a1) bei einer Temperatur von 50 bis 70 °C, insbesondere 55 bis 65, insbesondere 60 °C hergestellt.

In einer bevorzugten Ausführung der vorliegenden Erfindung sind die in Verfahrensschritt a1) eingesetzten mindestens zwei Kohlenhydrate ausgewählt aus der Gruppe bestehend aus Saccharose, insbesondere Saccharose aus Zuckerrübe oder Zuckerrohr, Glucose und/oder Fructose, insbesondere in Form von Invertzuckersirup, Saccharose in Form von Karamellzuckersirup und Saccharose aus Zuckerrohr in Form von Rohrzuckersirup.

In einer bevorzugten Ausführung der vorliegenden Erfindung sind die in Verfahrensschritt a) bereitgestellten Kohlenhydrate der wasserhaltigen kristallinen Kohlenhydrat-Ausgangspräparation und der Kohlenhydratpartikel identische Kohlenhydrate.

In einer bevorzugten Ausführung der vorliegenden Erfindung umfassen die in Verfahrensschritt a) bereitgestellten Kohlenhydratpartikel eine Saccharose-haltige Zusammensetzung, eine Isomaltulose- und Trehalulose-haltige Zusammensetzung und/oder eine Isomalt-haltige Zusammensetzung oder bestehen aus diesen.

In einer bevorzugten Ausführung der vorliegenden Erfindung bestehen die Kohlenhydratpartikel aus Saccharose oder Isomaltulose und Trehalulose oder Isomalt oder einer Mischung davon.

In einer bevorzugten Ausführung der vorliegenden Erfindung bestehen die Kohlenhydratpartikel aus Saccharose, insbesondere in kristalliner Form, insbesondere in Form eines Pulvers.

In einer bevorzugten Ausführung der vorliegenden Erfindung bestehen die Kohlenhydratpartikel aus Isomaltulose und Trehalulose, insbesondere in kristalliner Form, insbesondere in Form eines Pulvers.

In einer bevorzugten Ausführung der vorliegenden Erfindung bestehen die Kohlenhydratpartikel aus Isomalt, insbesondere in kristalliner Form, insbesondere in Form eines Pulvers.

In einer bevorzugten Ausführung der vorliegenden Erfindung weisen die in Verfahrensschritt a) bereitgestellten Kohlenhydratpartikel einen Anteil von mindestens 80 Gew.-% an Kohlenhydratkristallen mit einem Durchmesser von kleiner oder gleich 100 µm, insbesondere kleiner oder gleich 80 µm, insbesondere kleiner oder gleich 70 µm, insbesondere kleiner oder gleich 60 µm, insbesondere kleiner oder gleich 50 µm, insbesondere kleiner oder gleich 40 µm, insbesondere kleiner oder gleich 32 µm, insbesondere kleiner oder gleich 20 µm, insbesondere kleiner oder gleich 10 µm auf (jeweils bezogen auf Gesamtgewicht der Kohlenhydratpartikel).

In einer bevorzugten Ausführung der vorliegenden Erfindung weisen die in Verfahrensschritt a) bereitgestellten Kohlenhydratpartikel einen Anteil von mindestens 80 Gew.-%, insbesondere mindestens 85 Gew.-%, insbesondere mindestens 90 Gew.-%, insbesondere mindestens 95 Gew.- %, an Kohlenhydratkristallen mit einem Durchmesser von kleiner oder gleich 100 µm auf.

In einer bevorzugten Ausführung der vorliegenden Erfindung weisen die in Verfahrensschritt a) bereitgestellten Kohlenhydratpartikel einen Anteil von mindestens 80 Gew.-%, insbesondere mindestens 85 Gew.-%, insbesondere mindestens 90 Gew.-%, insbesondere mindestens 95 Gew.- %, an Kohlenhydratkristallen mit einem Durchmesser von kleiner oder gleich 100 µm auf und einen Anteil von mindestens 70 Gew.-% an Kohlenhydratpartikeln mit einem Durchmesser von höchstens 32 µm.

In einer bevorzugten Ausführung der vorliegenden Erfindung weisen die in Verfahrensschritt a) bereitgestellten Kohlenhydratpartikel einen Anteil von mindestens 80 Gew.-%, insbesondere mindestens 85 Gew.-%, insbesondere mindestens 90 Gew.-%, insbesondere mindestens 95 Gew.- %, an Kohlenhydratkristallen mit einem Durchmesser von kleiner oder gleich 50 µm auf.

In einer bevorzugten Ausführung der vorliegenden Erfindung weisen die in Verfahrensschritt a) bereitgestellten Kohlenhydratpartikel einen Anteil von mindestens 80 Gew.-%, insbesondere mindestens 85 Gew.-%, insbesondere mindestens 90 Gew.-%, insbesondere mindestens 95 Gew.- %, an Kohlenhydratkristallen mit einem Durchmesser von kleiner oder gleich 100 µm, insbesondere kleiner oder gleich 80 µm, insbesondere kleiner oder gleich 70 µm, insbesondere kleiner oder gleich 60 µm, insbesondere kleiner oder gleich 50 µm, insbesondere kleiner oder gleich 40 µm, insbesondere kleiner oder gleich 32 µm, insbesondere kleiner oder gleich 20 µm, insbesondere kleiner oder gleich 10 µm auf (jeweils bezogen auf Gesamtgewicht der Kohlenhydratpartikel).

In einer bevorzugten Ausführung der vorliegenden Erfindung sind die in Verfahrensschritt a) bereitgestellten Kohlenhydratpartikel kristallin, bevorzugt vollständig kristallin.

In einer bevorzugten Ausführung der vorliegenden Erfindung weisen die in Verfahrensschritt a) bereitgestellten Kohlenhydratpartikel keine oder nur geringe amorphe Strukturen auf.

In einer bevorzugten Ausführung der vorliegenden Erfindung werden die in Verfahrensschritt a) bereitgestellten Kohlenhydratpartikel in Verfahrensschritt b) in einer Menge von 2 bis 30 Gew.- %, insbesondere 7 bis 30 Gew.-%, insbesondere 10 bis 30 Gew.-%, insbesondere 7 bis 25 Gew.- %, insbesondere 10 bis 25 Gew.-%. insbesondere 7 bis 20 Gew.-%, insbesondere 10 bis 25 Gew.- %, insbesondere 7 bis 15 Gew.-%, insbesondere 10 bis 15 Gew.-%, insbesondere 7 bis 10 Gew.- % (jeweils bezogen auf Gesamtgewicht der Kohlenhydrat-Ausgangspräparation) eindosiert.

In einer bevorzugten Ausführung der vorliegenden Erfindung werden die in Verfahrensschritt a) bereitgestellten Kohlenhydratpartikel in Verfahrensschritt b) in einer Menge von 2 bis 25 Gew.- %, insbesondere 5 bis 20 Gew.-%, insbesondere 7 bis 15 Gew.-% und insbesondere 8 bis 10 Gew.- % (jeweils bezogen auf Gesamtgewicht der Kohlenhydrat-Ausgangspräparation) eindosiert.

In einer bevorzugten Ausführung der vorliegenden Erfindung werden die in Verfahrensschritt a) bereitgestellten Kohlenhydratpartikel in Verfahrensschritt b) in einer Menge von 4 bis 25 Gew.- %, insbesondere 4 bis 20 Gew.-%, insbesondere 4 bis 15 Gew.-% und insbesondere 4 bis 10 Gew.- % (jeweils bezogen auf Gesamtgewicht der Kohlenhydrat-Ausgangspräparation) eindosiert.

In einer bevorzugten Ausführung der vorliegenden Erfindung wird Verfahrensschritt b) bei einer Temperatur von 20 bis 35 °C, insbesondere 20 bis 30 °C, durchgeführt.

In einer bevorzugten Ausführung der vorliegenden Erfindung wird das Eindosieren der Kohlenhydratpartikel in Verfahrensschritt b) in einem Über- und Unterdruck-freien Trocknungsreaktor unter atmosphärischem Druck, in einem Vakuum-Trocknungsreaktor unter reduziertem Druck, insbesondere 10 bis 900 mbar, insbesondere 20 bis 600 mbar, insbesondere 30 bis 400 mbar, insbesondere 40 bis 200 mbar, insbesondere 50 bis 100 mbar oder insbesondere 600 bis 800 mbar, insbesondere 650 bis 750 mbar, insbesondere 700 mbar, oder in einem Druck-Trocknungsreaktor unter einem Druck von atmosphärischem Druck bis 1100 mbar, insbesondere oberhalb des atmosphärischen Drucks bis 1100 mbar, durchgeführt.

In einer bevorzugten Ausführung der vorliegenden Erfindung wird das Eindosieren, insbesondere das Eindosieren über eine Dosiervorrichtung, insbesondere über rotierende Dosiersysteme, Vibrations- oder Schwingdosiersysteme oder über pneumatische Dosiersysteme, insbesondere bei Überdruck oder bei Unterdruck durchgeführt.

In einer bevorzugten Ausführung der vorliegenden Erfindung ist das Eindosieren in Verfahrensschritt b) ein mechanisches Eindosieren über rotierende Dosierysteme, wobei das rotierende Dosiersystem eine Zellradschleuse, eine Dosierschleuse oder ein Schneckenförderer ist.

In einer bevorzugten Ausführung der vorliegenden Erfindung ist das Eindosieren in Verfahrensschritt b) ein Vibrations- oder Schwing-Eindosieren über Vibrations- oder Schwingdosiersysteme, wobei das Vibrations- oder Schwingdosiersystem eine Schwingrinne, insbesondere eine lineare Schwingrinne, oder ein Wendelförderer ist.

In einer bevorzugten Ausführung der vorliegenden Erfindung ist das Eindosieren in Verfahrensschritt b) ein pneumatisches Eindosieren über pneumatische Dosiersysteme, wobei das pneumatische Dosiersystem ein Flugförder-System, ein Dichtstromförder-System, ein Schubförder-System oder ein Pfropfenförder-System ist.

In einer bevorzugten Ausführung der vorliegenden Erfindung sind an dem pneumatischen Dosiersystem mindestens eine, insbesondere mindestens zwei, insbesondere mindestens drei, insbesondere mindestens vier, insbesondere mindestens 5 Düsen angebracht. Bevorzugt dienen die Düsen vorteilhafterweise der Verbesserung der Verteilung der feinkristallinen Kohlenhydrate.

Gegebenenfalls kann das Eindosieren auch manuell gesteuert mittels einer Dosierschaufel oder eines Dosierlöffels durchgeführt werden.

In einer bevorzugten Ausführung der vorliegenden Erfindung umfasst die in Verfahrensschritt b) erhaltene Mischung die Kohlenhydrate Saccharose, Glucose, Fructose sowie Wasser.

In bevorzugter Ausführung der vorliegenden Erfindung weist die in Verfahrensschritt c) eingesetzte, in Verfahrensschritt b) erhaltene Mischung eine Temperatur von 20 bis 80 °C, insbesondere 25 bis 60 °C, insbesondere 30 °C bis 50 °C, insbesondere 40 °C bis 50 °C, insbesondere 25 bis 40 °C, auf.

In einer bevorzugten Ausführung der vorliegenden Erfindung weist die in Verfahrensschritt c) erhaltene getrocknete Kohlenhydratpräparation einen Wassergehalt von höchstens 6,0 Gew.-%, insbesondere höchstens 5,0 Gew.-%, insbesondere höchstens 1,9 Gew.-%, insbesondere höchstens 1,5 Gew.-%, insbesondere höchstens 1,0 Gew.-%, insbesondere höchstens 0,8 Gew.-%, insbesondere höchstens 0,7 Gew.-%, insbesondere höchstens 0,6 Gew.-%, insbesondere höchstens 0,5 Gew.-%, insbesondere höchstens 0,4 Gew.-%, insbesondere höchstens 0,3 Gew.-%, insbesondere höchstens 0,2 Gew.-%, insbesondere höchstens 0,1 Gew.-%, insbesondere 0,01 bis 6,00 Gew.-%, insbesondere 0,01 bis 5,00 Gew.-%, insbesondere 0,01 bis 0,70 Gew.-%, insbesondere 0,05 bis 6,00 Gew.-%, insbesondere 0,05 bis 5,00 Gew.-%, insbesondere 0,05 bis 0,60 Gew.-%, insbesondere 0,10 bis 0,50 Gew.-%, insbesondere 0,20 bis 0,40 Gew.-%, insbesondere 0,20 bis 0,30 Gew.-%, insbesondere 0,12 Gew.-%, insbesondere 0,13 Gew.-%, insbesondere 0,27 Gew.-%, insbesondere 0,29 Gew.-%, insbesondere 0,31 Gew.-%, insbesondere 0,34 Gew.-%, insbesondere 0,42 Gew.-%, insbesondere 0,43 Gew.-%, insbesondere 0,47 Gew.-% (jeweils bezogen auf Gesamtgewicht der getrockneten Kohlenhydratpräparation) auf. Bevorzugt ist der Wassergehalt der in Verfahrensschritt c) erhaltenen getrockneten Kohlenhydratpräparation allein der in der Kohlenhydratpräparation vorhandene Kristallwassergehalt.

Gemäß der vorliegenden Erfindung weist die in Verfahrensschritt c) erhaltene getrocknete Kohlenhydratpräparation einen geringeren Wassergehalt als die in Verfahrensschritt a) bereitgestellte kristalline wasserhaltige Kohlenhydrat-Ausgangspräparation auf.

In einer bevorzugten Ausführung der vorliegenden Erfindung weist die in Verfahrensschritt a) bereitgestellte kristalline wasserhaltige Kohlenhydrat-Ausgangspräparation 0,4 bis 22,5 Gew.-%, insbesondere 0,5 bis 22,5 Gew.-%, insbesondere 0,7 bis 20 Gew.-%, insbesondere 1,0 bis 18 Gew.- %, insbesondere 1,5 bis 15 Gew.-%, insbesondere 2,0 bis 13 Gew.-%, insbesondere 2,5 bis 12 Gew.-%, insbesondere 4,0 bis 10 Gew.-%, insbesondere 6,0 bis 8,0 Gew.-%, insbesondere 0,4 bis 4,0 Gew.-%, insbesondere 0,4 bis 3,5 Gew.-%, insbesondere 0,4 bis 3,0 Gew.-%, insbesondere 0,4 bis 2,5 Gew.-%, insbesondere 0,6 bis 2,0 Gew.-%, insbesondere 0,8 bis 1,5 Gew.-%, insbesondere 1,0 Gew.-%, Wasser (jeweils bezogen auf Gesamtgewicht der Kohlenhydrat-Ausgangspräparation) und die in Verfahrensschritt c) erhaltene getrocknete Kohlenhydratpräparation einen Wassergehalt von höchstens 6,0 Gew.-%, insbesondere höchstens 5,0 Gew.-%, insbesondere höchstens 1,9 Gew.-%, insbesondere höchstens 1,5 Gew.-%, insbesondere höchstens 1,0 Gew.-%, insbesondere höchstens 0,8 Gew.-%, insbesondere höchstens 0,7 Gew.-%, insbesondere höchstens 0,6 Gew.-%, insbesondere höchstens 0,5 Gew.-%, insbesondere höchstens 0,4 Gew.-%, insbesondere höchstens 0,3 Gew.-%, insbesondere höchstens 0,2 Gew.-%, insbesondere höchstens 0,1 Gew.-%, insbesondere 0,01 bis 6,00 Gew.-%, insbesondere 0,01 bis 5,00 Gew.-%, insbesondere 0,01 bis 0,70 Gew.-%, insbesondere 0,05 bis 6,00 Gew.-%, insbesondere 0,05 bis 5,00 Gew.-%, insbesondere 0,05 bis 0,60 Gew.-%, insbesondere 0,10 bis 0,50 Gew.-%, insbesondere 0,20 bis 0,40 Gew.-%, insbesondere 0,20 bis 0,30 Gew.-%, insbesondere 0,12 Gew.-%, insbesondere 0,13 Gew.-%, insbesondere 0,27 Gew.- %, insbesondere 0,29 Gew.-%, insbesondere 0,31 Gew.-%, insbesondere 0,34 Gew.-%, insbesondere 0,42 Gew.-%, insbesondere 0,43 Gew.-%, insbesondere 0,47 Gew.-% (jeweils bezogen auf Gesamtgewicht der getrockneten Kohlenhydratpräparation) auf, wobei die in Verfahrensschritt c) erhaltene getrocknete Kohlenhydratpräparation einen geringeren Wassergehalt als die in Verfahrensschritt a) bereitgestellte wässrige kristalline Kohlenhydrat-Ausgangspräparation aufweist. Bevorzugt ist der Wassergehalt der in Verfahrensschritt c) erhaltenen getrockneten Kohlenhydratpräparation allein der in der Kohlenhydratpräparation vorhandene Kristallwassergehalt.

In einer bevorzugten Ausführung der vorliegenden Erfindung umfasst die in Verfahrensschritt c) erhaltene getrocknete Kohlenhydratpräparation Saccharose, Glucose, Fructose und Wasser.

In einer bevorzugten Ausführung der vorliegenden Erfindung umfasst die in Verfahrensschritt c) erhaltene getrocknete Kohlenhydratpräparation Saccharose, insbesondere 92,0 bis 98,5 Gew.-%, insbesondere 92,5 bis 98,2 Gew.-%, insbesondere 92,7 bis 98,1 Gew.-%, insbesondere 93,0 bis 98,1 Gew.-% (jeweils bezogen auf das Gesamtgewicht der in Verfahrensschritt c) erhaltenen getrockneten Kohlenhydratpräparation), Glucose, insbesondere 0,40 bis 1,00 Gew.-%, insbesondere 0,44 bis 0,74 Gew.-%, insbesondere 0,46 bis 0,72 Gew.-%, insbesondere 0,48 bis 0,69 Gew.-% (jeweils bezogen auf das Gesamtgewicht der in Verfahrensschritt c) erhaltenen getrockneten Kohlenhydratpräparation) Fructose, insbesondere 0,30 bis 0,50 Gew.-%, insbesondere 0,41 bis 0,48 Gew.-%, insbesondere 0,43 bis 0,47 Gew.-%, insbesondere 0,44 bis 0,45 Gew.-% (jeweils bezogen auf das Gesamtgewicht der in Verfahrensschritt c) erhaltenen getrockneten Kohlenhydratpräparation) und Wasser, insbesondere 0,40 bis 6,00 Gew.-%, insbesondere 0,40 bis 5,00 Gew.-%,, insbesondere 0,40 bis 2,0 Gew.-%, insbesondere 0,58 bis 1,87 Gew.-%, insbesondere 0,61 bis 1,82 Gew.-%, insbesondere 0,63 bis 1,74 Gew.-%, insbesondere 0,33 bis 0,49 Gew.-%, insbesondere 0,37 bis 0,46 Gew.-%, insbesondere 0,44 bis 0,45 Gew.-% (jeweils bezogen auf das Gesamtgewicht der in Verfahrensschritt c) erhaltenen getrockneten Kohlenhydratpräparation). Bevorzugt ist der Wassergehalt der in Verfahrensschritt c) erhaltenen getrockneten Kohlenhydratpräparation allein der in der Kohlenhydratpräparation vorhandene Kristallwassergehalt.

In einer bevorzugten Ausführung der vorliegenden Erfindung umfasst die in Verfahrensschritt c) erhaltene getrocknete Kohlenhydratpräparation Saccharose, insbesondere 92,0 bis 98,5 Gew.-%, insbesondere 92,5 bis 98,2 Gew.-%, insbesondere 92,7 bis 98,1 Gew.-%, insbesondere 93,0 bis 98,1 Gew.-% (jeweils bezogen auf das Gesamtgewicht der in Verfahrensschritt c) erhaltenen getrockneten Kohlenhydratpräparation), Glucose, insbesondere 0,40 bis 1,00 Gew.-%, insbesondere 0,44 bis 0,74 Gew.-%, insbesondere 0,46 bis 0,72 Gew.-%, insbesondere 0,48 bis 0,69 Gew.-% (jeweils bezogen auf das Gesamtgewicht der in Verfahrensschritt c) erhaltenen getrockneten Kohlenhydratpräparation) Fructose, insbesondere 0,30 bis 0,50 Gew.-%, insbesondere 0,41 bis 0,48 Gew.-%, insbesondere 0,43 bis 0,47 Gew.-%, insbesondere 0,44 bis 0,45 Gew.-% (jeweils bezogen auf das Gesamtgewicht der in Verfahrensschritt c) erhaltenen getrockneten Kohlenhydratpräparation), Nebenkomponenten, insbesondere 0,40 bis 4,50 Gew.-%, insbesondere 0,40 bis 4,36 Gew.-%, insbesondere 0,42 bis 4,24 Gew.-%, insbesondere 0,40 bis 4,04 Gew.-% (jeweils bezogen auf das Gesamtgewicht der in Verfahrensschritt c) erhaltenen getrockneten Kohlenhydratpräparation) und Wasser, insbesondere 0,40 bis 6,00 Gew.-%, insbesondere 0,40 bis 5,00 Gew.-%, insbesondere 0,40 bis 2,0 Gew.-%, insbesondere 0,58 bis 1,87 Gew.-%, insbesondere 0,61 bis 1,82 Gew.-%, insbesondere 0,63 bis 1,74 Gew.-%, insbesondere 0,33 bis 0,49 Gew.-%, insbesondere 0,37 bis 0,46 Gew.-%, insbesondere 0,44 bis 0,45 Gew.-% (jeweils bezogen auf das Gesamtgewicht der in Verfahrensschritt c) erhaltenen getrockneten Kohlenhydratpräparation). Bevorzugt ist der Wassergehalt der in Verfahrensschritt c) erhaltenen getrockneten Kohlenhydratpräparation allein der in der Kohlenhydratpräparation vorhandene Kristallwassergehalt.

In einer bevorzugten Ausführung der vorliegenden Erfindung weist die in Verfahrensschritt c) erhaltene getrocknete Kohlenhydratpräparation einen Böschungswinkel von 30,0 bis 45,0 °, insbesondere 38,0 bis 42,0 °, insbesondere 39,0 bis 41,0 °, insbesondere 38,1 °, insbesondere 40,7 °, insbesondere 41,9°, auf.

In einer bevorzugten Ausführung der vorliegenden Erfindung weist die in Verfahrensschritt c) erhaltene getrocknete Kohlenhydratpräparation eine Rieselfähigkeit von 0,1 bis 45,0 s/100 g, insbesondere 0,2 bis 45,0 s/100 g, insbesondere 0,5 bis 40,0 s/100 g, insbesondere 1,5 bis 35,0 s/100 g, insbesondere 2,0 bis 25,0 s/100 g, auf.

In einer bevorzugten Ausführung der vorliegenden Erfindung weist die in Verfahrensschritt c) erhaltene getrocknete Kohlenhydratpräparation bei einer Auslauföffnungsgröße von 6 mm des für den Test verwendeten Trichters eine Rieselfähigkeit von 20,0 bis 45,0 s/100 g, insbesondere 30,0 bis 40,0 s/100 g, insbesondere 32,0 bis 40,0 s/100 g, insbesondere 34,0 bis 40,0 s/100 g, insbesondere 34,2 s/100 g, insbesondere 39,1 s/100 g, auf.

In einer bevorzugten Ausführung der vorliegenden Erfindung weist die in Verfahrensschritt c) erhaltene getrocknete Kohlenhydratpräparation bei einer Auslauföffnungsgröße von 8 mm des für den Test verwendeten Trichters eine Rieselfähigkeit von 10,0 bis 25,0 s/100 g, insbesondere 12,0 bis 23,0 s/100 g, insbesondere 14,0 bis 18 s/100 g, insbesondere 15,5 s/100 g, insbesondere 15,7 s/100 g, insbesondere 22,5 s/100 g, auf.

In einer bevorzugten Ausführung der vorliegenden Erfindung weist die in Verfahrensschritt c) erhaltene getrocknete Kohlenhydratpräparation bei einer Auslauföffnungsgröße von 10 mm des für den Test verwendeten Trichters eine Rieselfähigkeit von 6,0 bis 20,0 s/100 g, insbesondere 7,0 bis 19,0 s/100 g, insbesondere 8,0 bis 15,0 s/100 g, insbesondere 8,2 s/100 g, insbesondere 8,3 s/100 g, insbesondere 13,2 s/100 g, insbesondere 18,1 s/100 g, auf.

In einer bevorzugten Ausführung der vorliegenden Erfindung weist die in Verfahrensschritt c) erhaltene getrocknete Kohlenhydratpräparation bei einer Auslauföffnungsgröße von 11,3 mm des für den Test verwendeten Trichters eine Rieselfähigkeit von 3,0 bis 15,0 s/100 g, insbesondere 4,0 bis 14,0 s/100 g, insbesondere 5,0 bis 13,0 s/100 g, insbesondere 5,2 s/100 g, insbesondere 5,3 s/100 g, insbesondere 8,1 s/100 g, insbesondere 12,6 s/100 g, auf.

In einer bevorzugten Ausführung der vorliegenden Erfindung weist die in Verfahrensschritt c) erhaltene getrocknete Kohlenhydratpräparation bei einer Auslauföffnungsgröße von 15 mm des für den Test verwendeten Trichters eine Rieselfähigkeit von 1,0 bis 8,0 s/100 g, insbesondere 1,5 bis 7,0 s/100 g, insbesondere 2,0 bis 6,5 s/100 g, insbesondere 2,2 s/100 g, insbesondere 2,3 s/100 g, insbesondere 3,9 s/100 g, insbesondere 6,5 s/100 g, auf.

In einer bevorzugten Ausführung der vorliegenden Erfindung weist die in Verfahrensschritt c) erhaltene getrocknete Kohlenhydratpräparation bei einer Auslauföffnungsgröße von 25 mm des für den Test verwendeten Trichters eine Rieselfähigkeit von 0,1 bis 4,0 s/100 g, insbesondere 0,2 bis 3,0 s/100 g, insbesondere 0,2 bis 2,0 s/100 g, insbesondere 0,2 s/100 g, insbesondere 0,6 s/100 g, insbesondere 1,9 s/100 g, auf.

In einer bevorzugten Ausführung der vorliegenden Erfindung weist die in Verfahrensschritt c) erhaltene getrocknete Kohlenhydratpräparation einen Gesamtwassergehalt von 0,1 bis 0,5 g/100 g, insbesondere 0,2 bis 0,5 g/100 g, insbesondere 0,3 bis 0,5 g/100 g, insbesondere 0,33 bis 0,46 g/100 g, insbesondere 0,33 g/100 g, insbesondere 0,37 g/100 g, insbesondere 0,44 g/100 g, insbesondere 0,46 g/100 g, auf.

In einer bevorzugten Ausführung der vorliegenden Erfindung weist die in Verfahrensschritt c) erhaltene getrocknete Kohlenhydratpräparation einen Oberflächenwassergehalt von 0,2 bis 0,5 g/100 g, insbesondere 0,3 bis 0,5 g/100 g, insbesondere 0,31 bis 0,43 g/100 g, insbesondere 0,31 g/100 g, insbesondere 0,34 g/100 g, insbesondere 0,42 g/100 g, insbesondere 0,43 g/100 g, auf.

In einer besonders bevorzugten Ausführungsform der vorliegenden Erfindung dient die in Verfahrensschritt c) eingesetzte Mischvorrichtung einem Vermischen der in Verfahrensschritt b) erhaltenen Mischung und damit einer Homogenisierung, das heißt möglichst gleichmäßigen Verteilung der vermischten Komponenten. Die Mischvorrichtung dient daher der Erzeugung einer mechanischen Agitation der erhaltenen Mischung.

In einer bevorzugten Ausführung der vorliegenden Erfindung ist die Mischvorrichtung in Verfahrensschritt c) eine in dem Trocknungsreaktor angeordnete Mischvorrichtung, insbesondere eine oder mehrere Rührwellen.

In einer bevorzugten Ausführung der vorliegenden Erfindung ist die Mischvorrichtung in Verfahrensschritt c) integral mit dem Trocknungsreaktor verbunden oder ein Bestandteil von diesem und ausgebildet als ein zur Ausführung von, bevorzugt, regelmäßigen, Mischbewegungen ausgebildeter Trocknungsreaktor, der insbesondere in der Lage ist, rotierende oder schwenkende Bewegungen durchzuführen, insbesondere wobei in dem Trocknungsreaktor, eine oder mehrere Einbauten vorhanden sind.

In besonders bevorzugter Ausführungsform werden Verfahrensschritte a), b) und c) in einem Trocknungsreaktor ausgeführt, der als ein zur Ausführung von, bevorzugt regelmäßigen, Mischbewegungen ausgebildeter Trocknungsreaktor ausgebildet ist, insbesondere in der Lage ist, rotierende oder schwenkende Bewegungen durchzuführen, insbesondere wobei in dem Trocknungsreaktor, eine oder mehrere Einbauten vorhanden sind. Trocknungsreaktor und Mischvorrichtung stellen in einer bevorzugten Ausführung eine einzige Vorrichtung dar.

In einer bevorzugten Ausführung der vorliegenden Erfindung wird Verfahrensschritt c) bei einer Temperatur von 20 bis 35 °C, insbesondere 20 bis 30 °C, durchgeführt.

In einer bevorzugten Ausführung der vorliegenden Erfindung erfolgt im Anschluss an Verfahrensschritt c) in einem Verfahrensschritt d) ein Konditionieren.

In einer bevorzugten Ausführung der vorliegenden Erfindung wird das Konditionieren gemäß Verfahrensschritt d) bei einer Temperatur von mindestens 30 °C, insbesondere 30 bis 180 °C, insbesondere 35 bis 160 °C, insbesondere 45 bis 100 °C, insbesondere 35 bis 60°C, insbesondere 40 bis 60 °C, insbesondere 50 bis 60 °C, durchgeführt.

In einer bevorzugten Ausführung der vorliegenden Erfindung wird das Konditionieren gemäß Verfahrensschritt d) bei einem Druck von 10 bis 1100 mbar, insbesondere 10 bis 1000 mbar, insbesondere 10 bis 900 mbar, insbesondere 20 bis 600 mbar, insbesondere 30 bis 400 mbar, insbesondere 40 bis 200 mbar, insbesondere 50 bis 100 mbar, durchgeführt.

In einer bevorzugten Ausführung der vorliegenden Erfindung ist das in Verfahrensschritt d) durchgeführte Konditionieren eine Trocknung, insbesondere eine Lufttrocknung, insbesondere bei einer Lufttemperatur von 85 bis 95 °C, insbesondere 90 °C.

In einer bevorzugten Ausführung der vorliegenden Erfindung weist die in Verfahrensschritt d) erhaltene konditionierte Kohlenhydratpräparation einen Wassergehalt von höchstens 6,0 Gew.-%, insbesondere höchstens 5,0 Gew.-%, insbesondere höchstens 1,9 Gew.-%, insbesondere höchstens 1,5 Gew.-%, insbesondere höchstens 1,0 Gew.-%, insbesondere höchstens 0,8 Gew.-%, insbesondere höchstens 0,7 Gew.-%, insbesondere höchstens 0,6 Gew.-%, insbesondere höchstens 0,5 Gew.-%, insbesondere höchstens 0,4 Gew.-%, insbesondere höchstens 0,3 Gew.-%, insbesondere höchstens 0,2 Gew.-%, insbesondere höchstens 0,1 Gew.-%, insbesondere 0,01 bis 6,00 Gew.-%, insbesondere 0,01 bis 5,00 Gew.-%, insbesondere 0,01 bis 0,70 Gew.-%, insbesondere 0,05 bis 6,00 Gew.-%, insbesondere 0,05 bis 5,00 Gew.-%, insbesondere 0,05 bis 0,60 Gew.-%, insbesondere 0,10 bis 0,50 Gew.-%, insbesondere 0,20 bis 0,40 Gew.-%, insbesondere 0,20 bis 0,30 Gew.-%, insbesondere 0,12 Gew.-%, insbesondere 0,13 Gew.-%, insbesondere 0,27 Gew.-%, insbesondere 0,29 Gew.-%, insbesondere 0,31 Gew.-%, insbesondere 0,34 Gew.-%, insbesondere 0,42 Gew.-%, insbesondere 0,43 Gew.-%, insbesondere 0,47 Gew.-% (jeweils bezogen auf Gesamtgewicht der getrockneten Kohlenhydratpräparation) auf. Bevorzugt ist der Wassergehalt der in Verfahrensschritt d) erhaltenen konditionierten Kohlenhydratpräparation allein der in der Kohlenhydratpräparation vorhandene Kristallwassergehalt.

In einer bevorzugten Ausführung der vorliegenden Erfindung umfasst die in Verfahrensschritt d) erhaltene konditionierte Kohlenhydratpräparation Saccharose, insbesondere 94,0 bis 99,0 Gew.- %, insbesondere 94,0 bis 98,7 Gew.-%, insbesondere 94,1 bis 98,7 Gew.-%, insbesondere 94,2 bis 98,6 Gew.-%, insbesondere 94,3 bis 98,5 Gew.-% (jeweils bezogen auf das Gesamtgewicht der in Verfahrensschritt d) erhaltenen konditionierten Kohlenhydratpräparation), Glucose, insbesondere 0,40 bis 1,00 Gew.-%, insbesondere 0,45 bis 0,76 Gew.-%, insbesondere 0,47 bis 0,73 Gew.-%, insbesondere 0,48 bis 0,70 Gew.-% (jeweils bezogen auf das Gesamtgewicht der in Verfahrensschritt d) erhaltenen konditionierten Kohlenhydratpräparation) Fructose, insbesondere 0,30 bis 0,50 Gew.-%, insbesondere 0,41 bis 0,49 Gew.-%, insbesondere 0,43 bis 0,47 Gew.-%, insbesondere 0,44 bis 0,45 Gew.-% (jeweils bezogen auf das Gesamtgewicht der in Verfahrensschritt d) erhaltenen konditionierten Kohlenhydratpräparation) und Wasser, insbesondere 0,10 bis 6,00 Gew.-%, insbesondere 0,10 bis 5,00 Gew.-%, insbesondere 0,40 bis 6,00 Gew.-%, insbesondere 0,10 bis 0,50 Gew.-%, insbesondere 0,12 bis 0,42 Gew.-%, insbesondere 0,13 bis 0,29 Gew.-%, insbesondere 0,27 bis 0,28 Gew.-% (jeweils bezogen auf das Gesamtgewicht der in Verfahrensschritt d) erhaltenen konditionierten Kohlenhydratpräparation). Bevorzugt ist der Wassergehalt der in Verfahrensschritt c) erhaltenen getrockneten Kohlenhydratpräparation allein der in der Kohlenhydratpräparation vorhandene Kristallwassergehalt.

In einer bevorzugten Ausführung der vorliegenden Erfindung umfasst die in Verfahrensschritt d) erhaltene konditionierte Kohlenhydratpräparation Saccharose, insbesondere 94,0 bis 99,0 Gew.- %, insbesondere 94,0 bis 98,7 Gew.-%, insbesondere 94,1 bis 98,7 Gew.-%, insbesondere 94,2 bis 98,6 Gew.-%, insbesondere 94,3 bis 98,5 Gew.-% (jeweils bezogen auf das Gesamtgewicht der in Verfahrensschritt d) erhaltenen konditionierten Kohlenhydratpräparation), Glucose, insbesondere 0,40 bis 1,0 Gew.-%, insbesondere 0,45 bis 0,76 Gew.-%, insbesondere 0,47 bis 0,73 Gew.-%, insbesondere 0,48 bis 0,70 Gew.-% (jeweils bezogen auf das Gesamtgewicht der in Verfahrensschritt d) erhaltenen konditionierten Kohlenhydratpräparation) Fructose, insbesondere 0,30 bis 0,50 Gew.-%, insbesondere 0,41 bis 0,49 Gew.-%, insbesondere 0,43 bis 0,47 Gew.-%, insbesondere 0,44 bis 0,45 Gew.-% (jeweils bezogen auf das Gesamtgewicht der in Verfahrensschritt d) erhaltenen konditionierten Kohlenhydratpräparation), Nebenkomponenten, insbesondere 0,20 bis 4,50 Gew.-%, insbesondere 0,40 Gew.-% bis 4,43, insbesondere 0,42 bis 4,30 Gew.-%, insbesondere 0,40 bis 4,1 Gew.-% (jeweils bezogen auf das Gesamtgewicht der in Verfahrensschritt d) erhaltenen konditionierten Kohlenhydratpräparation) und Wasser, insbesondere 0,10 bis 0,50 Gew.-%, insbesondere 0,12 bis 0,42 Gew.-%, insbesondere 0,13 bis 0,29 Gew.-%, insbesondere 0,27 bis 0,28 Gew.-% (jeweils bezogen auf das Gesamtgewicht der in Verfahrensschritt d) erhaltenen konditionierten Kohlenhydratpräparation).

In einer bevorzugten Ausführung der vorliegenden Erfindung weist die in Verfahrensschritt d) erhaltene konditionierte Kohlenhydratpräparation einen Böschungswinkel von 30,0 bis 45,0 °, insbesondere 33,0 bis 41,0 °, insbesondere 35,0 bis 38,5 °, insbesondere 36,0 bis 37,0 °, insbesondere 33,7 °, insbesondere 35,1 °, insbesondere 36,4 °, insbesondere 36,6 °, insbesondere 38,5 °, insbesondere 40,8 °, auf.

In einer bevorzugten Ausführung der vorliegenden Erfindung weist die in Verfahrensschritt d) erhaltene konditionierte Kohlenhydratpräparation eine Rieselfähigkeit von 0 bis 45,0 s/100 g, insbesondere 0,1 bis 45,0 s/100 g, insbesondere 1,3 bis 40,0 s/100 g, insbesondere 2,0 bis 30,0 s/100 g, insbesondere 3,0 bis 25,0 s/100 g, auf.

In einer bevorzugten Ausführung der vorliegenden Erfindung weist die in Verfahrensschritt d) erhaltene konditionierte Kohlenhydratpräparation bei einer Auslauföffnungsgröße von 6 mm des für den Test verwendeten Trichters eine Rieselfähigkeit von 20,0 bis 45,0 s/100 g, insbesondere 28,0 bis 37,0 s/100 g, insbesondere 30,0 bis 37,0 s/100 g, insbesondere 32,0 bis 36,5 s/100 g, insbesondere 28,5 s/100 g, insbesondere 30,6 s/100 g, insbesondere 32,6 s/100 g, insbesondere 36,2 s/100 g, insbesondere 36,4 s/100 g, auf.

In einer bevorzugten Ausführung der vorliegenden Erfindung weist die in Verfahrensschritt d) erhaltene konditionierte Kohlenhydratpräparation bei einer Auslauföffnungsgröße von 8 mm des für den Test verwendeten Trichters eine Rieselfähigkeit von 10,0 bis 20,0 s/100 g, insbesondere 12,0 bis 18,0 s/100 g, insbesondere 14,0 bis 16,0 s/100 g, insbesondere 12,6 s/100, insbesondere 14,7 s/100 g, insbesondere 14,8 s/100 g, insbesondere 15,0 s/100 g, insbesondere 15,1 s/100 g, insbesondere 17,4 s/100 g, auf.

In einer bevorzugten Ausführung der vorliegenden Erfindung weist die in Verfahrensschritt d) erhaltene konditionierte Kohlenhydratpräparation bei einer Auslauföffnungsgröße von 10 mm des für den Test verwendeten Trichters eine Rieselfähigkeit von 6,0 bis 12,0 s/100 g, insbesondere 7,0 bis 10,0 s/100 g, insbesondere 8,0 bis 9,5 s/100 g, insbesondere 6,6 s/100 g, insbesondere 7,7 s/100 g, insbesondere 8,0 s/100 g, insbesondere 8,2 s/100 g, insbesondere 8,3 s/100 g, insbesondere 9,3 s/100 g, insbesondere 9,7 s/100 g, auf.

In einer bevorzugten Ausführung der vorliegenden Erfindung weist die in Verfahrensschritt d) erhaltene konditionierte Kohlenhydratpräparation bei einer Auslauföffnungsgröße von 11,3 mm des für den Test verwendeten Trichters eine Rieselfähigkeit von 3,5 bis 7,0 s/100 g, insbesondere 5,0 bis 6,0 s/100 g, insbesondere 4,1 s/100 g, insbesondere 5,0 s/100 g, insbesondere 5,3 s/100 g, insbesondere 5,4 s/100 g, insbesondere 6,1 s/100 g, auf.

In einer bevorzugten Ausführung der vorliegenden Erfindung weist die in Verfahrensschritt d) erhaltene konditionierte Kohlenhydratpräparation bei einer Auslauföffnungsgröße von 15 mm des für den Test verwendeten Trichters eine Rieselfähigkeit von 1,0 bis 6,0 s/100 g, insbesondere 1,5 bis 5,0 s/100 g, insbesondere 2,0 bis 4,0 s/100 g, insbesondere 1,7 s/100 g, insbesondere 2,1 s/100 g, insbesondere 3,8 s/100 g, insbesondere 4,6 s/100 g, auf.

In einer bevorzugten Ausführung der vorliegenden Erfindung weist die in Verfahrensschritt d) erhaltene konditionierte Kohlenhydratpräparation bei einer Auslauföffnungsgröße von 25 mm des für den Test verwendeten Trichters eine Rieselfähigkeit von 0,0 bis 4,0 s/100 g, insbesondere 0,0 bis 3,0 s/100 g, insbesondere 0,1 s/100 g, insbesondere 1,3 s/100 g, insbesondere 2,5 s/100 g, auf.

In einer bevorzugten Ausführung der vorliegenden Erfindung weist die in Verfahrensschritt d) erhaltene konditionierte Kohlenhydratpräparation einen Gesamtwassergehalt von 0,1 bis 6,00 g/100 g, insbesondere 0,1 bis 5,00 g/100 g, insbesondere 0,1 bis 0,5 g/100 g, insbesondere 0,12 bis 0,42 g/100 g, insbesondere 0,2 bis 0,42 g/100 g, insbesondere 0,3 bis 0,42 g/100 g, insbesondere 0,12 g/100 g, insbesondere 0,13 g/100 g, insbesondere 0,27 g/100 g, insbesondere 0,28 g/100 g, insbesondere 0,29 g/100 g, insbesondere 0,42 g/100 g, auf. Insbesondere wird der Gesamtwassergehalt der erhaltenen Kohlenhydratpräparation allein durch den Kristallwassergehalt gebildet.

In einer bevorzugten Ausführung der vorliegenden Erfindung weist die in Verfahrensschritt d) erhaltene konditionierte Kohlenhydratpräparation einen Oberflächenwassergehalt von 0,050 bis 0,100 g/100 g, insbesondere 0,050 bis 0,090 g/100 g, insbesondere 0,060 bis 0,080 g/100 g, insbesondere 0,070 bis 0,080 g/100 g, insbesondere 0,054 g/100 g, insbesondere 0,067 g/100 g, insbesondere 0,076 g/100 g, insbesondere 0,084, auf, insbesondere weist sie keinen Oberflächenwassergehalt auf.

In einer bevorzugten Ausführung der vorliegenden Erfindung wurde die wasserhaltige Saccharose-haltige Ausgangspräparation, insbesondere die Saccharose, aus Zuckerrohr gewonnen.

In einer bevorzugten Ausführung der vorliegenden Erfindung wurde die wasserhaltige Saccharose-haltige Ausgangspräparation, insbesondere die Saccharose, aus Zuckerrüben gewonnen.

In einer bevorzugten Ausführung der vorliegenden Erfindung weist die wasserhaltige Isomalt-haltige Ausgangspräparation einen 1,1-GPM (1-O-alpha-D-glucopyranosyl-D-mannit)- und 1,6-GPS (6-O-alpha-D-glucopyranosyl-D-sorbit)-Gehalt von 95,0 bis 100,0 Gew.-% (bezogen auf Gesamttrockenmasse der wasserhaltigen Isomalt-haltigen Ausgangspräparation) auf.

In einer bevorzugten Ausführung der vorliegenden Erfindung weist die wasserhaltige Isomalt-haltige Ausgangspräparation einen 1,1-GPM (1-O-alpha-D-glucopyranosyl-D-mannit)-Gehalt von 45,0 bis 50,0 Gew.-% und einen 1,6-GPS (6-O-alpha-D-glucopyranosyl-D-sorbit)-Gehalt von 50 bis 55 Gew.-% (jeweils bezogen auf Gesamttrockenmasse der wasserhaltigen Isomalt-haltigen Ausgangspräparation) auf.

In einer bevorzugten Ausführung der vorliegenden Erfindung weist die wasserhaltige Isomalt-haltige Ausgangspräparation einen 1,1-GPM (1-O-alpha-D-glucopyranosyl-D-mannit)-Gehalt von 20,0 bis 30,0 Gew.-% und einen 1,6-GPS (6-O-alpha-D-glucopyranosyl-D-sorbit)-Gehalt von 70,0 bis 80,0 Gew.-% (jeweils bezogen auf Gesamttrockenmasse der wasserhaltigen Isomalt-haltigen Ausgangspräparation) auf.

In einer bevorzugten Ausführung der vorliegenden Erfindung weist die wasserhaltige Isomalt-haltige Ausgangspräparation 1,1-GPS (1-O-alpha-D-glucopyranosyl-D-sorbit), Sorbit, Mannit, oder GPI oder eine Mischung von zwei oder mehr davon auf.

In einer bevorzugten Ausführung der vorliegenden Erfindung weist die wasserhaltige Isomaltulose- und Trehalulose-haltige Ausgangspräparation Fructose, Glucose, Isomaltose oder Isomelezitose oder eine Mischung von zwei oder mehr davon auf.

In einer bevorzugten Ausführung der vorliegenden Erfindung wird nach Verfahrensschritt c) oder Verfahrensschritt d) in einem Verfahrensschritt x) die getrocknete Kohlenhydratpräparation verpackt.

Im Zusammenhang mit der vorliegenden Erfindung wird unter einer "wasserhaltigen kristallinen Kohlenhydrat-Ausgangspräparation" eine Kohlenhydratpräparation verstanden, die einen Wassergehalt von 0,4 bis 22,5 Gew.-%, insbesondere 2,5 bis 12 Gew.-% (bezogen auf Gesamtgewicht der Kohlenhydrat-Ausgangspräparation) und einen Kohlenhydratgehalt aufweist, wobei die Kohlenhydrate zumindest teilweise, vorzugsweise vollständig, in kristalliner Form, insbesondere in in der wässrigen Lösung suspendierter oder von der wässrigen Lösung überzogener Form, vorliegen. In bevorzugter Ausführungsform kann die wasserhaltige Kohlenhydrat-Ausgangspräparation flüssig, insbesondere als Suspension, oder halbflüssig oder fest vorliegen, insbesondere liegt sie in fester Form vor. In besonders bevorzugter Ausführungsform liegen die Kohlenhydrate der wasserhaltigen kristallinen Kohlenhydrat-Ausgangspräparation als kristalline, von einem Flüssigkeitsfilm (im Rahmen der vorliegenden Erfindung auch als Sirupfilm bezeichnet) überzogene feste Kohlenhydratkristalle vor. Der Sirupfilm stellt eine oberflächlich auf den Kristallpartikeln der Kohlenhydrat-Ausgangspräparation vorliegende wäßrige Lösung oder Suspension des Kohlenhydrats oder der Kohlenhydrate der Ausgangspräparation dar und kann aus vorhergehenden Prozeßschritten der Kohlenhydrataufarbeitung stammen. Der Sirupfilm stellt damit einen nicht integral im kristallinen Partikel vorhandenen Wasseranteil dar, sondern liegt oberflächlich auf den kristallinen Partikeln vor, insbesondere ist der Wasseranteil des Sirupfilms kein Kristallwasser. Sofern in einem Kohlenhydrat Kristallwasser vorliegt, stellt der Sirupfilm einen weiteren, also zusätzlichen Wasseranteil des Kohlenhydrats dar. Eine wasserhaltige kristalline Kohlenhydrat-Ausgangspräparation der vorliegenden Erfindung weist daher im Falle von Kristallwasser-haltigen Kohlenhydraten wie Isomalt, insbesondere dessen Komponente 1,1-GPM, oder Isomaltulose, einen darüber hinaus gehenden, zusätzlichen Wassergehalt in Form des Flüssigkeitsfilms auf. Eine wasserhaltige kristalline Kohlenhydrat-Ausgangspräparation der vorliegenden Erfindung weist daher im Falle von Kristallwasser-freien Kohlenhydraten wie Saccharose einen Wassergehalt in Form des Flüssigkeitsfilms auf.

Der Wassergehalt der wasserhaltigen kristallinen Kohlenhydrat-Ausgangspräparation ermittelt sich durch Subtraktion des Trockengewichts der wasserhaltigen kristallinen Kohlenhydrat-Ausgangspräparation ohne Berücksichtigung von gegebenenfalls vorhandenem Kristallwasser von dem Gesamtgewicht der wasserhaltigen kristallinen Kohlenhydrat-Ausgangspräparation.

Der Wassergehalt der wasserhaltigen kristallinen Kohlenhydrat-Ausgangspräparation wird im Falle von Kohlenhydraten, die kein Kristallwasser aufweisen, von dem Wasser ausgebildet, in dem die kristallinen Kohlenhydratpartikel vorliegen, insbesondere von dem sie überzogen sind, also von dem Flüssigkeitsfilm.

Der Wassergehalt der wasserhaltigen kristallinen Kohlenhydrat-Ausgangspräparation wird im Falle von Kohlenhydraten, die Kristallwasser aufweisen, von dem Wasser ausgebildet, in dem die kristallinen Kohlenhydratpartikel vorliegen, insbesondere von dem sie überzogen sind, also dem Flüssigkeitsfilm, und zusätzlich dem Kristallwasser.

Kristallwasser ist die Bezeichnung für das Wasser, das in kristallinen Festkörpern auf festen Gitterplätzen im Kristallgitter eingebaut ist. Der stöchiometrischen Kristallwassergehalt kann über eine Röntgenstrukturanalyse des Festkörpers ermittelt werden. Der reale Kristallwassergehalt eines Kohlenhydrats lässt sich aus der Differenz zwischen dem Gesamtwassergehalt und dem Gehalt an freiem Wasser bestimmen. Dabei wird der Gesamtwassergehalt mittels Karl-Fischer Titration gemessen und der Anteil an freiem Wasser über Trocknungsexperimente.

Die Anwesenheit eines Sirupfilms lässt sich beispielsweise auch mittels Rieselfähigkeits-, Klebrigkeits- und/oder Verbackungstest nachweisen, insbesondere gemäß des Methodenteils der vorliegenden Beispiele, in denen vergleichend kristalline Partikel ohne Sirupfilmbeschichtung, insbesondere vollständig getrocknete oder übertrocknete, kristalline Partikel, mit den zu testenden gegebenenfalls Sirupfilm aufweisenden kristallinen Partikeln verglichen werden, und eine Sirupfilmbeschichtung durch eine signifikante Verschlechterung der Rieselfähigkeit, Erhöhung der Klebrigkeit und/oder erhöhte Verbackungsneigung, insbesondere aller drei, festgestellt wird.

Übertrocknete kristalline Partikel sind solche, die einem Trocknungsprozeß ausgesetzt wurden, im Rahmen dessen nicht nur oberflächliches Wasser verdampft wurde, sondern darüber hinaus auch Kristallwasser aus den kristallinen Partikeln entfernt wurde.

Im Zusammenhang mit der vorliegenden Erfindung wird unter "getrockneter Kohlenhydratpräparation" eine Kohlenhydratpräparation verstanden, die einen gegenüber der wasserhaltigen kristallinen Kohlenhydrat-Ausgangspräparation reduzierten Wassergehalt von höchstens 6,0, insbesondere höchstens 5,0, insbesondere höchstens 1,9 Gew.-%, insbesondere 0,01 bis 0,7 Gew.-%, (bezogen auf Gesamtgewicht der getrockneten Kohlenhydratpräparation) aufweist. Bevorzugt weist die getrocknete Kohlenhydratpräparation keinen Sirupfilm-überzogene kristalline Partikel auf, insbesondere ist ein gegebenenfalls noch vorhandener Wassergehalt allein das Kristallwasser.

Im Zusammenhang mit der vorliegenden Erfindung wird unter "Kohlenhydratpartikel" mindestens ein Kohlenhydrat verstanden, das in festem, partikulärem Zustand vorliegt. Bevorzugt liegen die Kohlenhydratpartikel als Pulver vor. Insbesondere liegen die Kohlenhydratpartikel in kristalliner Form vor.

Im Zusammenhang mit der vorliegenden Erfindung wird unter "teil-kristallin" eine morphologische Struktur eines Stoffes verstanden, in der der Stoff sowohl geordnete, kristalline als auch ungeordnete, amorphe Bereiche aufweist.

Im Zusammenhang mit der vorliegenden Erfindung wird unter "amorph" eine morphologische Struktur eines Stoffes verstanden, bei denen die Bausteine, insbesondere Moleküle oder Atome, nicht in einer Struktur mit Fernordnung angeordnet sind. Bevorzugt bilden die Bausteine, insbesondere Moleküle, eines amorphen Stoffes ein unregelmäßiges Muster und verfügen über eine Nahordnung. Bevorzugt verfügen amorphe Stoffe über eine Nahordnung und keine Fernordnung.

Im Zusammenhang mit der vorliegenden Erfindung wird unter "Fernordnung" eine regelmäßige und periodische Anordnung von Bausteinen, insbesondere Molekülen oder Atomen, in Stoffen über ihre Nachbarbausteine, insbesondere Nachbarmoleküle oder Nachbaratome, hinaus, verstanden. Demgemäß kann aus der genauen Position weniger Bausteine, insbesondere Moleküle oder Atome, vorteilhafterweise die Position aller Bausteine, insbesondere Moleküle oder Atome, in einem kristallinen Stoff bestimmt werden. Erfindungsgemäß bevorzugt weisen kristalline Stoffe zumindest teilweise eine Fernordnung auf.

Im Zusammenhang der vorliegenden Erfindung wird unter "Nahordnung" eine nur in der Nähe eines Bezugsbausteins, insbesondere Bezugmoleküls oder Bezugatoms, regelmäßige Gruppierung von Bausteinen, insbesondere Molekülen oder Atomen, verstanden. Bevorzugt bezieht sich die Nahordnung auf den nächsten Nachbarn.

Im Zusammenhang mit der vorliegenden Erfindung wird unter "voll-kristallin" eine morphologische Struktur eines Stoffes verstanden, bei denen die Bausteine, insbesondere Moleküle oder Atome, regelmäßig in einer Kristallstruktur angeordnet sind. Bevorzugt weisen voll-kristalline Stoffe sowohl Nah- als auch Fernordnung auf.

Im Zusammenhang mit der vorliegenden Erfindung wird unter "pulverförmig" ein fester, partikulärer Zustand eines Stoffes verstanden.

Im Zusammenhang mit der vorliegenden Erfindung wird unter "Böschungswinkel" der Winkel zwischen der Oberfläche eines Pulverkegels und seiner Basis verstanden. Bevorzugt wird der Böschungswinkel aus der Höhe des Pulverkegels h, dem Radius des Kegels r und tanα gemäß der Formel (1): tanα = h/r berechnet.

Im Zusammenhang mit der vorliegenden Erfindung wird unter dem Begriff "Rieselfähigkeit" das Ausmaß der freien Beweglichkeit von Pulvern oder Agglomeraten bezeichnet. Bevorzugt erfolgt die Bestimmung der Rieselfähigkeit beispielsweise mit Messtrichtern oder Rieselfähigkeitsprüfgeräten, wobei die Rieselzeit bei vorgegebener Masse oder vorgegebenen Volumen gemessen wird.

Insbesondere wird die "Rieselfähigkeit" gemessen gemäß der EUROPEAN PHARMACOPOEIA 10.0 Method 2.9.16. Flowability.

Im Zusammenhang mit der vorliegenden Erfindung wird unter dem Begriff "Klebrigkeit" die mittels des Klebrigkeits-Testverfahrens gemäß des Methodenteils der Beispiele gemessene Eigenschaft eines festen Kohlenhydratpräparation verstanden. Die Kraft kann als Maß für dieHaftwirkung eines Sirupfilms auf den Partikeloberflächen und damit die Stärke der Flüssigkeitsbrücken zwischen den Partikeln bewertet werden.

Im Zusammenhang mit der vorliegenden Erfindung wird unter dem Begriff "verbacken" eine über die Lagerzeit eintretende zumindest teilweise oder vollständige Verfestigung eines ursprünglich rieselfähigen Stoffes verstanden. Bevorzugt kann die Verfestigung optisch durch Klumpenbildung wahrgenommen werden. Im Zusammenhang mit der vorliegenden Erfindung wird die Verbackungseigenschaft mittels des Testverfahrens gemäß des Methodenteils der Beispiele bestimmt.

Im Zusammenhang mit der vorliegenden Erfindung wird unter dem Begriff "Kohlenhydrat" (im Folgenden auch als Saccharid bezeichnet) ein Zucker und/oder ein Zuckeralkohol sowohl in monomerer, dimerer als auch polymerer Form verstanden. Bevorzugt wird unter "Zucker" ein Mono- oder Disaccharidzucker, insbesondere Glucose, Fructose, Isomaltulose, Trehalulose und/oder Saccharose, verstanden.

Im Zusammenhang mit der vorliegenden Erfindung wird unter "Zuckeralkohol" ein Mono- oder Disaccharidalkohol, insbesondere Isomalt, Mannit und Sorbit, verstanden.

Sofern im Zusammenhang mit der vorliegenden Erfindung ein Wert für einen Druck angegeben wird, ist dieser als absoluter Druck und nicht als relativer Druck zum Atmosphärendruck zu verstehen. Ein Unterdruck ist ein Druck, welcher niedriger als der vorherrschende Atmosphärendruck (1 bar) ist. Unter einem Vakuum wird insbesondere ein Unterdruck verstanden. Ein Überdruck ist ein Druck, welcher höher als der vorherrschende Atmosphärendruck (1 bar) ist.

Im Zusammenhang mit der vorliegenden Erfindung wird unter "Invertzuckersirup" eine wässrige Lösung von teilweise durch Hydrolyse invertierter Saccharose mit einem definierten Trockensubstanzgehalt und einem definierten Anteil an Invertzucker in der Trockensubstanz verstanden. Invertzuckersirup weist insbesondere einen Trockensubstanzgehalt von 60 bis 70, insbesondere von 65 ± 0,5 % (refraktometrisch), einen Invertzuckergehalt (also summarischen Gehalt an Glucose und Fructose) von 95 - 99 % (HPLC), einen Glucosegehalt von 47 - 50 % (HPLC), einen Fructosegehalt von 47 - 50 % (HPLC) und einen Saccharosegehalt von 1 - 5 % (HPLC) auf.

Im Zusammenhang mit der vorliegenden Erfindung wird unter "Karamellzuckersirup" eine dunkelbraune wässrige Lösung aus Kohlenhydraten und Karamellstoffen verstanden, die auf Basis von Zucker durch kontrollierte Hitzeeinwirkung hergestellt wird. Karamellzuckersirup weist bevorzugt einen Trockensubstanzgehalt von 70 bis 80, insbesondere 75 ± 0,5 % (refraktometrisch), einen Glucosegehalt von 5 - 8 % (HPLC), einen Fructosegehalt von 1 - 4 % (HPLC) und einen Saccharosegehalt von 0 - 3 % (HPLC) auf.

Im Zusammenhang mit der vorliegenden Erfindung wird unter "Rohrzuckersirup" ein Saccharosesirup aus dem Zuckerrohr verstanden, der bevorzugt einen. Trockensubstanzgehalt von 70 bis 80, insbesondere von 75 ± 0,5 % (refraktometrisch), einen Glucosegehalt von 32 - 38 % (HPLC), einen Fructosegehalt von 25 - 30 % (HPLC) und einen Saccharosegehalt (Saccharose aus Zuckerrohr) von 10 - 15 % (HPLC) aufweist.

Im Zusammenhang mit der vorliegenden Erfindung wird unter dem Begriff "Stoff X in Form von Y" verstanden, daß der fragliche Stoff X als Bestandteil einer Zusammensetzung Y vorliegt, also X gemeinsam mit anderen, nicht spezifizierten Bestandteilen von Y vorliegt.

Im Zusammenhang mit der vorliegenden Erfindung wird unter dem Begriff "Nebenkomponenten" alle in einer Kohlenhydratpräparation vorhandenen Stoffe verstanden, die keine Mono- oder Disaccharide ausgewählt aus der Gruppe bestehend aus Glucose, Fructose, Isomaltulose, Trehalulose, Isomalt und Saccharose sind.

Im Zusammenhang mit der vorliegenden Erfindung sind "einzelne Nebenkomponente" individuelle Stoffe, die in ihrer Gesamtheit die Nebenkomponenten darstellen und wobei diese individuellen Stoffe, zum Beispiel Isomaltose, Glycerin, Glucopyranosylidit, Isomelezitose, jeweils einzelne in die Stoffgruppen der Monosaccharide, Disaccharide, Desoxydisaccharidalkohole, Trisaccharide, Glucosylglycerine, Glucosyltetritole, Glucosylpentitole, Trisaccharidalkohole, glucosylierte Disaccharidalkohole oder hydrierten Oligomere gehörende Stoffe sind.

Im Zusammenhang mit der vorliegenden Erfindung wird unter "Isomalt" ein Gemisch aus 6-O-α-D-Glucopyranosyl-D-sorbit (1,6-GPS) und 1-O-α-D-Glucopyranosyl-D-mannit (1,1-GPM) und optional 1-O-α-D-Glucopyranosyl-D-sorbit (1,1-GPS) verstanden, insbesondere Isomalt GS oder Isomalt ST.

Im Zusammenhang mit der vorliegenden Erfindung wird unter "Isomalt GS" ein Gemisch aus 72 bis 78 Gew.-%, bevorzugt 75 Gew.-%, 1,6-GPS und 22 bis 28 Gew.-%, insbesondere 25 Gew.-%, 1,1-GPM (jeweils bezogen auf Trockensubstanz des Isomalts) verstanden. Insbesondere weist das Isomalt GS einen 1,1-GPM (1-O-alpha-D-glucopyranosyl-D-mannit)-Gehalt von 20,0 bis 30,0 Gew.-% und einen 1,6-GPS (6-O-alpha-D-glucopyranosyl-D-sorbit)-Gehalt von 70,0 bis 80,0 Gew.-% (jeweils bezogen auf Gesamttrockenmasse des Isomalts) auf.

Im Zusammenhang mit der vorliegenden Erfindung wird unter "Isomalt ST" eine Mischung aus 54 bis 47 Gew.-% 1,6-GPS und 46 bis 53 Gew.-% 1,1-GPM (jeweils bezogen auf Trockengewicht des Isomalts) verstanden. Insbesondere weist das Isomalt ST einen 1,1-GPM (1-O-alpha-D-glucopyranosyl-D-mannit)-Gehalt von 45,0 bis 50,0 Gew.-% und einen 1,6-GPS (6-O-alpha-D-glucopyranosyl-D-sorbit)-Gehalt von 50 bis 55 Gew.-% (jeweils bezogen auf Gesamttrockenmasse des Isomalts) auf.

Im Zusammenhang mit der vorliegenden Erfindung wird unter dem Begriff "Isomalt" oder "hydrierter Isomaltulose" bevorzugt ein Gemisch bestehend aus oder umfassend 1,1-GPM und 1,6-GPS verstanden, insbesondere ein Gemisch bestehend aus oder umfassend 35 bis 61 Gew.-% 1,1-GPM und 65 bis 39 Gew.-% 1,6-GPS, insbesondere ein äquimolares oder nahezu äquimolares Gemisch bestehend aus oder umfassend 1,1-GPM und 1,6-GPS (jeweils bezogen auf Trockensubstanz des Isomalts).

Unter Isomalt können demgemäß auch Gemische bestehend aus oder umfassend 1,1-GPM und 1,6-GPS verstanden werden, die kein äquimolares Verhältnis von 1,1-GPM zu 1,6-GPS aufweisen, sondern in denen ein höherer 1,1-GPM- als 1,6-GPS-Gehalt oder ein höherer 1,6-GPS- als 1,1-GPM-Gehalt vorliegt.

In besonders bevorzugter Ausführungsform weist das Isomalt neben den beiden Komponenten 1,1-GPM und 1,6-GPS keine weiteren Komponenten auf.

In besonders bevorzugter Ausführungsform weist das Isomalt neben den beiden Komponenten 1,1-GPM und 1,6-GPS zusätzlich eine oder mehrere weitere Komponenten auf, beispielsweise Mannit, Sorbit, Saccharose, 1,1-GPS (1-O-α-D-Glucopyranosyl-D-sorbit), Glycosylglycitole, Desoxy-Disaccharidalkohole, GPI (Glucopyranosyl-idit), Isomaltose, Isomaltulose, Isomelezitose, hydrierte oder nicht-hydrierte Oligosaccharide, insbesondere hydrierte oder nicht-hydrierte Trisaccharide, oder/und andere Stoffe.

Im Zusammenhang mit der vorliegenden Erfindung wird unter einem "Isomaltulose- und Trehalulose-haltigen Gemisch" ein Gemisch umfassend Isomaltulose und Trehalulose verstanden, insbesondere erhalten aus einer enzymatischen Umsetzung von Saccharose mittels einer Saccharose-Isomerase zu einem Saccharose-Isomerengemisch enthaltend Isomaltulose und Trehalulose, sowie, optional, eine oder mehrere weitere Substanzen ausgewählt aus der Gruppe bestehend aus Saccharose, Fructose, Glucose, Turanose, Leucrose, Isomaltose, Raffinose, Isomelezitose, 6-Gluc Isomalt und 1-Gluc Isomalt.

Im Zusammenhang mit der vorliegenden Erfindung wird die Löslichkeit eines Kohlenhydrats bei 20 °C in Wasser, insbesondere destilliertem Wasser bestimmt.

Im Zusammenhang mit der vorliegenden Erfindung wird unter einem "schlecht-löslichen Kohlenhydrat" ein Kohlenhydrat verstanden, das in reinem Wasser, insbesondere destilliertem Wasser, mit einer Temperatur von 20 °C eine Löslichkeit von höchstens 53 g/100 g (g Trockensubstanz in 100 g Lösung) aufweist. Ein schlecht-lösliches Kohlenhydrat ist insbesondere Isomaltulose, Glucose, Mannit und 1,1-GPM.

Im Zusammenhang mit der vorliegenden Erfindung wird unter einem "gut-löslichen Kohlenhydrat" ein Kohlenhydrat verstanden, das in reinem Wasser, insbesondere destilliertem Wasser, mit einer Temperatur von 20 °C eine Löslichkeit von mehr als 53 g/100 g, insbesondere mindestens 66,7 g/100 g, insbesondere von mindestens 68,7 g/100 g, insbesondere von mindestens 70,0 g/100 g, insbesondere von mindestens 78,9 g/100 g (jeweils g Trockensubstanz in 100 g Lösung) aufweist. Ein gut-lösliches Kohlenhydrat ist insbesondere Saccharose, 1,6-GPS, Fructose, Trehalulose und Sorbit.

Im Zusammenhang mit der vorliegenden Erfindung wird der Durchmesser eines Kohlenhydratpartikels bestimmt mittels Luftstrahl-Siebanalyse mit einem Sieb der Maschenweite 100 µm.

Der mengenmäßige Anteil an Partikeln mit einem Durchmesser kleiner oder gleich 100 µm wird berechnet aus dem Anteil, der bei der Luftstrahlsiebanalyse das Sieb mit der Maschenweite 100 µm passiert, geteilt durch die Einwaage.

Im Zusammenhang mit der vorliegenden Erfindung sind Saccharose-haltige Ausgangspräparationen aus Zuckerrohr oder Zuckerrüben, obgleich sich diese ähneln, und zwar auch dann, wenn es sich um aufgereinigte oder hoch aufgereinigte Saccharose-haltige Ausgangspräparationen mit Saccharose-Gehalten von über 99,8 Gew.-% handelt, dennoch unterschiedlich. Insbesondere sind diese Präparationen unterschiedlich in Hinblick auf ihr Aromaprofil, ihre Anwendungseigenschaften, zum Beispiel ihre Löslichkeit, ihr thermisches Verhalten, die regelmäßig in ihnen vorhandenen Pflanzen-spezifischen Verunreinigungen und ihre sensorischen Eigenschaften (Lu et al., Journal of Food Engineering (2017), 214, 193 bis 208). Dies insbesondere deshalb, da Zuckerrohr, als zu der Familie der Süßgräser gehörige C4-Pflanze, physiologisch und morphologisch große Abweichungen zu der zur Familie der Fuchsschwanzgewächse gehörigen C3-Pflanze Zuckerrübe aufweist. Insbesondere ist es bekannt, dass sich aus C4-Pflanzen stammende Präparationen von Saccharose-Molekülen aufgrund einer stoffwechselbedingten Isotopendiskriminierung durch ein anderes ¹³C-/¹²C-Isotopenverhältnis von Saccharose aus C3-Pflanzen, zum Beispiel Zuckerrübe, unterscheidet (Martin et al., Journal of Science of Food and Agriculture, (1991), 56, 419-435). Insbesondere Saccharose-haltige Ausgangspräparationen aus C4-Pflanzen lassen sich daher von denen aus C3-Pflanzen durch ¹²C- und ¹³C-Isotopen-Bestimmung unterscheiden, da in C3-Pflanzen durch die Isotopendiskriminierung das ¹³C/¹²C-Verhältnis kleiner ist als in C4-Pflanzen.

Insbesondere offenbart Averill et al. (Journal of Thermal Analysis and Calorimetry, (2019), 127, 513 bis 538), dass sich kristalline Saccharose aus Zuckerrohr von der aus Rübenzucker auch bei hoher, über 99,8 Gew.-% liegender Saccharose-Reinheit durch unterschiedliches thermisches Verhalten unterscheidet und sich aus Zuckerrohr gewonnene Saccharosepräparationen in einigen Fällen durch das Vorhandensein eines kleinen, in DSC-Messungen (Differential Scanning Calorimetry, Dynamische Differenzkalorimetrie) erkennbaren Signals bei einer Temperatur unterhalb der des Hauptsignals bei etwa 185 °C bis 190 °C auszeichnet. Demgegenüber zeigen kommerzielle Saccharosepräparationen aus Zuckerrüben dieses Schmelzverhalten nicht, sondern weisen in DSC-Messungen lediglich ein Hauptsignal im Bereich von 185 °C bis 190 °C auf (US 8,273,873 B2).

Bevorzugt lassen sich grundsätzlich die Unterschiede der aus diesen verschiedenen Pflanzenarten stammenden Saccharosepräparationen durch den Metabolismus der Pflanzenart selbst, durch den jeweils verwendeten Aufreinigungsprozess der Saccharosepräparation und durch in der Regel in der erhaltenen Saccharosepräparation häufig noch vorhandene Pflanzen-spezifische Verunreinigungen, beispielsweise an Saccharosekristallen anhaftende Sirupreste und deren Feststoffe, erklären. Eine Saccharose aus Zuckerrohr enthaltende Präparation, insbesondere die Gesamtheit deren Saccharosemoleküle, ist daher eine andere Präparation als eine Saccharose aus Zuckerrübe enthaltende Präparation, insbesondere deren Gesamtheit an Saccharosemolekülen.

Im Zusammenhang mit der vorliegenden Erfindung bedeutet "Gew.-%" "Gewichts-%", "GC" bedeutet Gaschromatographie, "HPLC" bedeutet Hochleistungsflüssigkeitschromatographie, "Lfd. Nr." bedeutet Laufende Nummer und "KF" bedeutet Karl-Fischer-Methode.

Im Zusammenhang mit der vorliegenden Erfindung wird der Wassergehalt mittels der Methode nach Karl-Fischer tritrimetrisch bestimmt.

Sofern im Zusammenhang mit der vorliegenden Erfindung ein "Vorhandensein", ein "Enthalten", ein "Aufweisen" oder ein "Gehalt" einer Komponente ausdrücklich erwähnt oder impliziert wird bedeutet dies, dass die jeweilige Komponente vorhanden ist, insbesondere in messbarer Menge vorhanden ist.

Sofern im Zusammenhang mit der vorliegenden Erfindung ein "Vorhandensein", ein "Enthalten" oder ein "Aufweisen" einer Komponente in einer Menge von 0 [Einheit], insbesondere mg/kg, µg/kg oder Gew.-%, ausdrücklich erwähnt oder impliziert wird, bedeutet dies, dass die jeweiligen Komponenten nicht in messbarer Menge vorhanden, insbesondere nicht vorhanden ist.

Sofern im Zusammenhang mit der vorliegenden Erfindung quantitative Angaben, insbesondere Prozentangaben, von Komponenten eines Produktes oder einer Zusammensetzung angegeben sind, addieren diese, sofern nicht explizit anders angegeben oder fachmännisch ersichtlich, zusammen mit den anderen explizit angegeben oder fachmännisch ersichtlichen weiteren Komponenten der Zusammensetzung oder des Produktes auf 100 % der Zusammensetzung und/oder des Produktes auf.

Im Zusammenhang mit der vorliegenden Erfindung wird unter dem Begriff "mindestens eine" eine Mengenangabe verstanden, die eine Anzahl von 1 oder 2 oder 3 oder 4 oder 5 oder 6 oder 7 oder 8 oder 9 oder 10 und so weiter ausdrückt. In einer besonders bevorzugten Ausführungsform kann die Bezeichnung "mindestens eine" genau die Anzahl 1 darstellen. In einer weiteren bevorzugten Ausführungsform kann die Begrifflichkeit "mindestens eine" auch 2 oder 3 oder 4 oder 5 oder 6 oder 7 bedeuten.

Die Zahl der angegebenen Nachkommastellen entspricht der Präzision der jeweils angewandten Messmethode.

Sofern im Zusammenhang mit der vorliegenden Erfindung für eine Zahl die erste und zweite Nachkommastelle oder die zweite Nachkommastelle nicht angegeben sind/ist, sind/ist diese als Null zu setzen.

Unter dem Begriff "und/oder" wird in Zusammenhang mit der vorliegenden Erfindung verstanden, dass alle Mitglieder einer Gruppe, welche durch den Begriff "und/oder" verbunden sind, sowohl alternativ zueinander als auch jeweils untereinander kumulativ in einer beliebigen Kombination offenbart sind. Dies bedeutet für den Ausdruck "A, B und/oder C", dass folgender Offenbarungsgehalt darunter zu verstehen ist: a) A oder B oder C oder b) (A und B), oder c) (A und C), oder d) (B und C), oder e) (A und B und C).

Im Zusammenhang mit der vorliegenden Erfindung wird unter den Begriffen "umfassend" und "aufweisend" verstanden, dass zusätzlich zu den von diesen Begriffen explizit erfassten Elementen noch weitere, nicht explizit genannte Elemente hinzutreten können. Im Zusammenhang mit der vorliegenden Erfindung wird unter diesen Begriffen auch verstanden, dass allein die explizit genannten Elemente erfasst werden und keine weiteren Elemente vorliegen. In dieser besonderen Ausführungsform ist die Bedeutung der Begriffe "umfassend" und "aufweisend" gleichbedeutend mit dem Begriff "bestehend aus". Darüber hinaus erfassen die Begriffe "umfassend" und "aufweisend" auch Zusammensetzungen, die neben den explizit genannten Elementen auch weitere nicht genannte Elemente enthalten, die jedoch von funktioneller und qualitativ untergeordneter Natur sind. In dieser Ausführungsform sind die Begriffe "umfassend" und "aufweisend" gleichbedeutend mit dem Begriff "im Wesentlichen bestehend aus".

Weitere vorteilhafte Ausgestaltungen ergeben sich aus den Unteransprüchen.

Die Erfindung wird nachstehend ohne Einschränkung des allgemeinen Erfindungsgedankens anhand von Beispielen näher beschrieben.

### BEISPIELE:

Zusammensetzungen der in den Beispielen 1 und 2 genannten Ausgangsmaterialien
Sämtliche Saccharose stammt aus Zuckerrübe, wenn nicht anders angegeben (Rohrzuckersirup).

### Saccharose:

- Saccharose: min. 99,7°Z (Polarisation)
- Invertzuckersirup: < 0,04 % (enzym.)
- Feuchte: < 0,06 % (KF)

### Invertzucker:

- Trockensubstanzgehalt: 65 ± 0,5 % (refraktometrisch)
- Invertzuckergehalt: 95 - 99 % (HPLC)
   ∘ Glucosegehalt: 47 - 50 % (HPLC)
   ∘ Fructosegehalt: 47 - 50 % (HPLC)
   ∘ Saccharosegehalt: 1 - 5 % (HPLC)

### Karamellzuckersirup

- Trockensubstanzgehalt: 75 ± 0,5 % (refraktometrisch)
- Glucosegehalt: 5 - 8 % (HPLC)
- Fructosegehalt: 1 - 4 % (HPLC)
- Saccharosegehalt: 0 - 3 % (HPLC)

### Rohrzuckersirup (Saccharose aus Zuckerrohr)

- Trockensubstanzgehalt: 75 ± 0,5 % (refraktometrisch)
- Glucosegehalt: 32 - 38 % (HPLC)
- Fructosegehalt: 25 - 30 % (HPLC)
- Saccharosegehalt: 10 - 15 % (HPLC)

### Methodenteil der Beispiele - Beschreibung der Testverfahren:

### Rieselfähigkeitstest:

Die Rieselfähigkeit wurde gemäß EUROPEAN PHARMACOPOEIA 10.0 Method 2.9.16. Flowability bestimmt.

### Klebrigkeitstest:

Die Klebrigkeit wurde ermittelt gemäß der Beschreibungen in: "Handbook of Food Science, Endress und Mattes, Technology, and Engineering, 19.12.2005, CRC Press, Volume 3, Kapitel 140; Uhlenbrock, Gordian, Jahrgang 83, Seiten 148-150 (1983); Food Technology International Europe, 1990, Endress und Dilger, Seiten 279 bis 282; Schilling et al. Eur Food Res Technol (2008) 226:1389-1398; Sirisakulwat et al., International Journal of Food Science and Technology, 20210, 45, 1647-1658 ; Zedler, "Die industrielle Obst- und Gemüseverwertung" Jahrgang 68, 12/83, Seiten 523-527 und Endreß et al., Intern. Zeitschrift für Lebensmittelt-Technologie und - Verfahrenstechnik, 38. Jahrgang, 1987, Heft 5.

Die Bruchfestigkeit der Gele wird danach beurteilt, welcher Zugkraft eine in das Gel eingelierte, genormte, so genannte Zerreißfigur ausgesetzt werden muss, um das Gel zu zerbrechen."Verwendet wird für diesen Test ein Prüfbnecher und Einsätze für Prüfbecher = Zerreißfigur.In einem standardisierten Kunststoffbecher wird eine Zerreißfigur gelegt.

Die SAP-Artikelnummern sind Herbstreith Pektinometer Mark IV - 901172, Prüfbecher - 900054, Zerreißfiguren - 901173.

100 g der zu untersuchenden Probe werden in den HPE-Becher gegeben. Durch leichtes 3-maliges Aufstampfen des Bechers wird die Probe leicht verdichtet und eine Stunde stehengelassen. Anschließend wird die Zerreißfigur mittels Texture Analyser (Firma Winopal) aus der Probe gezogen und die benötigte Kraft ermittelt.

Die Kraft kann als Maß für die Festigkeit des Materials und damit die Stärke der Wechselwirkungen zwischen den Kristallen bewertet werden.

### Verbackungstest:

Pro Proben werden drei Metalltiegel zu 2/3 gefüllt. Danach wird eine Kunststofffolie auf die Oberfläche der Probe gelegt und die Oberfläche mit einem Stempel (7000 Pa Gewicht) beschwert. Die Proben wird in einen Klimaschrank bei 25 °C und 65 % relativer Luftfeuchte für eine Woche gelagert. Nach der Lagerung werden Stempel und Kunststofffolie vorsichtig entfernt und die Probe vermessen.

Zur Messung der Einstichkraft wird eine Metallnadel einer definierten Geometrie in die Oberfläche der Probe eingetaucht und die Kraft über den Weg aufgezeichnet. Das erfolgt fünfmal pro Probe an jeweils unberührten Stellen. Die zum Eintauchen benötigte Kraft ist ein Indikator für die Verdichtung bzw. Klebrigkeit der Probe.

Als Messgerät wurde das AT-XT Plus Extended Height der Firma Stable Micro Systems verwendet.

Beispiel 1: Erfindungsgemäßes Verfahren zur Herstellung einer getrockneten und konditionierten Kohlenhydratpräparation (Proben 2 bis 4) und Herstellung einer Vergleichsprobe ohne Zugabe von Kohlenhydratpartikeln (Probe 1):
Im Folgenden wird die Herstellung beispielhaft an den Einwaagen der Probe mit der Versuchsnummer 2, Tabelle 1 beschrieben. Für die erfindungsgemäßen Proben mit den Versuchsnummern 3 bis 4, Tabelle 1 gelten die folgenden Ausführungen entsprechend. Probe 1 stellt eine Vergleichsprobe ohne Zugabe von feinkristalliner Saccharose dar.

Zur Herstellung einer wasserhaltigen kristallinen Kohlenhydrat-Ausgangspräparation werden in einem als Trocknungsreaktor fungierenden Dragierkessel 922,0 g Saccharose in Form von Kristallzucker (Südzucker EU2 Qualität Körnung M) vorgelegt. In einem beheizten und gerührten Gefäß werden 60 g Karamellzuckersirup (Komponente B) sowie 18 g Rohrzuckersirup (Komponente C) vermengt und auf circa (im Folgenden abgekürzt: ca.) 60 °C erwärmt. Die erhaltene erwärmte Sirupmischung wird langsam im drehenden Dragierkessel zu der Saccharose gegeben (Verfahrensschritt a1)). Die resultierende Mischung wird ca. 5 Minuten homogenisiert und eine wasserhaltige kristalline Kohlenhydrat-Ausgangspräparation gemäß Verfahrensschritt a) der vorliegenden Erfindung erhalten, wobei die kristallinen Saccharosepartikel mit einem Sirupfilm überzogen sind. Zu der wasserhaltigen kristallinen Kohlenhydrat-Ausgangspräparation mit einer Temperatur von 21 bis 22 °C werden für Proben 2 bis 4 über eine Schwingrinne bei einer Temperatur von 20 °C die Menge an in Tabelle 4 angegebener feinkristalliner Saccharose (mindestens 80 Gew.-% kleiner 100 µm und mind. 70 Gew. % kleiner als 32 µm) unter atmosphärischem Druck, bei ca. 1000 mbar gemäß Verfahrensschritt b) der vorliegenden Erfindung eindosiert. In Versuch 1 entfällt das Eindosieren von feinkristalliner Saccharose. Die Mischung mit der Temperatur von 20 bis 21 °C wird 5 Minuten unter atmosphärischem Druck bei ca. 1000 mbar gemäß Verfahrensschritt c) der vorliegenden Erfindung homogenisiert, bis die Kohlenhydratpartikel optisch erkennbar verteilt sind. Es werden Zusammensetzungen der getrockneten Kohlenhydratpräparation, aufgelistet in Tabellen 5 und 6, gemäß dem erfindungsgemäßen Verfahren erhalten. Mit einem Gebläse wird zum Konditionieren gemäß Verfahrensschritt d) der vorliegenden Erfindung auf ca. 90 °C vorgewärmte Umgebungsluft über die getrocknete Kohlenhydratpräparation geleitet. Die getrocknete Kohlenhydratpräparation erreicht dabei eine Temperatur von ca. 50 °C. Eine getrocknete und konditionierte Kohlenhydratpräparation wird erhalten (Tabelle 7), wenn die Mischung besonders leicht rieselfähig geworden ist und keine Verklumpungen mehr aufweist. Die Beheizung des Gebläses wird abgeschaltet und Umgebungsluft mit Raumtemperatur wird solange über die getrocknete und konditionierte Kohlenhydratpräparation geleitet, bis diese eine Temperatur von unter 35 °C erreicht hat.

Vergleichsprobe 1 dient als Kontrolle. Da keine feinkristalline Saccharose zugegeben wird, wird während des Versuches der Konditionierung mit warmer Luft kein rieselfähiges Produkt, sondern eine zähe und sehr klebrige Masse erhalten. Weitere Versuche oder Messungen können mit diesem Produkt nicht durchgeführt werden (k.A., nicht messbar, siehe Tabellen 7 und 8).

Die Charakteristika der durch das erfindungsgemäße Verfahren erhaltenen getrockneten und konditionierten Kohlenhydratpräparationen sind in den nachfolgenden Tabellen 1 bis 7 und die Ergebnisse von Untersuchungen zur Rieselfähigkeit und zum Böschungswinkel der jeweiligen konditionierten Kohlenhydratpräparationen sind in Tabelle 8 dargestellt. Die erfindungsgemäßen konditionierten Kohlenhydratpräparationen zeigen einen besonders guten Böschungswinkel und eine besonders gute Rieselfähigkeit.

**Tabelle 1: Mengen der Ausgangsstoffe zur Herstellung der kristallinen wasserhaltigen Saccharosepräparation**

| Versuchsnummer | Lfd. Nr. | Saccharose [g] | Komponente A Invertzuckersirup [g] | Komponente B Karamellzuckersirup [g] | Komponente C Rohrzuckersirup [g] |
|---|---|---|---|---|---|
| 1 | 202111129 | 922,0 | | 60,0 | 18,0 |
| 2 | 202111133 | 922,0 | - | 60,0 | 18,0 |
| 3 | 202111134 | 922,0 | - | 60,0 | 18,0 |
| 4 | 202111135 | 922,0 | - | 60,0 | 18,0 |

**Tabelle 2: Zusammensetzung der kristallinen wasserhaltigen Saccharosepräparation**

| Versuchsnummer | Lfd. Nr. | Saccharose [%] | Glucose [%] | Fructose [%] | Rest [%] | Wasser [%] |
|---|---|---|---|---|---|---|
| 1 | 202111129 | 92,39 | 0,76 | 0,49 | 4,45 | 1,91 |
| 2 | 202111133 | 92,39 | 0,76 | 0,49 | 4,45 | 1,91 |
| 3 | 202111134 | 92,39 | 0,76 | 0,49 | 4,45 | 1,91 |
| 4 | 202111135 | 92,39 | 0,76 | 0,49 | 4,45 | 1,91 |

**Tabelle 3: Zusammensetzung der Trockensubstanz der kristallinen wasserhaltigen Saccharosepräparation**

| Versuchsnummer | Lfd. Nr. | Saccharose [%] | Glucose [%] | Fructose [%] | Rest [%] |
|---|---|---|---|---|---|
| 1 | 202111129 | 94,19 | 0,77 | 0,50 | 4,53 |
| 2 | 202111133 | 94,19 | 0,77 | 0,50 | 4,53 |
| 3 | 202111134 | 94,19 | 0,77 | 0,50 | 4,53 |
| 4 | 202111135 | 94,19 | 0,77 | 0,50 | 4,53 |

**Tabelle 4: Mengen der Ausgangsstoffe zur Herstellung der getrockneten Kohlenhydratpräparationen sowie von Vergleichsprobe 1**

| Versuchs -nummer | Lfd. Nr. | Saccharose [g] | Komponente A Invertzucker -sirup [g] | Komponente B Karamellzucker -sirup [g] | Komponente C Rohrzucker -sirup [g] | Feinkristalline Saccharose [g] |
|---|---|---|---|---|---|---|
| 1 | 202111129 | 922,0 | | 60,0 | 18,0 | - |
| 2 | 202111133 | 922,0 | - | 60,0 | 18,0 | 20,0 |
| 3 | 202111134 | 922,0 | - | 60,0 | 18,0 | 50,0 |
| 4 | 202111135 | 922,0 | - | 60,0 | 18,0 | 100,0 |

**Tabelle 5: Zusammensetzung der getrockneten Kohlenhydratpräparationen sowie von Vergleichsprobe 1**

| Versuchsnumme r | Lfd. Nr. | Saccharose [%] | Glucose [%] | Fructose [%] | Rest [%] | Wasser [%] |
|---|---|---|---|---|---|---|
| 1 | 20211112 9 | 92,39 | 0,76 | 0,49 | 4,45 | 1,91 |
| 2 | 20211113 3 | 92,54 | 0,74 | 0,48 | 4,36 | 1,87 |
| 3 | 20211113 4 | 92,76 | 0,72 | 0,47 | 4,24 | 1,82 |
| 4 | 20211113 5 | 93,08 | 0,69 | 0,44 | 4,04 | 1,74 |

**Tabelle 6: Zusammensetzung der Gesamttrockenmasse der getrockneten Kohlenhydratpräparationen sowie von Vergleichsprobe 1**

| Versuchsnummer | Lfd. Nr. | Saccharose [%] | Glucose [%] | Fructose [%] | Rest [%] |
|---|---|---|---|---|---|
| 1 | 202111129 | 94,19 | 0,77 | 0,50 | 4,53 |
| 2 | 202111133 | 94,31 | 0,76 | 0,49 | 4,44 |
| 3 | 202111134 | 94,47 | 0,74 | 0,47 | 4,31 |
| 4 | 202111135 | 94,73 | 0,70 | 0,45 | 4,11 |

**Tabelle 7: Zusammensetzung der getrockneten und konditionierten Kohlenhydratpräparationen sowie von Vergleichsprobe 1**

| Versuchsnumme r | Lfd. Nr. | Saccharose [%] | Glucose [%] | Fructose [%] | Rest [%] | Wasser [%] |
|---|---|---|---|---|---|---|
| 1 | 20211112 9 | k.A. | k.A. | k.A. | k.A. | k.A. |
| 2 | 20211113 3 | 94,05 | 0,76 | 0,49 | 4,43 | 0,28 |
| 3 | 20211113 4 | 94,20 | 0,73 | 0,47 | 4,30 | 0,29 |
| 4 | 20211113 5 | 94,33 | 0,70 | 0,45 | 4,10 | 0,42 |

| | | | | | | |
|---|---|---|---|---|---|---|
| k.A.: nicht messbar | | | | | | |

**Tabelle 8: Rieselfähigkeit (abgekürzt Rieself.) und Böschungswinkel (abgekürzt Bö-winkel) der getrockneten und konditionierten Kohlenhydratpräparationen sowie von Vergleichsprobe 1**

| Versuchsnummer | Lfd. Nr. | Bö-winkel [°] | Rieself. 6 mm [s/100g] | Rieself. 8 mm [s/100g] | Rieself. 10 mm [s/100g] | Rieself. 11,3 mm [s/100g] | Rieself. 15 mm [s/100g] | Rieself. 25 mm [s/100g] |
|---|---|---|---|---|---|---|---|---|
| 1 | 202111129 | k.A. | k.A. | k.A. | k.A. | k.A. | k.A. | k.A. |
| 2 | 202111133 | 33,7 | 32,6 | 14,7 | 7,7 | 4,5 | 2 | 0,1 |
| 3 | 202111134 | 36,4 | k.A. | 17,4 | 9,3 | 5,3 | 2,4 | 0,2 |
| 4 | 202111135 | 38,5 | k.A. | 17,4 | 9,7 | 6,1 | 2,7 | 0,3 |

| | | | | | | | | |
|---|---|---|---|---|---|---|---|---|
| k.A.: nicht messbar | | | | | | | | |

Beispiel 2: Vergleiche erfindungsgemäß erhaltener Kohlenhydratpräparationen (Brauner Zucker Typ A) (Proben 2 bis 9) und einer Vergleichsprobe ohne Zugabe von Kohlenhydratpartikeln (Probe 1).

Die Herstellung der erfindungsgemäß erhaltenen Kohlenhydratpräparationen sowie der Vergleichsprobe 1 erfolgte analog zu Beispiel 1. Aus Tabellen 9 bis 15 ergeben sich die Charakteristika der Vergleichsprobe 1 (ohne Zugabe von feinkristalliner Saccharose, also ohne Durchführung von Verfahrensschritten b) und c)), von erfindungsgemäß getrockneten Produkten (Proben 2 bis 5) und von erfindungsgemäß getrockneten und konditionierten Produkten (Proben 6 bis 9).

Die Proben wurden, soweit möglich, anhand des Wassergehalts (KF-Methode) insgesamt (Wasser ges.) und des Wassergehalts an der Oberfläche (KF-Methode) (Wasser oberfl.), des Böschungswinkels und der Rieselfähigkeit charakterisiert und verglichen Der eingesetzte Puderzucker (feinkristalline Saccharose) entspricht der kristallinen Saccharose gemäß Beispiel 1.

Die Ergebnisse sind in Tabelle 16 zusammengefasst. Hierbei ist zu erkennen, dass getrocknete Brauner Zucker Typ A-Proben (Proben 2 bis 5) durch das Erhöhen der Zugabemenge der feinkristallinen Saccharose von 2 Gew.-% auf bis zu 20 Gew.-% (bezogen auf Ausgangsgewicht Brauner Zucker Typ A-Probe) und im Vergleich zum Vergleichsbeispiel (Probe 1, 0 Gew.-% Kohlenhydratpartikel in Form von feinkristalliner Saccharose) eine deutlich erhöhte Rieselfähigkeit zeigen. Beispielsweise zeigt insbesondere Tabelle 16, dass die gemessene Zeit, die benötigt wird, deutlich verringert wird, wenn erfindungsgemäße Proben im Vergleich mit Probe 1 eingesetzt werden, insbesondere bei einer Auslauföffnungsgröße von 15 mm. Die Vergleichsprobe 1 zeigt mit Auslauföffnungsgrößen kleiner 15 mm überhaupt keine messbare Rieselfähigkeit (k.A. in Tabelle 16), anders als die erfindungsgemäßen Proben, die auch bei kleineren Öffnungen gut rieselfähig sind.

Es wird gezeigt, dass mit steigender Zugabemenge der feinkristallinen Kohlenhydratpartikel die benötigte Zeit von 2,3 s (Vergleichsbeispiel, Probe 1, 0 Gew.-% feinkristalline Kohlenhydratpartikel) auf 0,2 s (Probe 5, 20 Gew.-% feinkristalline Kohlenhydratpartikel) abnimmt (Rieself. 25 mm). Außerdem zeigen die getrockneten Proben mit zunehmender Zugabemenge der feinkristallinen Kohlenhydratpartikel sowohl einen abnehmenden Gesamtwassergehalt als auch einen abnehmenden Wassergehalt an der Oberfläche der Proben.

Die Proben 6 bis 9 wurden zusätzlich zu den erfindungsgemäßen Verfahrensschritten b) und c) einem daran anschließenden Konditionierschritt d), so wie in Beispiel 1 beschrieben, unterzogen. Die erhaltenen getrockneten und konditionierten Präparationen zeigten ein nahezu gleiches Verhalten wie Proben 2 bis 5, wobei die Proben 6 bis 9 im Vergleich zu den getrockneten Proben 2 bis 5 bei gleicher Zugabemenge der feinkristallinen Kohlenhydratpartikel und insbesondere bei niedrigen Zugabemengen der feinkristallinen Kohlenhydratpartikel von 2 % und 5 % (Proben 6 und 7 im Vergleich zu Proben 2 und 3) eine nochmals bessere Rieselfähigkeit sowie niedrigere Gesamtwassergehalte und Wassergehalte an der Oberfläche der Proben aufweisen. Der Wassergehalt bei den getrockneten und konditionierten Proben ab 10 % Zugabe von feinkristallinen Kohlenhydratpartikeln (Proben 8 und 9) nimmt nicht weiter ab. Bei getrockneten und konditionierten Proben ist ebenfalls zu sehen, dass der Böschungswinkel mit steigender Menge an feinkristallinen Kohlenhydratpartikeln abnimmt, was ebenfalls für eine erhöhte Rieselfähigkeit spricht. Auch in diesem Beispiel wurde die Kontrollprobe (Probe 1) dem Konditionierungsschritt unterworfen. Es resultierte ein sehr teigiges, sehr klebriges Produkt mit nicht messbarer Rieselfähigkeit.

**Tabelle 9: Mengen der Ausgangsstoffe zur Herstellung der kristallinen wasserhaltigen Saccharosepräparation**

| Versuchsnr. | Llf. Nr. | Saccharose (g) | Invertzuckersirup (g) | Karamellzuckersirup (g) |
|---|---|---|---|---|
| 1 | 202108480 | 979,8 | 14,1 | 6,1 |
| 2 | 202108481 | 979,8 | 14,1 | 6,1 |
| 3 | 202108482 | 979,8 | 14,1 | 6,1 |
| 4 | 202108483 | 979,8 | 14,1 | 6,1 |
| 5 | 202108484 | 979,8 | 14,1 | 6,1 |
| 6 | 202108485 | 979,8 | 14,1 | 6,1 |
| 7 | 202108486 | 979,8 | 14,1 | 6,1 |
| 8 | 202108487 | 979,8 | 14,1 | 6,1 |
| 9 | 202108488 | 979,8 | 14,1 | 6,1 |

**Tabelle 10: Zusammensetzung der kristallinen wasserhaltigen Saccharosepräparation gemäß Tabelle 9**

| Versuchsnr. | Lfd. Nr. | Saccharose in % | Glucose in % | Fructose in % | Rest in % | Wasser % |
|---|---|---|---|---|---|---|
| 1 | 202108480 | 97,99 | 0,49 | 0,45 | 0,44 | 0,64 |
| 2 | 202108481 | 97,99 | 0,49 | 0,45 | 0,44 | 0,64 |
| 3 | 202108482 | 97,99 | 0,49 | 0,45 | 0,44 | 0,64 |
| 4 | 202108483 | 97,99 | 0,49 | 0,45 | 0,44 | 0,64 |
| 5 | 202108484 | 97,99 | 0,49 | 0,45 | 0,44 | 0,64 |
| 6 | 202108485 | 97,99 | 0,49 | 0,45 | 0,44 | 0,64 |
| 7 | 202108486 | 97,99 | 0,49 | 0,45 | 0,44 | 0,64 |
| 8 | 202108487 | 97,99 | 0,49 | 0,45 | 0,44 | 0,64 |
| 9 | 202108488 | 97,99 | 0,49 | 0,45 | 0,44 | 0,64 |

**Tabelle 11: Zusammensetzung der Gesamttrockenmasse der wasserhaltigen Kohlenhydratpräparationen gemäß Tabelle 10**

| Versuchsnr. | Lfd. Nr. | Saccharose in % | Glucose in % | Fructose in % | Rest in % |
|---|---|---|---|---|---|
| 1 | 202108480 | 98,62 | 0,49 | 0,45 | 0,44 |
| 2 | 202108481 | 98,62 | 0,49 | 0,45 | 0,44 |
| 3 | 202108482 | 98,62 | 0,49 | 0,45 | 0,44 |
| 4 | 202108483 | 98,62 | 0,49 | 0,45 | 0,44 |
| 5 | 202108484 | 98,62 | 0,49 | 0,45 | 0,44 |
| 6 | 202108485 | 98,62 | 0,49 | 0,45 | 0,44 |
| 7 | 202108486 | 98,62 | 0,49 | 0,45 | 0,44 |
| 8 | 202108487 | 98,62 | 0,49 | 0,45 | 0,44 |
| 9 | 202108488 | 98,62 | 0,49 | 0,45 | 0,44 |

**Tabelle 12: eingesetzte Mengen der Ausgangsstoffe zur Herstellung der getrockneten kristallinen Kohlenhydratpräparation sowie von Vergleichsprobe 1**

| Versuchs nr. | Lfd. Nr. | Saccharose (g) | Invertzuckersirup (g) | Karamellzuckersirup (g) | feinkristalline Saccharose (g) |
|---|---|---|---|---|---|
| 1 | 2021084 80 | 979,8 | 14,1 | 6,1 | - |
| 2 | 2021084 81 | 979,8 | 14,1 | 6,1 | 20,0 |
| 3 | 2021084 82 | 979,8 | 14,1 | 6,1 | 50,0 |
| 4 | 2021084 83 | 979,8 | 14,1 | 6,1 | 100,0 |
| 5 | 2021084 84 | 979,8 | 14,1 | 6,1 | 200,0 |
| 6 | 2021084 85 | 979,8 | 14,1 | 6,1 | 20,0 |
| 7 | 2021084 86 | 979,8 | 14,1 | 6,1 | 50,0 |
| 8 | 2021084 87 | 979,8 | 14,1 | 6,1 | 100,0 |
| 9 | 2021084 88 | 979,8 | 14,1 | 6,1 | 200,0 |

**Tabelle 13: Zusammensetzung der getrockneten Kohlenhydratpräparationen Nr. 2 bis 9 (vor Konditionieren gemäß Verfahrensschritt d) für die Proben 6 bis 9) sowie von Vergleichsprobe 1 gemäß Tabelle 12**

| Versuchsnr. | Lfd. Nr. | Saccharose in % | Glucose in % | Fructose in % | Rest in % | Wasser % |
|---|---|---|---|---|---|---|
| 1 | 202108480 | 97,99 | 0,49 | 0,45 | 0,44 | 0,64 |
| 2 | 202108481 | 98,03 | 0,48 | 0,44 | 0,43 | 0,63 |
| 3 | 202108482 | 98,08 | 0,46 | 0,43 | 0,42 | 0,61 |
| 4 | 202108483 | 98,17 | 0,44 | 0,41 | 0,40 | 0,58 |
| 5 | 202108484 | 98,32 | 0,41 | 0,37 | 0,36 | 0,53 |
| 6 | 202108485 | 98,03 | 0,48 | 0,44 | 0,43 | 0,63 |
| 7 | 202108486 | 98,08 | 0,46 | 0,43 | 0,42 | 0,61 |
| 8 | 202108487 | 98,17 | 0,44 | 0,41 | 0,40 | 0,58 |
| 9 | 202108488 | 98,32 | 0,41 | 0,37 | 0,36 | 0,53 |

**Tabelle 14: Zusammensetzung der Gesamttrockenmasse der Kohlenhydratpräparationen gemäß Tabelle 13**

| Versuchsnr. | Lfd. Nr. | Saccharose in % | Glucose in % | Fructose in % | Rest in % |
|---|---|---|---|---|---|
| 1 | 202108480 | 98,62 | 0,49 | 0,45 | 0,44 |
| 2 | 202108481 | 98,65 | 0,48 | 0,44 | 0,43 |
| 3 | 202108482 | 98,69 | 0,47 | 0,43 | 0,42 |
| 4 | 202108483 | 98,75 | 0,45 | 0,41 | 0,40 |
| 5 | 202108484 | 98,85 | 0,41 | 0,37 | 0,37 |
| 6 | 202108485 | 98,65 | 0,48 | 0,44 | 0,43 |
| 7 | 202108486 | 98,69 | 0,47 | 0,43 | 0,42 |
| 8 | 202108487 | 98,75 | 0,45 | 0,41 | 0,40 |
| 9 | 202108488 | 98,85 | 0,41 | 0,37 | 0,37 |

**Tabelle 15: Zusammensetzung der getrockneten (Nr. 2 bis 5) und getrockneten und konditionierten (Nr. 6 bis 9) Kohlenhydratpräparationen sowie von Vergleichsprobe 1**

| Versuchsnr. | Lfd. Nr. | Saccharose in % | Glucose in % | Fructose in % | Rest in % | Wasser % |
|---|---|---|---|---|---|---|
| 1 | 202108480 | 97,99 | 0,49 | 0,45 | 0,44 | 0,64 |
| 2 | 202108481 | 98,03 | 0,48 | 0,44 | 0,43 | 0,63 |
| 3 | 202108482 | 98,08 | 0,46 | 0,43 | 0,42 | 0,61 |
| 4 | 202108483 | 98,17 | 0,44 | 0,41 | 0,40 | 0,58 |
| 5 | 202108484 | 98,32 | 0,41 | 0,37 | 0,36 | 0,53 |
| 6 | 202108485 | 98,52 | 0,48 | 0,44 | 0,43 | 0,13 |
| 7 | 202108486 | 98,42 | 0,47 | 0,43 | 0,42 | 0,27 |
| 8 | 202108487 | 98,63 | 0,45 | 0,41 | 0,40 | 0,12 |
| 9 | 202108488 | 98,72 | 0,41 | 0,37 | 0,36 | 0,13 |

**Tabelle 16: Böschungswinkel und Rieselfähigkeit sowie Wassergehalt und Wassergehalt an der Oberfläche von verschiedenen Brauner Zucker Typ A-Proben (Vergleichsbeispiel ohne Puderzuckerzusatz (Vergleichsprobe 1) und erfindungsgemäß getrockneten (Proben 2 bis 5) oder erfindungsgemäß getrockneten und konditionierten Kohlenhydratpräparationen (Proben 6 bis 9))**

| Pro be | Lfd. Nr. | Feinkristall ine Saccharose [Gew.-%] | Konditi oniert | Bö-Win kel [°] | Ries elf. 6m m [s/10 0 g] | Ries elf. 8m m [s/10 0 g] | Ries elf. 10m m [s/10 0 g] | Ries elf. 11,3 mm [s/10 0 g] | Ries elf. 15m m [s/10 0 g] | Ries elf. 25m m [s/10 0 g] | Wass er ges. KF [g/10 0g] | Wass er oberf 1. KF [g/10 0g] |
|---|---|---|---|---|---|---|---|---|---|---|---|---|
| 1 | 20210 8480 | 0 | Nein | k. A. | k. A. | k. A. | k. A. | k. A. | 99,7 8 | 2,3 | 0,49 | 0,47 |
| 2 | 20210 8481 | 2 | Nein | k. A. | k. A. | k. A. | 18,1 | 12,6 | 6,5 | 1,9 | 0,46 | 0,43 |
| 3 | 20210 8482 | 5 | Nein | 40,7 | k. A. | 22,5 | 13,2 | 8,1 | 3,9 | 0,6 | 0,44 | 0,42 |
| 4 | 20210 8483 | 10 | Nein | 38,1 | 34,2 | 15,5 | 8,2 | 5,2 | 2,3 | 0,2 | 0,37 | 0,34 |
| 5 | 20210 8484 | 20 | Nein | 41,9 | 39,1 | 15,7 | 8,3 | 5,3 | 2,2 | 0,2 | 0,33 | 0,31 |
| 6 | 20210 8485 | 2 | Ja | k. A. | 28,5 | 14,8 | 8,2 | 6,1 | 4,6 | 2,5 | 0,13 | 0,06 7 |
| 7 | 20210 8486 | 5 | Ja | 40,8 | 36,4 | 15,1 | 8,3 | 5,4 | 3,8 | 1,3 | 0,27 | 0,08 4 |
| 8 | 20210 8487 | 10 | Ja | 35,1 | 36,2 | 15,0 | 8,0 | 5,3 | 2,1 | 0,1 | 0,12 | 0,05 4 |
| 9 | 20210 8488 | 20 | Ja | 36,6 | 30,6 | 12,6 | 6,6 | 4,1 | 1,7 | 0,0 | 0,13 | 0,07 6 |

### Beispiel 3 Erfindungsgemäßes Verfahren zur Herstellung einer getrockneten und konditionierten Isomaltulose- und Trehalulose-haltigen Zusammensetzung und Herstellung einer Vergleichsprobe ohne Zugabe von Kohlenhydratpartikeln

Im Folgenden wird die Herstellung einer erfindungsgemäßen getrockneten Isomaltulose- und Trehalulose-haltigen Präparation aus einer wasserhaltigen kristallinen Isomaltulose- und Trehalulose-haltigen Ausgangspräparation beschrieben und deren Eigenschaften hinsichtlich ihrer Rieselfähigkeit und Klebrigkeit dargestellt.

Zunächst wird das Ausgangsmaterial der vorliegenden Erfindung, also die wasserhaltige kristalline Kohlenhydratausgangspräparation, aus einer festen kristallinen Isomaltulose- und Trehalulose-haltigen Präparation (im Folgenden auch "kristalline Präparation" genannt) und einem darauf aufzubringenden wässrigen Sirup einer Isomaltulose- und Trehalulose-haltigen Präparation hergestellt, wobei sich mit einem Sirupfilm überzogene Kohlenhydratpartikel bilden. Dieser das Ausgangsmaterial des erfindungsgemäßen Verfahrens bereitstellende Herstellschritt dient dazu, in standardisierter Weise ein Ausgangsprodukt für die vorliegenden Beispiele bereitzustellen und führt zu einem Produkt, wie es in der industriellen Herstellung anfällt und in industriellen nachfolgenden Trocknungsschritten weiterverarbeitet wird. Die erhaltenen Sirupfilm-überzogenen Partikel werden sodann in erfindungsgemäßer Weise durch Durchführung der Verfahrensschritt b) und c) getrocknet (Puderzugabe) und anschließend, optional, konditioniert.

**Tabelle 17 Analyse der festen kristallinen Isomaltulose- und Trehalulose-haltigen Präparation - (Zusammensetzung des Zuckerspektrums nach GC (Gaschromatografie) (GC-DB5-OTMS)**

| Probenbezeichn ung | Rieself. 15 mm Düse | Wassergeh alt ges. KF | Fructo se | Gluco se | Saccharo se | Trehal u. | Isomaltulo se |
|---|---|---|---|---|---|---|---|
| | chrono m. | titrim. | GC-DB5-OTMS | GC-DB5-OTMS | GC-DB5-OTMS | GC-DB5-OTMS | GC-DB5-OTMS |
| | s/100g | g/100g | g/100g | g/100g | g/100g | g/100g | g/100g |
| Kristalline Präparation | 3,0 | 5,1 | 0,06 | 0,04 | 0,01 | 0,43 | 93,8 |

| | | | | | | | |
|---|---|---|---|---|---|---|---|
| Trehalu. bedeutet Trehalulose | | | | | | | |

Puder Typ 1 und Typ 2 der festen kristallinen Isomaltulose- und Trehalulose-haltigen Präparation wiesen die gleiche Zusammensetzung wie die in der vorstehenden Tabelle 17 dargestellte kristalline Präparation auf, unterscheiden sich jedoch hinsichtlich ihrer Partikelgröße voneinander. Puder Typ 1 weist eine Partikelgröße von mindestens 90 Gewichts % kleiner als 0,05 mm auf. Puder Typ 2 weist eine Partikelgröße von mindestens 90 Gewichts % kleiner als 0,1 mm auf.

**Tabelle 18 Analyse des Sirups einer Isomaltulose- und Trehalulose-haltigen Präparation (Zusammensetzung des Zuckerspektrum nach GC (HPLC-NH2)**

| Probenbezeichnung | TS | Fructose | Glucose | Saccharose | Trehalu. | Isomaltulose |
|---|---|---|---|---|---|---|
| | ref. | HPLC-NH2 | HPLC-NH2 | HPLC-NH2 | HPLC-NH2 | HPLC-NH2 |
| | g/100g | g/100g | g/100g | g/100g | g/100g | g/100g |
| Sirup Typ A | 39,96 | 1,2 | 0,7 | 0,5 | 3,4 | 33 |

### Herstellung ohne Puderzugabe (Kontrolle):

In einem 3 L Mischer mit Flachrührer werden 1 kg kristalline Isomaltulose- und Trehalulose-haltige Präparation vorgelegt. Die entsprechende Menge wässrigen Sirups einer Isomaltulose- und Trehalulose-haltigen Präparation wird langsam, in kleinen Portionen und in definierten Abständen auf die kristalline Isomaltulose- und Trehalulose-haltige Präparation gesprüht. Es folgt eine circa 5-minütige Mischzeit, um den Sirup homogen zu verteilen. Nach beendeter Mischzeit wird die Mischung mit warmer Luft konditioniert. Die Temperatur der Masse beträgt dabei circa 35 °C.

### Durchführung mit Puderzugabe (erfindungsgemäß):

In einem 3 L Mischer mit Flachrührer werden 1 kg kristalline Isomaltulose- und Trehalulose-haltige Präparation vorgelegt. Die entsprechende Menge wässrigen Sirups einer Isomaltulose- und Trehalulose-haltigen Präparation wird langsam, in kleinen Portionen und in definierten Abständen auf die kristalline Isomaltulose- und Trehalulose-haltige Präparation gesprüht. Es folgt eine circa 5-minütige Mischzeit, um den Sirup homogen zu verteilen. Nach beendeter Zugabe wird bei atmosphärischem Druck und Raumtemperatur von 21 bis 22 °C eine feinkristalline Isomaltulose- und Trehalulose-haltige Präparation (Puder) über eine Dosiereinrichtung auf die Masse gegeben. Die Mischung mit der Temperatur von 20 bis 21 °C wird 5 Minuten unter atmosphärischem Druck bei ca. 1000 mbar gemäß Verfahrensschritt c) der vorliegenden Erfindung homogenisiert, bis die Kohlenhydratpartikel optisch erkennbar verteilt sind.

Es werden Zusammensetzungen der getrockneten Kohlenhydratpräparation gemäß dem erfindungsgemäßen Verfahren erhalten. Anschließend wird die Mischung mit warmer Luft konditioniert. Die Temperatur der Masse beträgt dabei circa 35 °C.

In einer ersten Versuchsreihe wurde die feste kristalline Isomaltulose- und Trehalulose-haltige Präparation mit 2,0 beziehungsweise 9,6 Gew.-% (bezogen auf Gesamtgewicht der Sirupfilm-beschichteten Ausgangspräparation) eines Isomaltulose- und Trehalulose-haltigen Sirups Typ A behandelt und anschließend mit je 4,6 Gew.- % (bezogen auf Gesamtgewicht der Sirupfilm-beschichteten Ausgangspräparation inklusive des Gewichts des Puders) eines Isomaltulose- und Trehalulose-haltigen feinkristallinen Puders eines Typ 1 beziehungsweise Typ 2 beschichtet.

**Tabelle 19 Erste Versuchsreihe**

| **Versuchs Nr.** | **kristalline Präparation** | **Sirup Typ A** | **Puder Typ 1** | **Puder Typ 2** |
|---|---|---|---|---|
| | g | g | g | g |
| 1 | 1000 | 20 | 0 | 0 |
| 2 | 1000 | 106 | 0 | 0 |
| 3 | 1000 | 106 | 53 | 0 |
| 4 | 1000 | 106 | 0 | 53 |

In einer zweiten Versuchsreihe wurde die feste kristalline Isomaltulose- und Trehalulose-haltige Präparation mit 2,0 beziehungsweise 4,8 Gew.-% (bezogen auf Gesamtgewicht der Sirupfilm-beschichteten Ausgangspräparation) des Sirups Typ A behandelt und anschließend mit je 4,8 Gew.-% (bezogen auf Gesamtgewicht der Sirupfilm-beschichteten Ausgangspräparation inklusive des Gewichts des Puders) des feinkristallinen Puders vom Typ 1 beziehungsweise Typ 2 beschichtet.

**Tabelle 20 Zweite Versuchsreihe**

| **Versuchs Nr.** | **kristalline Präparation** | **Sirup Typ A** | **Puder Typ 1** | **Puder Typ 2** |
|---|---|---|---|---|
| | **g** | **g** | **g** | **g** |
| 1 | 1000 | 20 | 0 | 0 |
| 5 | 1000 | 50 | 0 | 0 |
| 6 | 1000 | 50 | 0 | 53 |
| 7 | 1000 | 50 | 53 | 0 |

### Rieselfähigkeit:

In Tabelle 21 sind die Ergebnisse der ersten Versuchsreihe zusammengefasst. Es zeigt sich eine Zunahme der Zeit der Rieselfähigkeit gemessen in einer 15 mm Düse von dem kristallinen Präparat (Kontrolle) über zu den beiden mit Sirup beschichteten wasserhaltigen Ausgangspräparationen der Versuche 1 und 2. Bei erfindungsgemäßer Anwendung der feinkristallinen Puder Typ 1 und 2 in Versuchen 3 und 4 nimmt die Zeit der Rieselfähigkeit wieder ab.

**Tabelle 21 Ergebnisse der Analysen der Produkte der Versuchsreihe 1**

| Versuchs Nr. | Rieself. 15 mm Düse | Wassergehalt ges. nach KF | Trehalulose |
|---|---|---|---|
| | chronom. | titrim. | GC-DB5-OTMS |
| | s/100g | g/100g | g/100g |
| Kristallines Präparat | 3,0 | 5,10 | 0,43 |
| 1 | 4,2 | 5,33 | 0,51 |
| 2 | 4,8 | 5,47 | 0,75 |
| 3 | 2,3 | 5,20 | 0,81 |
| 4 | 2,4 | 5,18 | 0,72 |

| | | | |
|---|---|---|---|
| Chronom. bedeuted chronometrisch, s/100g: Sekunden Rieselzeit pro 100 g, tritrim. bedeutet titrimetrisch, g/100g: Gramm Wasser pro 100 g. | | | |

Ein analoges Bild ergibt sich bei der Versuchsreihe 2. In der Tabelle 22 sind die Ergebnisse der zweiten Versuchsreihe zusammengefasst. Es zeigt sich eine Zunahme der Zeit der Rieselfähigkeit von dem kristallinen Präparat (Kontrolle) über die beiden mit Sirup beschichteten wasserhaltigen Ausgangspräparationen der Versuche 1 und 5. Bei erfindungsgemäßer Anwendung der feinkristallinen Puder Typ 1 und 2 in Versuchen 5 und 6 nimmt die Zeit der Rieselfähigkeit wieder ab.

**Tabelle 22 Ergebnisse der Analysen der Produkte der Versuchsreihe 2**

| Versuchs Nr. | Rieself. 15 mm Düse | Wassergehalt ges. nach KF | Trehalulose |
|---|---|---|---|
| | chronom. | titrim. | GC-DBS-OTMS |
| | s/100g | g/100g | g/100g |
| Kristallines Präparat | 3,0 | 5,10 | 0,43 |
| 1 | 4,2 | 5,33 | 0,51 |
| 5 | 6,2 | 5,41 | 0,59 |
| 6 | 2,7 | 5,10 | 0,58 |
| 7 | 2,9 | 5,37 | 0,58 |

### Bestimmung der Klebrigkeit:

Die Bestimmung der Klebrigkeit wurde an drei ausgewählten Proben durchgeführt; die kristalline Isomaltulose- und Trehalulose-haltige Präparation vor Sirupbehandlung (Versuch 1), die mit Sirup beschichtete wasserhaltige kristalline Isomaltulose- und Trehalulose-haltige Ausgangspräparation (Versuch 2) und die mit Sirup beschichtete wasserhaltige kristalline Isomaltulose- und Trehalulose-haltige Ausgangspräparation, die anschließend erfindungsgemäß mit einem Puder einer kristallinen Isomaltulose- und Trehalulose-haltigen Präparation behandelt und getrocknet wurde (Versuch 3).

Die Messwerte gemäß Tabelle 23 zeigen, dass die die kristalline Präparation am wenigsten klebrig ist, mit einem Kraftwert von 84,5 g. Nach einer Behandlung mit Sirup (Versuch 2) resultiert eine deutlich höhere Kraft, die die deutlich höhere Klebrigkeit anzeigt. Nach der erfindungsgemäßen Behandlung dieser Proben mit mikrokristallinem Puder Typ 1 (Versuch 3) wird die Klebrigkeit deutlich reduziert.

**Tabelle 23 Klebrigkeit von ausgewählten Proben**

| **Versuch** | **Kraft in g** |
|---|---|
| Kristallines Präparat | 84,5 |
| 2 | 132,4 |
| 3 | 109,1 |

### Verbackungstest:

Ein Verbackungstest wurde anhand der Proben gemäß der Versuche 1 bis 3 zur Klebrigkeit durchgeführt. Anhand eines Verbackungstests kann bestimmt werden, welche Neigung zur Verbackung ein Material hat, wenn es verdichtet wird. Der Messwert wird sofort ohne weitere Lagerung und nach einer Woche Lagerung ermittelt. Die Verbackungsneigung eines partikulären Materials ist von einer Klebrigkeit der Einzelpartikel abhängig.

Die Messwerte, die von den nicht gelagerten Proben ermittelt worden sind, zeigen praktisch keinen signifikanten Unterschied und sind daher nicht aufgeführt. Tabelle 24 zeigt die gemessenen Werte nach einer Woche. Die kristalline Präparation (Versuch 1) zeigt einen besonders niedrigen Wert von 0,26 N. Die wasserhaltige Ausgangspräparation, die also gemäß Versuch 2 mit Sirup beschichtet worden ist, zeigt eine sehr deutliche Steigerung des Kraftwertes auf 6,33 N. Nach Behandlung mit Puder in Versuch 3 sinkt der Wert sehr deutlich auf einen Wert von 1,50 N ab.

**Tabelle 24 Verbackung von ausgewählten Proben**

| **Versuch** | **Kraft in N nach 1 Woche** |
|---|---|
| Kristallines Präparat | 0,26 |
| 2 | 6,33 |
| 3 | 1,50 |

Die Versuche zeigen, dass es möglich ist, durch Anwendung von feinkristalliner Isomaltulose- und Trehalulose-haltiger Präparation (Puder) eine wasserhaltige Isomaltulose- und Trehalulose-haltige Ausgangspräparation, welche aus einer kristallinen Isomaltulose- und Trehalulose-haltigen Präparation durch Beschichtung mit einem Sirup einer Isomaltulose- und Trehalulose-haltigen Präparation hergestellt worden ist, in der Rieselfähigkeit, insbesondere Klebrigkeit und Verbackungsneigung, zu verbessern. Überraschenderweise kommt es nicht zu einem Verbacken oder Verklumpen, sondern einer deutlichen Verbesserung der Rieselfähigkeit.

### Beispiel 4 Erfindungsgemäßes Verfahren zur Herstellung einer getrockneten und konditionierten Isomalt-haltigen Zusammensetzung und Herstellung einer Vergleichsprobe ohne Zugabe von Kohlenhydratpartikeln

Im Folgenden wird die Herstellung einer erfindungsgemäßen getrockneten Isomalt-haltigen Präparation aus einer wasserhaltigen kristallinen Isomalt-haltigen Ausgangspräparation beschrieben und deren Eigenschaften hinsichtlich ihrer Rieselfähigkeit und Klebrigkeit dargestellt.

Charakterisierung der Ausgangsmaterialien:
Kristallines Isomalt Typ 1 (mindestens 90 % der Kristalle mit Durchmesser von 0,8 - 1,5 mm),
Isomalt-Lösung (Sirup),
Isomalt-Puder Typ 1 (mindestens 90 % der Kristalle mit Durchmesser von <0,1 mm) (Puder).

Zur Herstellung der in Verfahrensschritt a) eingesetzten kristallinen, wasserhaltigen Isomalt-Ausgangspräparation wurde zunächst festes kristallines Isomalt Typ 1 (im Folgenden auch "kristalline Präparation" genannt) mit Isomalt-Lösung beschichtet, wobei sich mit einem Sirupfilm überzogene Kohlenhydratpartikel bilden. Dieser das Ausgangsmaterial des erfindungsgemäßen Verfahrens bereitstellende Herstellschritt dient dazu, in standardisierter Weise ein Ausgangsprodukt für die vorliegenden Beispiele bereitzustellen und führt zu einem Produkt, wie es in der industriellen Herstellung anfällt und in industriellen nachfolgenden Trocknungsschritten weiterverarbeitet wird. Diese Sirupfilm überzogenen Partikel werden sodann in erfindungsgemäßer Weise durch Durchführung der Verfahrensschritt b) und c) getrocknet (Puderzugabe) und anschließend, optional, konditioniert.

### Versuche ohne Puderzugabe (Kontrolle):

In einem 5 L Dragierkessel werden 1 kg kristallines Isomalt vorgelegt. Der Dragierkessel wird bei circa 21 UpM rotiert. Die entsprechende Menge der Isomalt-Lösung wird langsam, in kleinen Portionen und in definierten zeitlichen Abständen auf das Isomalt gesprüht. Zwischen dem Sprühen wird die Mischung kurz mittels warmer Luft angetrocknet und eine kristalline, wasserhaltige Isomalt-Ausgangspräparation erhalten, in der die Isomalt-Kristalle mit einem Sirupfilm überzogen sind. Nach beendeter Zugabe der Isomalt-Lösung wird die Mischung mit warmer Luft circa 10 Minuten konditioniert. Die Temperatur der Masse beträgt dabei circa 45 °C. Um eventuell auftretende Agglomerate zu entfernen, wird die Mischung gesiebt.

### Versuche mit Puderzugabe (erfindungsgemäß):

In einem 5 L Dragierkessel werden 1 kg Isomalt vorgelegt. Der Dragierkessel wird bei ca. 21 UpM rotiert. Die entsprechende Menge der Isomalt-Lösung wird langsam, in kleinen Portionen und in definierten zeitlichen Abständen auf das Isomalt gesprüht. Zwischen dem Sprühen wird die Mischung kurz mittels warmer Luft angetrocknet und eine kristalline, wasserhaltige Isomalt-Ausgangspräparation erhalten, in der die Isomalt-Kristalle mit einem Sirupfilm überzogen sind. Nach beendeter Zugabe wird bei atmosphärischem Druck und Raumtemperatur von 21 bis 22 °C die feinkristalline Isomalt-Komponente (Puder Typ 1) in einer Menge von 4,6 Gew.-% (bezogen auf Gesamtgewicht der Sirupfilm-beschichteten Ausgangspräparation inklusive Puderanteil) über eine Dosiereinrichtung auf die Masse gegeben. Die Mischung mit der Temperatur von 20 bis 21 °C wird 5 Minuten unter atmosphärischem Druck bei ca. 1000 mbar gemäß Verfahrensschritt c) der vorliegenden Erfindung homogenisiert, bis die Kohlenhydratpartikel optisch erkennbar verteilt sind. Nach beendeter Zugabe wird die erfindungsgemäß erhaltene getrocknete Isomalt-Präparation mit warmer Luft circa 10 Minuten konditioniert. Die Temperatur der Masse beträgt dabei zwischen circa 45 °C. Um eventuell auftretende Agglomerate zu entfernen, wird die Mischung gesiebt.

**Tabelle 25: Versuchsübersicht mit Rezeptur-Mengenangaben**

| Nr. | Isomalt Typ 1 | Isomalt Lösung | Isomalt Puder Typ 1 |
|---|---|---|---|
| - | [g] | [g] | [g] |
| 1 | 1000 | 50 | - |
| 2 | 1000 | 106 | - |
| 3 | 1000 | 106 | 53 |

**Tabelle 26: Analysen der Ausgangsmaterialien - Teil 1**

| Bezeichnung | Rieself. | Düse |
|---|---|---|
| | chronom. Ph.Eur. 2.9.16 | |
| | s/100g | |
| | MW | |
| Isomalt Typ 1 | 7,4 | Gemessen mit 10 mm Düse |

**Tabelle 27: Analysen der Ausgangsmaterialien - Teil 2**

| Bezeichnung | GPM | GPS | Sorbit |
|---|---|---|---|
| | GC-HT8-BOTMS | GC-HT8-BOTMS | GC-HT8-BOTMS |
| | g/100gTS | g/100gTS | g/100gTS |
| | MW | MW | MW |
| Isomalt Typ 1 | 47,7 | 51,1 | 0,09 |
| Isomalt Puder Typ 1 | 47,7 | 51,7 | 0,11 |

**Tabelle 28: Analysen der Ausgangsmaterialien - Teil 3**

| Produktanalyse Isomalt-Sirup | | |
|---|---|---|
| Parameter | Ist-Wert | Einheit |
| TS (refr.) korr. | 40,7 | % |
| Mannit | 1,7 | Norm-% |
| Sorbit | 4,1 | Norm-% |
| GPM | 47,7 | Norm-% |
| GPS | 45,1 | Norm-% |
| GPM+GPS | 92,9 | Norm-% |

| | | |
|---|---|---|
| Refr. bedeutet refraktometrisch. | | |

In Tabelle 29 sind die Ergebnisse der durchgeführten Versuche dargestellt. Es zeigt sich eine Zunahme der Zeit der Rieselfähigkeit des kristallinen Isomalt Typ 1 (Kontrolle) zu den beiden mit Sirup beschichteten wasserhaltigen kristallinen Isomalt-Ausgangspräparationen aus Versuchen 1 und 2. Nach erfindungsgemäßer Anwendung des feinkristallinen Isomalts in Versuch 3 nimmt die Zeit der Rieselfähigkeit wieder ab.

**Tabelle 29: Ergebnisse der Kontrollversuche und erfindungsgemäßem Versuch**

| **Versuch / Nr.** | **Rieselfähigkeit** | **Sorbit** | **Feuchte** |
|---|---|---|---|
| | **s/100g** | **g/100gTS** | **g/100g** |
| kristallines Isomalt Typ 1 | 7,4 * | 0,09 | 3,3 |
| 1 | 8,3 * | 0,13 | 3,8 |
| 2 | 8,9 * | 0,18 | 4,0 |
| 3 | 8,1 * | 0,17 | 3,9 |
| * mit 10 mm Düse | | | |

Die Rieselfähigkeit wurde gemäß EUROPEAN PHARMACOPOEIA 10.0 Method 2.9.16. Flowability bestimmt.

Tabelle 29 zeigt, dass sich eine Korrelation bei der Zugabe der Isomalt-Lösung zur Herstellung der wasserhaltigen kristallinen Isomalt-Ausgangspräparation hinsichtlich Rieselfähigkeit und Wassergehalt ergibt. Es wird eine Verschlechterung der Rieselfähigkeit gegenüber der kristallinen Kontrolle beobachtet. Die Rieseldauer verlängert sich durch die Sirupfilmbeschichtung je nach Sirup-Anteil um bis zu 20%. Ebenso steigt der Wassergehalt durch die Sirupfilmbeschichtung mit Isomalt-Sirup um ca. 17%.

Um eine erfolgreiche Sirupfilmbeschichtung des kristallinen Isomalts mit dem Sirup nachzuweisen, wurden die Kontrolle und die Versuchsproben auf den Sorbitgehalt geprüft, welcher im verwendeten Isomalt-Sirup in erhöhtem Maß auftritt. Wie in der Produktanalyse der Ausgangsmaterialien beschrieben, verfügt das Isomalt-Sirup über einen Sorbitgehalt von 4,1 Norm-%, während kristallines Isomalt Typ 1 lediglich 0,09 g/100gTS aufweist (Norm-% und g/100gTS in erster Näherung vergleichbar).

Tabelle 29 zeigt, dass mit steigendem Sirup Anteil auch der Sorbitgehalt ansteigt. Bei der mit Sirup (9,6%) beschichten Probe konnte ein doppelt so hoher Sorbitgehalt gegenüber der Isomalt- Typ-Kontrolle nachgewiesen werden. Somit konnte eine erfolgreiche Sirupfilmbeschichtung bestätigt werden.

Es konnte gezeigt werden, dass die Zugabe von feinkörnigen Isomalt-Partikeln überraschenderweise die Rieselfähigkeit von Sirupfilm-beschichteten Isomaltkristallen nicht durch zum Beispiel Verbacken, verschlechtert, sondern vielmehr deutlich verbessert.

## Patentansprüche

1. Verfahren zur Herstellung einer getrockneten Kohlenhydratpräparation aus einer wasserhaltigen kristallinen Kohlenhydrat-Ausgangspräparation, umfassend die Verfahrensschritte:
a) Bereitstellung einer wasserhaltigen 0,4 bis 22,5 Gew.-%, insbesondere 2,5 bis 12 Gew.-% Wasser, jeweils bezogen auf Gesamtgewicht der Kohlenhydrat-Ausgangspräparation, aufweisenden kristallinen Kohlenhydrat-Ausgangspräparation, wobei die Kohlenhydrat-Ausgangspräparation einen über einen gegebenenfalls vorhandenen Kristallwassergehalt hinausgehenden Gehalt an Wasser aufweist und wobei die Kohlenhydrat-Ausgangspräparation ausgewählt ist aus der Gruppe bestehend aus einer Saccharosehaltigen Zusammensetzung, einer Isomaltulose- und Trehalulose-haltigen Zusammensetzung und einer Isomalt-haltigen Zusammensetzung in einem Trocknungsreaktor, sowie von Kohlenhydratpartikeln, wobei die Kohlenhydratpartikel einen Anteil von Kohlenhydratkristallen mit einem Durchmesser von kleiner oder gleich 100 µm von mindestens 80 Gew.-% aufweisen,
b) Eindosieren der Kohlenhydratpartikel über eine dem Trocknungsreaktor zugeordnete Dosiervorrichtung in die in dem Trocknungsreaktor vorgelegte wasserhaltige kristalline Kohlenhydrat-Ausgangspräparation in einer Menge von 2 bis 30 Gew.-%, bezogen auf Gesamtgewicht der Kohlenhydrat-Ausgangspräparation, bei einem Druck von 10 bis 1100 mbar, wobei die in dem Trocknungsreaktor vorgelegte wasserhaltige Kohlenhydrat-Ausgangspräparation eine Temperatur von 20 bis 80 °C, insbesondere 40 bis 60 °C aufweist, und
c) Homogenisieren der erhaltenen Mischung mittels einer Mischvorrichtung unter den in Verfahrensschritt b) genannten Bedingungen zum Erhalten einer getrockneten Kohlenhydratpräparation.

2. Verfahren nach Anspruch 1, wobei die in Verfahrensschritt a) bereitgestellte wasserhaltige 0,4 bis 22,5 Gew.-%, insbesondere 2,5 bis 12 Gew.-% Wasser, jeweils bezogen auf Gesamtgewicht der Kohlenhydrat-Ausgangspräparation, aufweisende kristalline Kohlenhydrat-Ausgangspräparation Flüssigkeitsfilm-überzogene kristalline Kohlenhydratpartikel aufweist und in Verfahrensschritt c) eine getrocknete Kohlenhydratpräparation aus Flüssigkeitsfilm-befreiten kristallinen Kohlenhydratpartikeln erhalten wird.

3. Verfahren nach Anspruch 1 oder 2 wobei das Verfahren kontinuierlich, semi-kontinuierlich oder batch-weise durchgeführt wird.

4. Verfahren nach einem der vorhergehenden Ansprüche, wobei die wasserhaltige Kohlenhydrat-Ausgangspräparation mindestens ein schlecht-lösliches Kohlenhydrat mit einer Löslichkeit bei 20 °C in Wasser von 5 bis 53 g/100g, insbesondere 1,1-GPM, Isomaltulose, Glucose oder Mannit, und mindestens ein gut-lösliches Kohlenhydrat mit einer Löslichkeit bei 20 °C in Wasser von mehr als 53 g/100g, insbesondere Saccharose, 1,6-GPS, Fructose, Trehalulose oder Sorbit, umfasst.

5. Verfahren nach einem der vorhergehenden Ansprüche, wobei die wasserhaltige Kohlenhydrat-Ausgangspräparation eine wasserhaltige Saccharose-haltige Zusammensetzung ist, die kristalline Saccharose und mindestens eine weitere Kohlenhydrat-haltige Komponente ausgewählt aus der Gruppe bestehend aus Invertzucker, Karamellzuckersirup und Rohrzuckersirup aufweist.

6. Verfahren nach einem der vorhergehenden Ansprüche, wobei die Kohlenhydrate der wasserhaltigen Kohlenhydrat-Ausgangspräparation und der Kohlenhydratpartikel gleich sind.

7. Verfahren nach einem der vorhergehenden Ansprüche, wobei die Kohlenhydratpartikel in Verfahrensschritt b) in einer Menge von 2 bis 25 Gew.-%, insbesondere 4 bis 20 Gew.-%, insbesondere 4 bis 15 Gew.-% und insbesondere 4 bis 10 Gew.-%, jeweils bezogen auf Gesamtgewicht der Kohlenhydrat-Ausgangspräparation, eindosiert werden.

8. Verfahren nach einem der vorhergehenden Ansprüche, wobei das Eindosieren der Kohlenhydratpartikel in Verfahrensschritt b) in einem Über- und Unterdruck-freien Trocknungsreaktor unter atmosphärischem Druck, in einem Vakuum-Trocknungsreaktor unter reduziertem Druck, insbesondere 10 bis 900 mbar, insbesondere 20 bis 600, insbesondere 30 bis 400 mbar, insbesondere 40 bis 200 mbar, insbesondere 50 bis 100 mbar oder insbesondere 600 bis 800 mbar, insbesondere 650 bis 750 mbar, insbesondere 700 mbar, oder in einem Druck-Trocknungsreaktor unter einem Druck von atmosphärischem Druck bis 1100 mbar durchgeführt wird.

9. Verfahren nach einem der vorhergehenden Ansprüche, wobei im Anschluss an Verfahrensschritt c) in einem Verfahrensschritt d) ein Konditionieren erfolgt.

10. Verfahren nach einem der vorhergehenden Ansprüche, wobei das Konditionieren gemäß Verfahrensschritt d) bei einer Temperatur von mindestens 30 °C, insbesondere 30 bis 180 °C, insbesondere 35 bis 160 °C, insbesondere 45 bis 100 °C, insbesondere 35 bis 60°C, insbesondere 40 bis 60 °C, insbesondere 50 bis 60 °C, durchgeführt wird.

11. Verfahren nach einem der vorhergehenden Ansprüche, wobei das Konditionieren gemäß Verfahrensschritt d) bei einem Druck von 10 bis 1100 mbar, insbesondere 10 bis 900 mbar, insbesondere 20 bis 600 mbar, insbesondere 30 bis 400 mbar, insbesondere 40 bis 200 mbar, insbesondere 50 bis 100 mbar, durchgeführt wird.

12. Verfahren nach einem der vorhergehenden Ansprüche, wobei die wasserhaltige Saccharose-haltige Ausgangspräparation aus Zuckerrohr gewonnen wurde.

13. Verfahren nach einem der vorhergehenden Ansprüche, wobei die wasserhaltige Isomalthaltige Ausgangspräparation einen 1,1-GPM (1-O-alpha-D-glucopyranosyl-D-mannit)- und 1,6-GPS (6-O-alpha-D-glucopyranosyl-D-sorbit)-Gehalt von 95,0 bis 100,0 Gew.-%, bezogen auf Gesamttrockenmasse der wasserhaltigen Isomalt-haltigen Ausgangspräparation, aufweist.

14. Verfahren nach einem der vorhergehenden Ansprüche, wobei die wasserhaltige Isomalthaltige Ausgangspräparation einen 1,1-GPM (1-O-alpha-D-glucopyranosyl-D-mannit)-Gehalt von 45,0 bis 50,0 Gew.-% und einen 1,6-GPS (6-O-alpha-D-glucopyranosyl-D-sorbit)-Gehalt von 50 bis 55 Gew.-%, jeweils bezogen auf Gesamttrockenmasse der wasserhaltigen Isomalt-haltigen Ausgangspräparation, aufweist.

15. Verfahren nach einem der vorhergehenden Ansprüche, wobei die wasserhaltige Isomalthaltige Ausgangspräparation einen 1,1-GPM (1-O-alpha-D-glucopyranosyl-D-mannit)-Gehalt von 20,0 bis 30,0 Gew.-% und einen 1,6-GPS (6-O-alpha-D-glucopyranosyl-D-sorbit)-Gehalt von 70,0 bis 80,0 Gew.-%, jeweils bezogen auf Gesamttrockenmasse der wasserhaltigen Isomalt-haltigen Ausgangspräparation, aufweist.

16. Verfahren nach einem der vorhergehenden Ansprüche, wobei die wasserhaltige Isomalthaltige Ausgangspräparation 1,1-GPS (1-O-alpha-D-glucopyranosyl-D-sorbit), Sorbit, Mannit, oder GPI (Glucopyranosyl-idit) oder eine Mischung von zwei oder mehr davon aufweist.

17. Verfahren nach einem der vorhergehenden Ansprüche, wobei die wasserhaltige Isomaltulose- und Trehalulose-haltige Ausgangspräparation Fructose, Glucose, Isomaltose oder Isomelezitose oder eine Mischung von zwei oder mehr davon aufweist.

## Claims

1. A process for preparing a dried carbohydrate preparation from a water-containing crystalline carbohydrate starting preparation, comprising the process steps:
a) providing a water-containing crystalline carbohydrate starting preparation having 0.4 to 22.5 wt.-%, in particular 2.5 to 12 wt.-% water, each based on the total weight of the carbohydrate starting preparation, wherein the carbohydrate starting preparation has a content of water in excess of any possible water of crystallisation present and wherein the carbohydrate starting preparation is selected from the group consisting of a sucrose-containing composition, an isomaltulose- and trehalulose-containing composition and an isomalt-containing composition in a drying reactor, and carbohydrate particles, wherein the carbohydrate particles have a proportion of carbohydrate crystals with a diameter of less than or equal to 100 µm of at least 80 wt.-%,
b) metering in the carbohydrate particles via a metering device assigned to the drying reactor into the water-containing crystalline carbohydrate starting preparation presented in the drying reactor in an amount of 2 to 30 wt.-%, based on the total weight of the carbohydrate starting preparation, at a pressure of 10 to 1100 mbar, wherein the water-containing carbohydrate starting preparation presented in the drying reactor has a temperature of 20 to 80°C, in particular 40 to 60°C, and
c) homogenising the obtained mixture by means of a mixing device under the conditions mentioned in process step b) to obtain a dried carbohydrate preparation.

2. The process of claim 1, wherein the water-containing crystalline carbohydrate starting preparation having 0.4 to 22.5 wt.-%, in particular 2.5 to 12 wt.-% water, each based on the total weight of the carbohydrate starting preparation, provided in process step a) has liquid-film-coated crystalline carbohydrate particles and in process step c) a dried carbohydrate preparation is obtained from liquid-film-freed crystalline carbohydrate particles.

3. The process of claim 1 or 2 wherein the process is carried out continuously, semi-continuously or batchwise.

4. The process of one of the preceding claims, wherein the water-containing carbohydrate starting preparation comprises at least one poorly-soluble carbohydrate with a solubility at 20°C in water of 5 to 53 g/100g, in particular 1,1-GPM, isomaltulose, glucose or mannitol, and at least one well-soluble carbohydrate with a solubility at 20°C in water of more than 53 g/100g, in particular sucrose, 1,6-GPS, fructose, trehalulose or sorbitol.

5. The process of one of the preceding claims, wherein the water-containing carbohydrate starting preparation is a water-containing sucrose-containing composition having crystalline sucrose and at least one further carbohydrate-containing component selected from the group consisting of invert sugar, caramel sugar syrup and cane sugar syrup.

6. The process of one of the preceding claims, wherein the carbohydrates of the water-containing carbohydrate starting preparation and the carbohydrate particles are the same.

7. The process of one of the preceding claims, wherein the carbohydrate particles are metered in in process step b) in an amount of 2 to 25 wt.-%, in particular 4 to 20 wt.-%, in particular 4 to 15 wt.-% and in particular 4 to 10 wt.-%, each based on the total weight of the carbohydrate starting preparation.

8. The process of one of the preceding claims, wherein the metering-in of the carbohydrate particles in process step b) is carried out in a drying reactor free from excess pressure and negative pressure under atmospheric pressure, in a vacuum drying reactor under reduced pressure, in particular 10 to 900 mbar, in particular 20 to 600, in particular 30 to 400 mbar, in particular 40 to 200 mbar, in particular 50 to 100 mbar or in particular 600 to 800 mbar, in particular 650 to 750 mbar, in particular 700 mbar, or is carried out in a pressure drying reactor under a pressure of atmospheric pressure up to 1100 mbar.

9. The process of one of the preceding claims, wherein, following process step c), conditioning is carried out in a process step d).

10. The process of one of the preceding claims, wherein the conditioning according to process step d) is carried out at a temperature of at least 30 °C, in particular 30 to 180 °C, in particular 35 to 160 °C, in particular 45 to 100 °C, in particular 35 to 60 °C, in particular 40 to 60 °C, in particular 50 to 60 °C.

11. The process of one of the preceding claims, wherein the conditioning according to process step d) is carried out at a pressure of 10 to 1100 mbar, in particular 10 to 900 mbar, in particular 20 to 600 mbar, in particular 30 to 400 mbar, in particular 40 to 200 mbar, in particular 50 to 100 mbar.

12. The process of one of the preceding claims, wherein the water-containing sucrose-containing starting preparation was recovered from sugar cane.

13. The process of one of the preceding claims, wherein the water-containing isomalt-containing starting preparation has a 1,1-GPM (1-O-alpha-D-glucopyranosyl-D-mannitol) and 1,6-GPS (6-O-alpha-D-glucopyranosyl-D-sorbitol) content of 95.0 to 100.0 wt.-%, based on the total dry mass of the water-containing isomalt-containing starting preparation.

14. The process of one of the preceding claims, wherein the water-containing isomalt-containing starting preparation has a 1,1-GPM (1-O-alpha-D-glucopyranosyl-D-mannitol) content of 45.0 to 50.0 wt.-% and a 1,6-GPS (6-O-alpha-D-glucopyranosyl-D-sorbitol) content of 50 to 55 wt.-%, each based on the total dry mass of the water-containing isomalt-containing starting preparation.

15. The process of one of the preceding claims, wherein the water-containing isomalt-containing starting preparation has a 1,1-GPM (1-O-alpha-D-glucopyranosyl-D-mannitol) content of 20.0 to 30.0 wt.-% and a 1,6-GPS (6-O-alpha-D-glucopyranosyl-D-sorbitol) content of 70.0 to 80.0 wt.-%, each based on the total dry mass of the water-containing isomalt-containing starting preparation.

16. The process of one of the preceding claims, wherein the water-containing isomalt-containing starting preparation has 1,1-GPS (1-O-alpha-D-glucopyranosyl-D-sorbitol), sorbitol, mannitol, or GPI (glucopyranosyl-iditol) or a mixture of two or more thereof.

17. The process of one of the preceding claims, wherein the water-containing isomaltulose- and trehalulose-containing starting preparation has fructose, glucose, isomaltose or isomelecitose or a mixture of two or more thereof.

## Revendications

1. Procédé de fabrication d'une préparation de glucides séchée à partir d'une préparation initiale de glucides cristalline contenant de l'eau, comprenant les étapes de procédé :
a) préparer une préparation initiale de glucides cristalline contenant 0,4 à 22,5 % en poids, en particulier 2,5 à 12 % en poids d'eau rapporté respectivement au poids total de la préparation initiale de glucides, la préparation initiale de glucides présentant une teneur en eau dépassant une teneur en eau de cristallisation éventuellement présente, et la préparation initiale de glucides étant choisie dans le groupe constitué d'une composition contenant du saccharose, d'une composition contenant de l'isomaltulose et du tréhalulose et d'une composition contenant de l'isomaltose dans un réacteur de séchage, ainsi que des particules de glucides, les particules de glucides comportant une proportion d'au moins 80 % en poids de cristaux de glucides d'un diamètre inférieur ou égal à 100 µm,
b) ajouter les particules de glucides par un dispositif de dosage affecté au réacteur de séchage à la préparation initiale de glucides cristalline contenant de l'eau préalablement disposée dans le réacteur de séchage à hauteur de 2 à 30 % en poids rapporté au poids total de la préparation initiale de glucides sous une pression de 10 à 1100 mbar, la préparation initiale de glucides contenant de l'eau préalablement disposée dans le réacteur de séchage présentant une température de 20 à 80°C, en particulier de 40 à 60°C, et
c) homogénéiser le mélange obtenu au moyen d'un mélangeur dans les conditions indiquées à l'étape de procédé b) pour obtenir une préparation de glucides séchée.

2. Procédé selon la revendication 1, dans lequel la préparation initiale de glucides cristalline préparée à l'étape de procédé a) contenant 0,4 à 22,5 % en poids, en particulier 2,5 à 12 % en poids d'eau rapporté respectivement au poids total de la préparation initiale de glucides, présente des particules de glucides cristallines recouvertes d'un film de liquide, et à l'étape de procédé c), on obtient une préparation de particules de glucides séchée à partir de particules de glucides cristallines libérées de leur film de liquide.

3. Procédé selon la revendication 1 ou 2, le procédé étant réalisé en tant que procédé continu, semi-continu ou par lots.

4. Procédé selon l'une quelconque des revendications précédentes, dans lequel la préparation initiale de glucides contenant de l'eau comprend au moins un glucide faiblement soluble avec une solubilité à 20°C dans l'eau de 5 à 53 g/100 g, en particulier du 1,1-GPM, de l'isomaltulose, du glucose ou du mannitol, et au moins un glucide facilement soluble avec une solubilité à 20°C dans l'eau supérieure à 53 g/100 g, en particulier du saccharose, du 1,6-GPS, du fructose, du tréhalulose ou du sorbitol.

5. Procédé selon l'une quelconque des revendications précédentes, dans lequel la préparation initiale de glucides contenant de l'eau est une composition contenant de l'eau et du saccharose, qui comporte du saccharose cristallin et au moins un autre composant contenant des glucides, choisi dans le groupe constitué de sucre inverti, du sirop de sucre caramélisé et du sirop de sucre de canne.

6. Procédé selon l'une quelconque des revendications précédentes, dans lequel les glucides de la préparation initiale de glucides contenant de l'eau et des particules de glucides sont identiques.

7. Procédé selon l'une quelconque des revendications précédentes, dans lequel les particules de glucides sont ajoutées à l'étape de procédé b) à hauteur de 2 à 25 % en poids, en particulier de 4 à 20 % en poids, en particulier de 4 à 15 % en poids, et en particulier de 4 à 10 % en poids, rapporté respectivement au poids total de la préparation initiale de glucides.

8. Procédé selon l'une quelconque des revendications précédentes, dans lequel l'ajout des particules de glucides à l'étape de procédé b) est effectué dans un réacteur de séchage sans surpression ni dépression, sous pression atmosphérique, dans un réacteur de séchage sous vide sous pression réduite, en particulier de 10 à 900 mbar, en particulier de 20 à 600 mbar, en particulier de 30 à 400 mbar, en particulier de 40 à 200 mbar, en particulier de 50 à 100 mbar, ou en particulier de 600 à 800 mbar, en particulier de 650 à 750 mbar, en particulier à 700 mbar, ou dans un réacteur de séchage sous pression sous une pression de la pression atmosphérique à 1100 mbar.

9. Procédé selon l'une quelconque des revendications précédentes, dans lequel un conditionnement est effectué après une étape de procédé c) dans une étape de procédé d).

10. Procédé selon l'une quelconque des revendications précédentes, dans lequel le conditionnement selon l'étape de procédé d) est effectué à une température d'au moins 30°C, en particulier de 30 à 180°C, en particulier de 35 à 160°C, en particulier de 45 à 100°C, en particulier de 35 à 60°C, en particulier de 40 à 60°C, en particulier de 50 à 60°C.

11. Procédé selon l'une quelconque des revendications précédentes, dans lequel le conditionnement selon l'étape de procédé d) est effectué sous une pression de 10 à 1100 mbar, en particulier de 10 à 900 mbar, en particulier de 20 à 600 mbar, en particulier de 30 à 400 mbar, en particulier de 40 à 200 mbar, en particulier de 50 à 100 mbar.

12. Procédé selon l'une quelconque des revendications précédentes, dans lequel la préparation initiale contenant de l'eau et du saccharose est obtenue à partir de canne à sucre.

13. Procédé selon l'une quelconque des revendications précédentes, dans lequel la préparation initiale contenant de l'eau et de l'isomaltose présente une teneur en 1,1-GPM (1-O-alpha-D-glucopyranosyl-D-mannitol) et en 1,6-GPS (6-O-alpha-D-glucopyranosyl-D-sorbitol) de 95,0 à 100,0 % en poids rapporté au poids sec total de la préparation initiale contenant de l'eau et de l'isomaltose.

14. Procédé selon l'une quelconque des revendications précédentes, dans lequel la préparation initiale contenant de l'eau et de l'isomaltose présente une teneur en 1,1-GPM (1-O-alpha-D-glucopyranosyl-D-mannitol) de 45,0 à 50,0 % en poids et une teneur en 1,6-GPS (6-O-alpha-D-glucopyranosyl-D-sorbitol) de 50 à 55 % en poids, rapporté respectivement au poids sec total de la préparation initiale contenant de l'eau et de l'isomaltose.

15. Procédé selon l'une quelconque des revendications précédentes, dans lequel la préparation initiale contenant de l'eau et de l'isomaltose présente une teneur en 1,1-GPM (1-O-alpha-D-glucopyranosyl-D-mannitol) de 20,0 à 30,0 % en poids et une teneur en 1,6-GPS (6-O-alpha-D-glucopyranosyl-D-sorbitol) de 70,0 à 80,0 % en poids, rapporté respectivement au poids sec total de la préparation initiale contenant de l'eau et de l'isomaltose.

16. Procédé selon l'une quelconque des revendications précédentes, dans lequel la préparation initiale contenant de l'eau et de l'isomaltose contient du 1,1-GPS (1-O-alpha-D-glucopyranosyl-D-sorbitol), du sorbitol, du mannitol, ou du GPI (glucopyranosyl-iditol) ou un mélange d'au moins deux d'entre eux.

17. Procédé selon l'une quelconque des revendications précédentes, dans lequel la préparation initiale contenant de l'eau et de l'isomaltulose et du tréhalulose contient du fructose, du glucose, de l'isomaltose ou de l'isomélézitose ou un mélange d'au moins deux d'entre eux.
